(19) Europäisches Patentamt
European Patent Office
Office européen des brevets

(11) **EP 4 422 209 A1**

(12) **EUROPEAN PATENT APPLICATION**
published in accordance with Art. 153(4) EPC

(43) Date of publication:
**28.08.2024 Bulletin 2024/35**

(21) Application number: **22883275.4**

(22) Date of filing: **16.09.2022**

(51) International Patent Classification (IPC):
***H04R 19/00*** *(2006.01)*

(52) Cooperative Patent Classification (CPC):
**H04R 19/00**

(86) International application number:
**PCT/JP2022/034796**

(87) International publication number:
**WO 2023/067965 (27.04.2023 Gazette 2023/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.10.2021 JP 2021172667**

(71) Applicant: **Silicon & System Co., Limited
San Po Kong, Kowloon, Hong Kong 999077 (HK)**

(72) Inventors:
• **TADAKI Yoshitaka
Musashimurayama-shi, Tokyo 208-0023 (JP)**
• **UMEMURA Shin-ichiro
Sendai-shi, Miyagi 984-0053 (JP)**
• **OGAYA Kaoru
Musashimurayama-shi, Tokyo 208-0023 (JP)**
• **TAKEMOTO Yoshiaki
Musashimurayama-shi, Tokyo 208-0023 (JP)**

(74) Representative: **Ipey
Apex House
Thomas Street
Trethomas
Caerphilly CF83 8DP (GB)**

(54) **ACOUSTIC INDUCTION-TYPE SEMICONDUCTOR ELEMENT AND ACOUSTIC ELEMENT INTEGRATED CIRCUIT**

(57)    [Problem] To provide an AI semiconductor element, which has a high sensitivity for smaller element size, and an AEIC.

[Solution] AI semiconductor element encompasses a p-type channel-generation region (14); n-type first and second main electrode regions (15b, 15a)buried in the channel-generation region (14); gate insulating films (12, 16) disposed on the channel-generation region (14) sandwiched by the first and second main electrode regions; a main floating electrode (17c) in a floating state, provided on the gate insulating films; fixed-potential electrodes (17o) located adjacently to the main floating electrode (17c), being set to a first potential; a vibration membrane (23) opposed to the first and fixed-potential electrodes via a vibration cavity; and a vibration electrode (25c) in contact with the upper surface of the vibration membrane (23), is opposed to the fixed-potential electrodes via the vibration cavity, being set to a second potential.

FIG3

EP 4 422 209 A1

**Description**

[Technical Field]

The present invention relates to an acoustic induction-type (AI) semiconductor element for controlling surface potential in semiconductor region with acoustic signals, and an acoustic element integrated circuit (AEIC) in which a plurality of AI semiconductor elements is merged in a common substrate, each of the AI semiconductor elements serving as at least unit cell.

[Background Art]

In ultrasonic probes for medical purposes, piezoelectric acoustic-elements have been used in the past. However, in the case of the piezoelectric acoustic-elements, miss-matchings in acoustic impedances between vibrators of the acoustic-elements and a human body are large, which leads to bottle neck issues. In recent years, a capacitive acoustic-element having a vibration-cavity, in which micro-electromechanical systems (MEMS) technology is used, has been developed. The capacitive acoustic-element, which is called "capacitive micromachined ultrasonic transducer (CMUT)", has a feature that the acoustic impedance is close to that of the human body. However, the CMUT is the acoustic-element in which a vibration cavity having a height of several hundred nanometres is provided on a silicon (Si) substrate, an upper electrode is provided on the vibration cavity, a lower electrode is provided under the vibration cavity. When ultrasonic waves are transmitted, by applying an electric-field between the vibration cavity, electrostatic power is generated, which vibrates a vibration membrane on the vibration cavity. When the ultrasonic waves are received, the vibration membrane is mechanically vibrated, which changes capacitance between the upper and lower electrodes. Then, electric signals are detected with the change in the capacitance between the upper and lower electrodes as change of voltages. Moreover, the capacitive acoustic-element is preferable as the ultrasonic probe for medical purpose because substances harmful to the human body is not used, contrasting with the piezoelectric acoustic-elements.

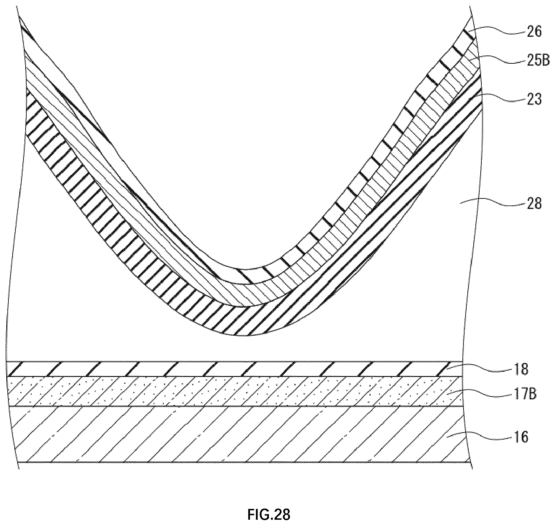

However, as illustrated in FIG. 28, an upper electrode 25B and a vibration membrane 23 under the upper electrode 25B do not approach a lower electrode 17B uniformly, as the parallel relation to a lower electrode 17 is not kept, by sound pressures of the ultrasonic waves. Then, since only centres of the upper electrode 25B and the lower electrode 17B are close to each other, the upper electrode 25B and the lower electrode 17B do not serve as a parallel plate capacitor, except for the centres. Thus, there are issues that the earlier CMUTs are low in reception sensibilities, because the sufficient uses of the capacitive changes as the parallel plate capacitors are impossible.

In view of the foregoing circumstances, one of the present inventors has proposed a structure in which a plurality of protrusions is provided on a lower surface of a vibration membrane, which is provided under an upper electrode in Patent Literature (PTL) 1. The protrusions are protruding downward in a vibration cavity, and a pattern of openings is further provided in the upper electrode, such that the positions of the openings are aligned with the positions of protrusions (see FIG. 14 and FIG. 15 in PTL 1).According to the invention recited in PTL 1, even if a higher voltage, the extent of which is such that the lower surface of the vibration membrane might be brought into contact with a lower insulating film covering the top surface of the lower electrode, is applied across the upper and lower electrodes, the situation such that the entire lower surface of the vibration membrane is brought into contact with the lower insulating film covering the lower electrode can be protected, because the protrusions serve as supporting posts. Then, an effectiveness of improving the operational reliability of the CMUT can be achieved is expected by the art disclosed in PTL 1.

However, even if the operational reliability is improved by the invention described in PTL 1, the situation in which only the centres of the upper and lower electrodes become close to each other is not improved. Therefore, because it is not always possible to sufficiently use the capacitance as the parallel plate capacitor, the issue that the reception sensitivity cannot be improved is still not solved.

Especially, the earlier capacitive acoustic-element which detects changes of electric charges as extensive variables has an inherent problem and theoretically, because detected values per element will become smaller and smaller when element sizes are miniaturized. That is, even if the size - -cell size- - of the earlier capacitive acoustic-element could be made smaller, the detected value per element will decrease. Thus, signals from a plurality of elements must be gathered. For this reason, in the earlier capacitive acoustic-elements, there were issues that the substantial miniaturization of the cell size is difficult, and it is impossible to reduce the effective cell sizes to achieve high-definition imaging. Also, when the signals from the plurality of elements are scheduled to be accumulated, there was a problem that the difficulty in assembling receiving circuits is bedevilled.

[Citation List]

[Patent Literature]

[PTL 1]JP2007-74263A

[Summary of Invention]

[Technical Problem]

In view of the above-mentioned problems, an objective of the present invention is to provide AI semiconductor elements, which can replace earlier capacitive acoustic-elements and can realize higher sensibilities even if the sizes of the elements are reduced, and AEICs in each of which the reduced size AI semiconductor elements are used for achieving high-definition imaging, such that the AI semiconductor elements are merged, as at least a part of unit cells of the AEIC, at high integration density on a common substrate.

[Solution to Problem]

A first aspect of the present invention inheres in an acoustic induction-type (AI) semiconductor element encompassing (a) a channel-generating region made of semiconductor region of first conductivity type, being set to a first potential, (b) first and second main-electrode regions of second conductivity type which are disposed in a mutually facing manner, and separated from each other in the channel-generating region, (c) a vibration electrode set to a second potential, facing through a vibration-cavity to a top surface of the channel-generating region, and (d) a cavity-surrounding insulating-film surrounding the vibration-cavity so that the vibration-cavity is provided in a location between the channel-generating region and the vibration electrode, configured to implement a hermetically confined space with the vibration-cavity. In the AI semiconductor element pertaining to the first aspect of the present invention, displacements of the vibration electrode by ultrasonic waves are detected as changes of current flowing between the first and second main-electrode regions.

A second aspect of the present invention inheres in an acoustic element integrated circuit (AEIC) encompassing a plurality of unit cells which are arrayed on a common substrate. For at least a part of unit cells in an array of the AEIC pertaining to the second aspect of the present invention, the AI semiconductor element pertaining to the first aspect of the present invention is employed.

[Advantageous Effects of Invention]

According to the present invention, AI semiconductor elements, which can realize higher sensibilities even if the sizes of the elements are reduced, and AEIC scan be provided, wherein the reduced size AI semiconductor elements are used in each of AEICs for achieving the high-definition imaging, such that the AI semiconductor elements are merged, as at least a part of unit cells of the AEIC, on a common substrate at high integration density.

[Brief Description of Drawings]

FIG. 1 is a plan view illustrating an outline of a planar pattern of an element array in which AI semiconductor elements are arrayed, in a AEIC pertaining to a first embodiment of the present invention;
FIG. 2 is an enlarged plan view focusing a cell - -AI semiconductor element- - in i-th column and j-th row in the element array illustrated in FIG. 1;
FIG. 3 is a cross-sectional view of the AI semiconductor element pertaining to the first embodiment, taken from III-III direction in FIG. 2;
FIG. 4A is an equivalent circuit diagram of the AI semiconductor element pertaining to the first embodiment, when an output is taken out in a scheme of source follower;
FIG. 4B is an equivalent circuit diagram of the AI semiconductor element pertaining to the first embodiment, when current signals is taken out from a drain region side;
FIG. 4C is a large-signal equivalent circuit diagram to explain inner capacitances of an element, contrasting with a physical structure of an insulated-gate (IG) semiconductor element integrated as a part of the AI semiconductor element pertaining to the first embodiment illustrated in FIG. 3;
FIG. 5A is a cross-sectional view explaining the profiles of the deformed shapes of a vibration electrode and a vibration membrane when ultrasonic waves are entered to the AI semiconductor element of the first embodiment;

FIG. 5B is a cross-sectional view explaining the ideally deformed shapes of the vibration electrode and the vibration membrane when the ultrasonic waves are entered to the AI semiconductor element of the first embodiment;

FIG. 6A illustrates the various improved-schemes of the capacitive acoustic-elements to get close to the profile of the ideally deformed shape as illustrated in FIG. 5B, by using a plurality of curves as the results simulated for the effects due to the improved-schemes, comparing with the case of earlier scheme, respectively;

FIG. 6B is a cross-sectional view illustrating a curved capacitor structure implemented by double curved planes when a lower electrode side is defined as the curved topology, considering a practical situation that the ideal parallel-plate structure cannot be realized, in view of the simulated results illustrated in FIG. 6A;

FIG. 6C is a cross-sectional view explaining a structure of an AI semiconductor element pertaining to an improved structure of the first embodiment;

FIG. 7A is a process-flow cross-sectional view explaining a manufacturing method of the AI semiconductor element pertaining to the improved structure of the first embodiment illustrated in FIG. 6C;

FIG. 7B is a process-flow cross-sectional view explaining the manufacturing method of the AI semiconductor element pertaining to the improved structure of the first embodiment, which explains a process-step following FIG. 7A;

FIG. 7C is a process-flow cross-sectional view explaining the manufacturing method of the AI semiconductor element pertaining to the improved structure of the first embodiment, which explains a process-step following FIG. 7B;

FIG. 7D is a process-flow cross-sectional view explaining the manufacturing method of the AI semiconductor element pertaining to the improved structure of the first embodiment, which explains a process-step following FIG. 7C;

FIG. 7E is a process-flow cross-sectional view explaining the manufacturing method of the AI semiconductor element pertaining to the improved structure of the first embodiment, which explains a process-step following FIG. 7D;

FIG. 7F is a process-flow cross-sectional view explaining the manufacturing method of the AI semiconductor element pertaining to the improved structure of the first embodiment, which explains a process-step following FIG. 7E;

FIG. 7G is a process-flow cross-sectional view explaining the manufacturing method of the AI semiconductor element pertaining to the improved structure of the first embodiment, which explains a process-step following FIG. 7F;

FIG. 7H is a process-flow cross-sectional view explaining the manufacturing method of the AI semiconductor element pertaining to the improved structure of the first embodiment, which explains a process-step following FIG. 7G;

FIG. 7I is a process-flow cross-sectional view explaining the manufacturing method of the AI semiconductor element pertaining to the improved structure of the first embodiment, which explains a process-step following FIG. 7H;

FIG. 7J is a process-flow cross-sectional view explaining the manufacturing method of the AI semiconductor element pertaining to the improved structure of the first embodiment, which explains a process-step following FIG. 7I;

FIG. 7K is a process-flow cross-sectional view explaining the manufacturing method of the AI semiconductor element pertaining to the improved structure of the first embodiment, which explains a process-step following FIG. 7J;

FIG. 7L is a process-flow cross-sectional view explaining the manufacturing method of the AI semiconductor element pertaining to the improved structure of the first embodiment, which explains a process-step following FIG. 7K;

FIG. 7M is a process-flow cross-sectional view explaining the manufacturing method of the AI semiconductor element pertaining to the improved structure of the first embodiment, which explains a process-step following FIG. 7L;

FIG. 7N is a process-flow cross-sectional view explaining the manufacturing method of the AI semiconductor element pertaining to the improved structure of the first embodiment, which explains a process-step following FIG. 7M;

FIG. 7O is a process-flow cross-sectional view explaining the manufacturing method of the AI semiconductor element pertaining to the improved structure of the first embodiment, which explains a process-step following FIG. 7N;

FIG. 7P is a process-flow cross-sectional view explaining the manufacturing method of the AI semiconductor element pertaining to the improved structure of the first embodiment, which explains a process-step following FIG. 7O;

FIG. 7Q is a process-flow cross-sectional view explaining the manufacturing method of the AI semiconductor element pertaining to the improved structure of the first embodiment, which explains a process-step following FIG. 7P;

FIG. 7R is a process-flow cross-sectional view explaining the manufacturing method of the AI semiconductor element pertaining to the improved structure of the first embodiment, which explains a process-step following FIG. 7Q;

FIG. 8 is a cross-sectional view explaining a structure of an AI semiconductor element pertaining to a first variation of the first embodiment;

FIG. 9 is a plan view explaining a two-dimensional layout of AEIC pertaining to a second variation of the first embodiment;

FIG. 10 is a schematic view exemplifying an outline of a structure of a hydrophone, as an application example of the AEIC pertaining to the second variation of the first embodiment;

FIG. 11 is a plan view explaining a two-dimensional layout of AEICs pertaining to a third variation of the first embodiment in which the elements are divided into transmitting elements and receiving elements and arrayed;

FIG. 12 is an equivalent circuit diagram when an output is taken out in a scheme of source follower, in the receiving element in the AEIC pertaining to the third variation of the first embodiment;

FIG. 13 is a plan view of an AI semiconductor element pertaining to a second embodiment of the present invention;

FIG. 14 is a cross-sectional view of the AI semiconductor element pertaining to the second embodiment, taken from XIV-XIV direction in FIG. 13;

FIG. 15 is a cross-sectional view of the AI semiconductor element pertaining to the second embodiment, taken from XV-XV direction in FIG. 13;

FIG. 16 is a plan view of an AI semiconductor element pertaining to a third embodiment of the present invention;

FIG. 17 is a cross-sectional view of the AI semiconductor element pertaining to the third embodiment, taken from XVII-XVII direction in FIG. 16;

FIG. 18 is an equivalent circuit diagram of the AI semiconductor element pertaining to the third embodiment when current signals is taken out from a drain region side;

FIG. 19A (a) illustrates sensitivity characteristics for structures of divided and uniform flat-plate lower electrodes, when an epoxy resin protection-film is not coated on a vibration electrode, and FIG. 19A (b) illustrates sensitivity characteristics for the structures of the divided and uniform flat-plate lower electrodes, when the epoxy resin protection-film is coated on the vibration electrode;

FIG. 19B (a) illustrates dependence of sensitivity characteristics on values of delay resistors serving as ground resistors, and FIG. 19B (b) illustrates frequency dependence of energy-conversion efficiencies from ultrasonic wave to electricity;

FIG. 19C is an equivalent circuit of the AI semiconductor element pertaining to the third embodiment, generated by electro-mechano-acoustical (EMA) analogy;

FIG. 20A is a plan view of an AI semiconductor element pertaining to a first variation of the third embodiment of the present invention;

FIG. 20B (a) is an equivalent circuit diagram of the AI semiconductor element pertaining to the first variation of the third embodiment, FIG. 20B (b) is an equivalent circuit diagram of an AI semiconductor element pertaining to a second variation of the third embodiment, and FIG. 20B (c) is an equivalent circuit diagram of the AI semiconductor element pertaining to a first variation of the third embodiment;

FIG. 20C is a plan view of an AI semiconductor element pertaining to a fourth variation of the third embodiment of the present invention;

FIG. 20D is an equivalent circuit diagram of an AI semiconductor element pertaining to a fifth variation of the third embodiment;

FIG. 21 is a cross-sectional view of an AI semiconductor element pertaining to a fourth embodiment of the present invention;

FIG. 22A is a plan view of an AI semiconductor element pertaining to a first variation of the fourth embodiment;

FIG. 22B is a cross-sectional view of the AI semiconductor element pertaining to the first variation of the fourth embodiment, taken from XXIIB-XXIIB direction in FIG. 22A;

FIG. 23 is a cross-sectional view of an AI semiconductor element pertaining to a second variation of the fourth embodiment;

FIG. 24 is a cross-sectional view of an AI semiconductor element pertaining to a third variation of the fourth embodiment;

FIG. 25 is a cross-sectional view of an AI semiconductor element pertaining to a fourth variation of the fourth embodiment;

FIG. 26 illustrates bending rigidities of flat plates plotted for various materials, with values ($k/h^3$) which are obtained by dividing spring constants k by $h^3$ as ordinate and values of $Y/(1-\sigma^2)$ as abscissa;

FIG. 27 is a cross-sectional view of an AI semiconductor element pertaining to another embodiment of the present invention; and

FIG. 28 is a schematic view explaining a vibration profile of a vibration membrane of an earlier capacitive acoustic-elements.

[Description of Embodiments]

Hereinafter, first to fourth embodiments of the present invention will be described with reference to the drawings. In the following description of the drawings for the first to fourth embodiments, the same or similar parts are denoted by the same or similar reference numerals. However, it should be noted that the drawings are schematic, the relationship between the thickness and the planar dimension, the ratio of the size of each member, and the like may be different from the practical one. Therefore, specific thicknesses, dimensions, sizes, and the like should be determined more variously by considering the gist of the technical ideas that can be understood from the following description. In addition, it should also be understood that the respective drawings are illustrated with the dimensional relationships and proportions different from each other.

In this specification, "a first main-electrode region" of an insulated-gate (IG) semiconductor element constructing the basic structure of the AI semiconductor element means a semiconductor region serving as any one of a source region or a drain region in a field effect transistor (FET), a static induction transistor (SIT) or a new transistor structure equivalent to the above transistors. In an insulated gate bipolar transistor (IGBT) or a semiconductor element of new structure equivalent to the IGBT, the first main-electrode region means a semiconductor region serving as one of emitter or collector region. Also, in an insulated gate thyristor such as an MIS-controlled static induction thyristor (SI thyristor) or a semiconductor element of new structure equivalent to the insulated gate thyristor, the first main-electrode region means a semiconductor region serving as one of anode or cathode region. "A second main-electrode region 15" means a semiconductor region serving as one of the source or drain region that does not become the above first main-electrode region in the FET, SIT, etc. "The second main-electrode region 15a" means a region serving as one of the emitter or collector region that does not become the above first main-electrode region in the IGBT, etc. "The second main-electrode region 15a" means a semiconductor region serving as one of anode or cathode region that does not become the above first main-electrode region in the MIS-controlled SI thyristor, etc.

In this way, if "the first main-electrode region" is the source region, "the second main-electrode region 15a" means the drain region, and "a main current" flows between the first and second main-electrode regions 15a. If "the first main-electrode region" is the emitter region, "the second main-electrode region 15a" means the collector region. If "the first main-electrode region" means the anode region, "the second main-electrode region 15a" means the cathode region. When a bias relation is exchanged, in a case of MISFET, etc., there is a case that a function of "the first main-electrode region" and a function of "the second main-electrode region 15a" can be exchanged with each other. Moreover, in this specification, when an abbreviated term of "a main-electrode region" is simply used, the abbreviated term is an inclusive expression meaning one of the technically proper first main-electrode region or second main-electrode region 15a.

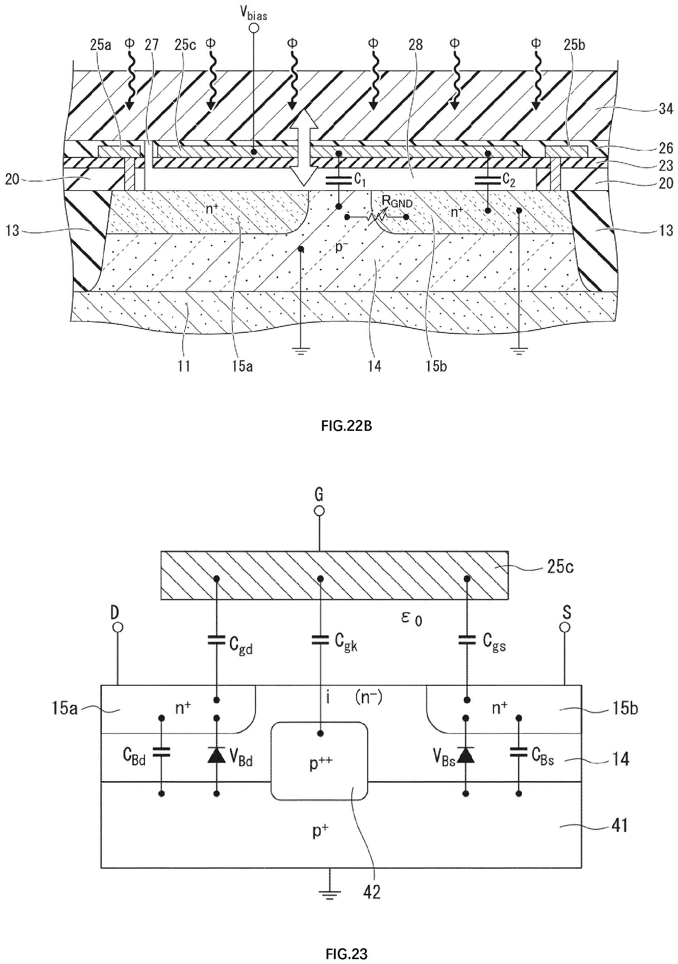

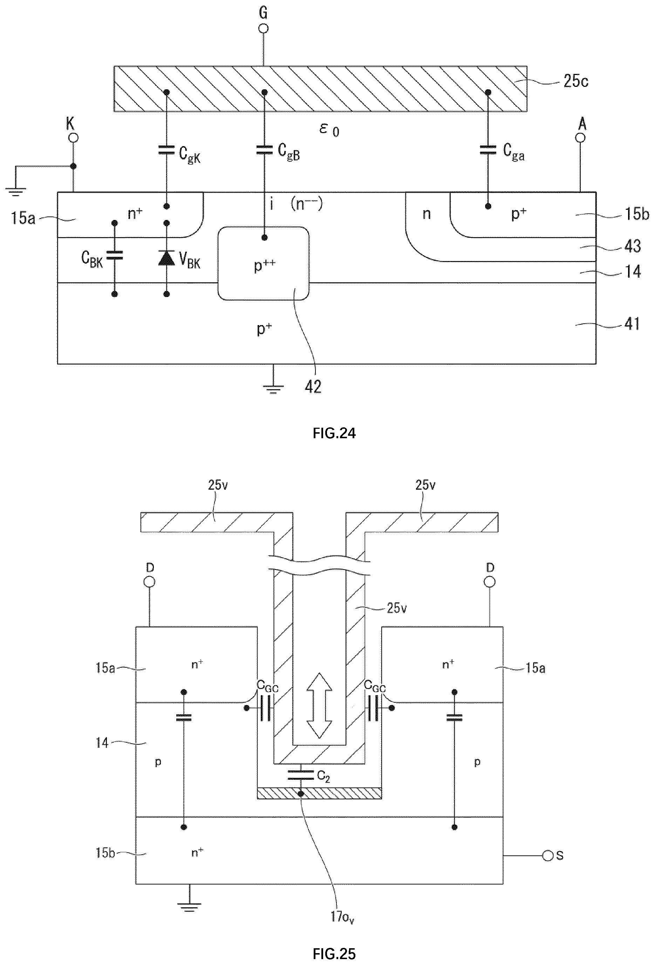

Also, in the following explanations, the definitions of words of "a top surface", "a lower portion", "upper", "lower" or the like, or the further definitions of directions using "upper" or "lower" are the definitions for convenience of mere explanation, and do not limit the technical ideas of the present invention. For example, it is natural that, when a target is rotated by 90° and observed, an upper-lower direction is read while being converted into a left-right direction, and when the target is rotated by 180° and observed, the upper-lower direction is read while being inverted. Also, in the following explanations, a case in which a first conductivity type is p-type and a second conductivity type is n-type is exemplarily explained. However, the first conductivity type can be defined as the p-type and the second conductivity type can be defined as the n-type, by selecting the conductivity type in a reverse relationship. Also, "+" and "-" which are attached as superscript to "n" and "p", respectively, mean the semiconductor regions whose impurity concentrations are relatively high or low, respectively, contrasting with the semiconductor regions to which "+" and "-" are not attached. Moreover, as illustrated in FIGs. 23, 24, etc., "++" attached as superscript to "p" means a semiconductor region to which p-type impurities are doped at very high impurity concentrations, close to the solid solubility limit concentration. However, even in a case of the semiconductor region to which the same "+" and "-" are attached as superscript or the semiconductor region to which the same "+" and "-" are not attached, the expression does not mean that the impurity concentrations of the respective semiconductor regions are strictly identical to each other.

### (FIRST EMBODIMENT)

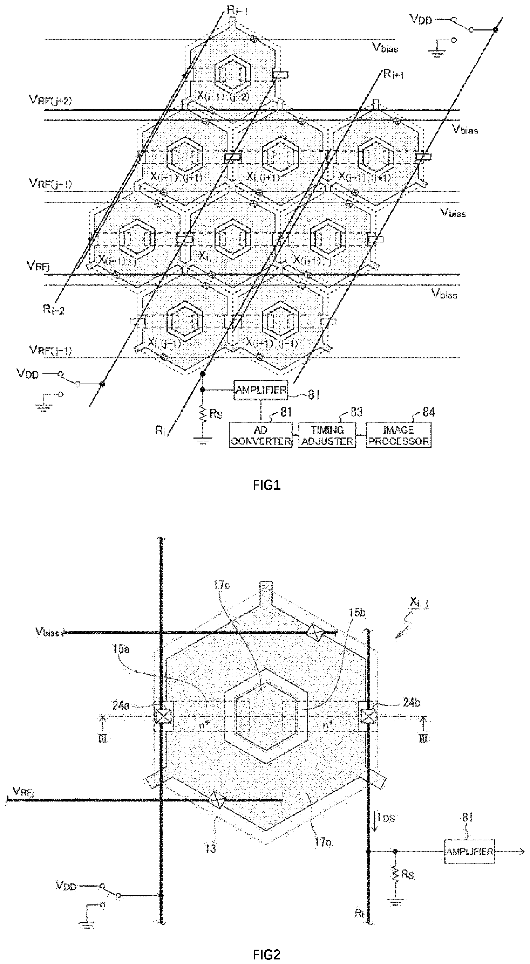

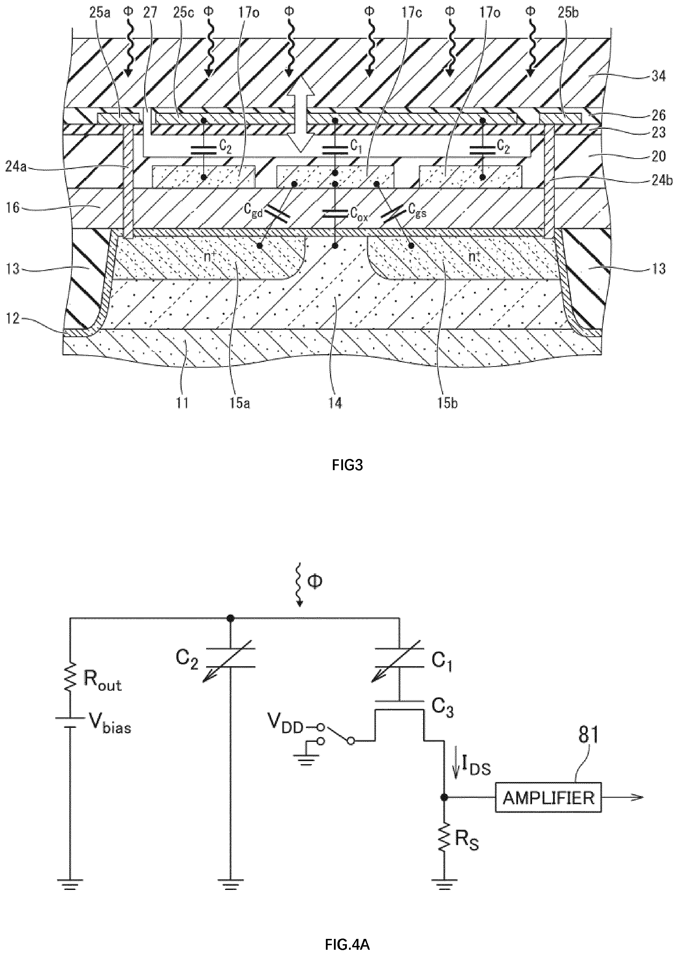

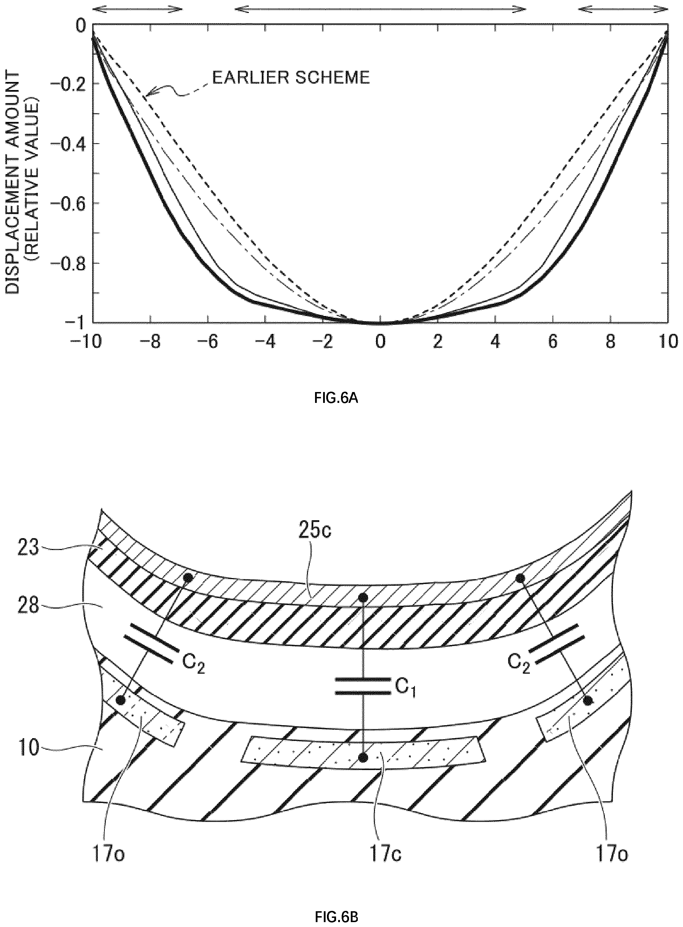

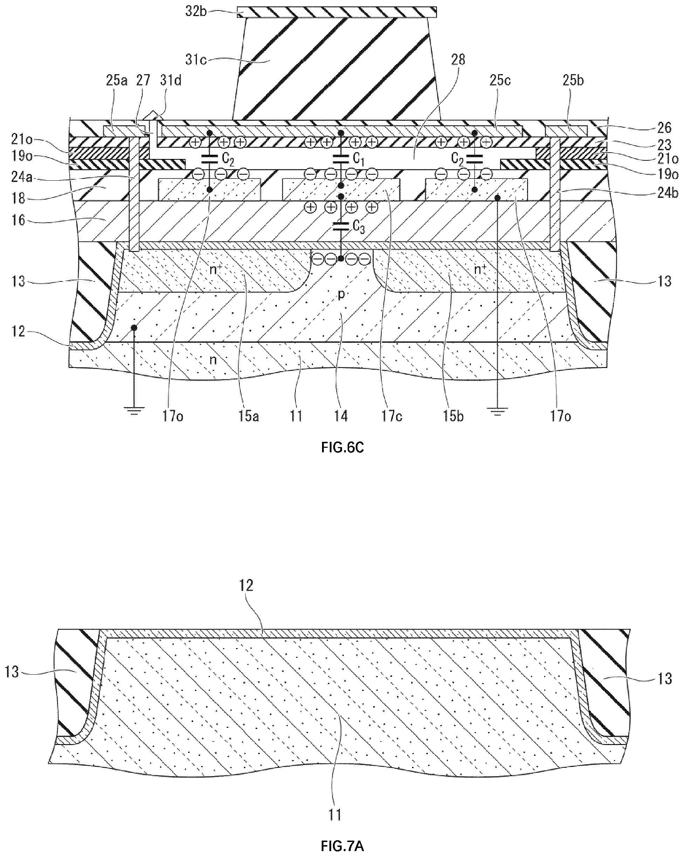

As illustrated in FIG. 1, a AEIC pertaining to a first embodiment of the present invention exhibits a planar layout constructing a two-dimensional(2D) matrix in which in an element-array portion, hexagonal unit cells $X_{(i-1),(j+2)}$, ---, $X_{(i-1),(j+1)}$, $X_{i,(j+1)}$, $X_{(i+1),(j+1)}$, ---, $X_{(i-1),j}$, $X_{i,j}$, $X_{(i+1)j}$, ---, $X_{i,(j-1)}$, $X_{(i+1),(j-1)}$, --- etc., are arrayed on a coplanar surface (on a same curved surface). When focusing to a single unit cell $X_{i,j}$ for inclusively representing, each of the unit cells $X_{i,j}$ corresponds to "an AI semiconductor element" whose new operational principle is introduced in the specification. As described later by referring to FIGs. 3 and 6, etc., each of the hexagonal unit cells $X_{(i-1),(j+2)}$, ---, $X_{(i-1),(j+1)}$, $X_{i,(j+1)}$, $X_{(i+1),(j+1)}$, ---, $X_{(i-1),j}$, $X_{i,j}$, $X_{(i+1),j}$, ---, $X_{i,(j-1)}$, $X_{(i+1),(j-1)}$, implementing the AEIC pertaining to the first embodiment encompasses a surface (main-surface) of a common base-body 11 having a flat main-surface made of semiconductor substrate, and a structure on the semiconductor surface.

In addition, "the coplanar surface" means a curved surface whose Gaussian curvature and average curvature are both zero (radius of curvature= infinity). However, more generally, the coplanar surface may be a same curved surface such as a cylindrical surface, a spherical surface, an elliptical surface, or a parabolic surface. In the explanation of the AEIC pertaining to the first embodiment on and after FIG. 2, the explanation focusing to the unit cell $X_{i,j}$ is mainly described. Also, FIG. 1 illustrates a case in which the planar pattern of the unit cell $X_{i,j}$, etc., is hexagonal. However, the planar pattern of the unit cell $X_{i,j}$, etc., is not limited to a hexagon. Then, various planar patterns such as a rectangle, an octagon, etc., can be adopted.

As will be described later by referring to FIG. 3, FIG. 6C, etc., in each of the hexagonal unit cells $X_{(i-1),(j+2)}$, ---, $X_{(i-1),(j+1)}$, $X_{i,(j+1)}$, $X_{(i+1),(j+1)}$, ---, $X_{(i-1),j}$, $X_{i,j}$, $X_{(i+1),j}$, ---, $X_{i,(j-1)}$, $X_{(i+1),(j-1)}$, implementing the AEIC pertaining to the first embodiment, a top surface of the common base-body 11 is separated into channel-generating regions 14, which are isolated from each other by element-isolation insulating-films 13, and the channel-generating regions 14 as the base-structures are two-dimensionally arrayed in the top surface of the common base-body 11. Moreover, an enclosure structure or a housing of the AI semiconductor element, similar to the housing of the capacitive acoustic-element, as a whole monolithic structure with a vibration-cavity 28is provided on each of the channel-generating regions 14. When the channel-generating region 14 is assumed to be a p-type semiconductor region, the channel-generating region 14 is the member having the structure and function corresponding to a p-well in the normal semiconductor integrated circuit. As illustrated in a cross-sectional view of FIG. 3, etc., on the surface of the channel-generating region 14 of a first conductivity type, a first main-electrode region 15b of a second conductivity type and a second main-electrode region 15a are arranged, which implements an IG semiconductor element.

The technical idea of the earlier capacitive acoustic-elements detects the variations of charges induced into capacitance between the upper and lower electrodes. However, as will be described later by using FIG. 6A, etc., in the earlier method of the capacitive acoustic-element to detect the changes of the charges accumulated in the capacitance between the upper and lower electrodes, there is a limitation in improving the detection sensitivity. Therefore, the inventors of the present invention, braking away from the earlier architecture of the capacitive acoustic-element to detect the variation of charges induced in the capacitance, merges an IG semiconductor element as an inner structure of the AI semiconductor element. That is, focusing to the charges induced in the surface of a semiconductor region by Gauss theorem and the potential changes associated with the induced charges, the inventors of the present invention have concentrated attentions on the changes in the capacitances by entered ultrasonic waves, by incorporating the IG semiconductor element as built-in structure. That is, the height of surface potential of semiconductor region is controlled by the charges induced in the surface of the semiconductor region. The inventors of the present invention propose the structure and operational principle of a new semiconductor element as an AI semiconductor element pertaining to the first embodiment and an AEIC using the AI semiconductor element. In the new semiconductor element, the potential-barrier height of a channel is controlled by changes of the capacitance caused by entered ultrasonic waves, a current of the new semiconductor element flows through the channel. And accordingly, the received ultrasonic waves are detected as the changes in the current flowing through the channel of the new semiconductor element.

Although reference numerals 15a and 15b are omitted in the plan view in FIG. 1, two rectangles illustrated by concealed lines inside the respective hexagons illustrate the first and second main-electrode regions making up the IG semiconductor elements, respectively. The two rectangles opposite to each other are arranged in each of the hexagonal unit cells $X_{(i-1),(j+2)}$, ---, $X_{(i-1),(j+1)}$, $X_{i,(j+1)}$, $X_{(i+1),(j+1)}$, ---, $X_{(i-1),j}$, $X_{i,j}$, $X_{(i+1),j}$, ---, $X_{i,(j-1)}$, $X_{(i+1),(j-1)}$, --- so that edges of the two rectangles bite into inner areas of the inner hexagon from the outer donut-shaped hexagon (hexagonal ring). In the AEIC pertaining to the first embodiment, the changes in capacitance are detected as the changes in current flowing between the first main-electrode region 15b and the second main-electrode region 15a.

For each of the two-dimensionally-arrayed IG semiconductor elements, configured to take out current signals from the first main-electrode region 15b or second main-electrode region 15a, vertical output-signal lines $R_{(i-1)}$, $R_i$, $R_{(i+1)}$, --- are connected to respective cells. The vertical output-signal lines $R_{(i-1)}$, $R_i$, $R_{(i+1)}$, --- are connected to first contact plugs provided on the left sides or right sides of the respective cells of the hexagon. For example, with the (i-1)-th column as an even column, the vertical output-signal line$R_{(i-1)}$ on the (i-1)-th column is connected to the first contact plug provided on the left side of the hexagon of each of the unit cells $X_{(i-1),(j+1)}$, $X_{(i-1),j}$, $X_{(i+1),(j-1)}$, --- on the (i-1)-th column. Similarly, the vertical output-signal line$R_{(i+1)}$ on the (i+1)-th column serving as the even column is connected to the first contact plug provided on the left side of the hexagon of each of the unit cells $X_{(i+1),(j+1)}$, $X_{(i+1),j}$, $X_{(i+1),(j-1)}$, --- on the (i+1)-th column.

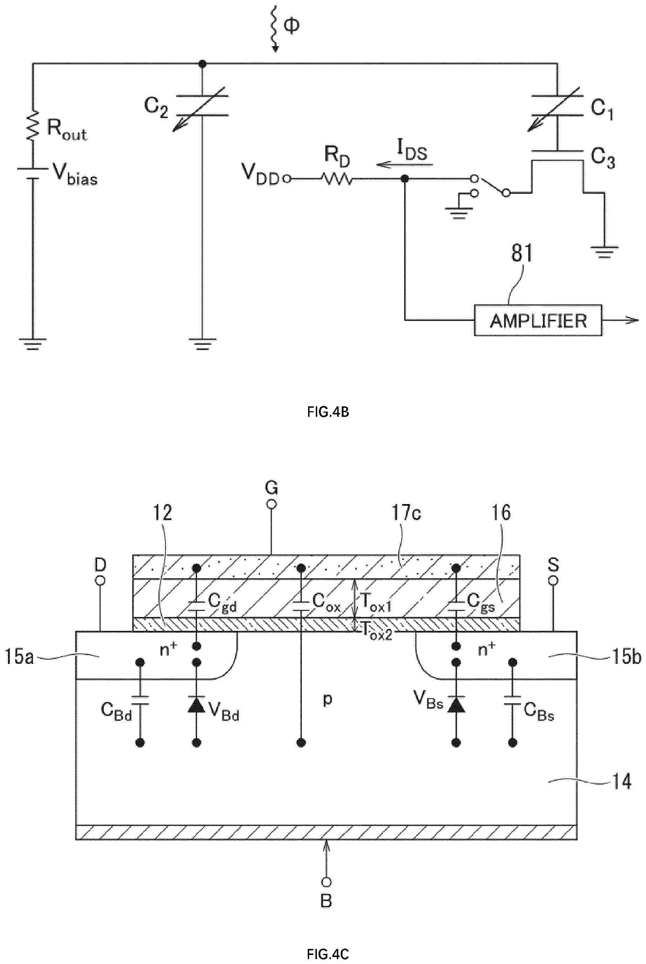

Against an array on the even column, the vertical output-signal line $R_i$ on the i-th column serving as an odd column is connected to the first contact plug provided on the right side of the hexagon in each of the unit cells $X_{i,(j+1)}$, $X_{i,j}$, $X_{i,(j-1)}$, --- on the i-th column. Although the illustration of the cell is omitted, even the vertical output-signal line $R_{(i-2)}$ on the (i-2)-th column is connected to the first contact plug provided on the right side of each of the hexagons. FIG. 1 exemplifies a case in which the current signals are read out in source-follower scheme using a source resistor $R_s$, from the first main-electrode region 15b (source region) of the IG semiconductor element, which is embedded as a partial structure of the AI semiconductor element. However, the circuit topology is not limited to the above configuration. For example, a configuration in which a source region is grounded (defined as a first potential) and the current signals are read out from the side of the second main-electrode region (drain region) 15a is allowed as illustrated in FIG. 4B.

FIG. 4A is an equivalent circuit diagram in a circuit topology of the source-follower scheme, and FIG. 4B is an equivalent circuit diagram a circuit topology that current signals are taken out from a drain region side. FIG. 1 exemplifies a case in which an output of the vertical output-signal lines $R_i$is supplied to an amplifier 81, an output of the amplifier 81 is transferred to an AD converter 82, an output of the AD converter 82 is transferred to a timing adjuster 83, and an output of the timing adjuster 83 is transferred to an image processor 84. Although illustration is omitted, for the other vertical output-signal lines $R_{(i-1)}$, $R_{(i+1)}$, ---, the similar circuit is connected.

To drive the IG semiconductor element provided in each of the channel-generating regions 14 in the respective cells, a power-supply interconnection $V_{DD}$is connected to the second main-electrode region 15a in the IG semiconductor element. As illustrated in FIG. 1, the power-supply interconnection $V_{DD}$ is connected to a second contact plug provided on the right side or left side of each cell in the hexagon. For example, the power-supply interconnection $V_{DD}$ on the (i-1)-th column in the even column is connected to a second contact plug provided on the right side of the hexagon in each of the unit cells $X_{(i-1),(j+1)}$, $X_{(i-1),j}$, $X_{(i-1),(j-1)}$, --- on the (i-1)-th column. The power-supply interconnection $V_{DD}$ on the (i-1)-th column in the even column becomes the power-supply interconnection shared with the power-supply interconnection $V_{DD}$ on the i-th column in the odd column. Then, the shared power-supply interconnection is connected to the second contact plug provided on the left side of the hexagon in each of the unit cells $X_{i,(j+1)}$, $X_{i,j}$, $X_{i,(j-1)}$,-- on the i-th column in the odd column.

Similarly, the power-supply interconnection $V_{DD}$ on the (i+1)-th column in the even column is connected to the second contact plug provided on the right side of the hexagon in each of the unit cells $X_{(i+1),(j+1)}$, $X_{(i+1),j}$, $X_{(i+1),(j-1)}$, --- on the (i+1)-th column. Although illustration is omitted, the power-supply interconnection $V_{DD}$ on the (i+1)-th column in the even column becomes the power-supply interconnection shared with the power-supply interconnection $V_{DD}$ on the (i+2)-th column in the odd column. Then, the shared power-supply interconnection is connected to the second contact plug provided on the left side of the hexagon in each of the cells on the (i+2)-th column in the odd column. In addition, the respective cells implementing the AEIC pertaining to the first embodiment are bidirectional acoustic-elements capable of transmission and reception. Thus, although an operating voltage $V_{DD}$ is supplied from the power-supply interconnection $V_{DD}$ at a time of receiving, the power-supply interconnection $V_{DD}$ is fixed (grounded) to a first potential at a time of transmitting.

In addition, depending on the shape of the hexagonal cell, FIG. 1 illustrates a layout of wiring in which the vertical output-signal line $R_i$ on the i-th column and the power-supply interconnection $V_{DD}$ on the i-th column run obliquely downward to the left. However, the layout is merely an exemplification. For example, for the exemplified vertical output-signal line $R_i$ and power-supply interconnection $V_{DD}$, it is allowed to adopt a topology in which for each cell on respective rows intersecting each other in running directions, the running directions are reversed one after another and meander in a zigzag pattern. Or, it is naturally possible to adopt various wirings differing from FIG. 1, such as a topology in which oblique rows and vertical rows meander in a zigzag pattern repeatedly along the side of the hexagon. In a case of zigzag running, a layout of wirings that run vertically can be realized, as the entire direction serving as the envelope line for the bent portions of the meandering.

As the bidirectional acoustic-element, configured to enable the operation of a transmitting mode, high-frequency signal-lines $V_{RF(j+2)}$, $V_{RF(j+1)}$, $V_{RFj}$, $V_{RF(j-1)}$, --- are connected to third contact plugs provided on an oblique latus at a low left portion in each cell of the hexagon. That is, the high-frequency signal-line $V_{RF(j+2)}$ on the (j+2)-th row is connected to the third contact plug of the unit cell $X_{(i-1),(j+2)}$, --- on the (j+2)-th row, and the high-frequency signal-line $V_{RF(j+1)}$ on the (j+1)-th row is connected to the third contact plug in each of the unit cells $X_{(i-1),(j+1)}$, $X_{i,(j+1)}$, $X_{(i+1),(j+1)}$ on the (j+1)-th row. Also, the high-frequency signal-line $V_{RFj}$ on the j-th row is connected to the third contact plug in each of the unit cells $X_{(i-1),j}$, $X_{i,j}$, $X_{(i+1),j}$ --- on the j-th row. Moreover, the high-frequency signal-line $V_{RF(j-1)}$ on the (j-1)-th row is connected to the third contact plug in each of the unit cells $X_{i,(j-1)}$, $X_{(i+1),(j-1)}$, --- on the (j-1)-th row.

On the other hand, as the bidirectional acoustic-element, configured to enable the operation of a receiving mode, a direct current(DC) bias-supply line $V_{bias}$ to supply a DC bias $V_{bias}$ of second potential between a vibration electrode and a fixed-potential electrode of each cell is connected to a fourth contact plug provided on an oblique latus at an upper right portion of each of the cells of the hexagon. That is, the DC bias-supply line $V_{bias}$ on the (j+2)-th row is connected to the fourth contact plug of the unit cells $X_{(i-1),(j+2)}$, --- on the (j+2)-th row, and the DC bias-supply line $V_{bias}$ on the (j+1)-th row is connected to the fourth contact plug in each of the unit cells $X_{(i-1),(j+1)}$, $X_{i,(j+1)}$, $X_{(i+1),(j+1)}$, --- on the (j+1)-th row. Also, the DC bias-supply line $V_{bias}$ on the j-th row is connected to the fourth contact plug in each of the unit cells $X_{(i-1),j}$, $X_{i,j}$, $X_{(i+1),j}$ on the j-th row. Moreover, the DC bias-supply line $V_{bias}$ on the (j-1)-th row is connected to the fourth contact plug in each of the unit cells $X_{i,(j-1)}$, $X_{(i+1),(j-1)}$, --- on the (j-1)-th row.

FIG. 2 illustrate an enlarged plan view taking a particular note to the unit cell $X_{i,j}$ on the i-column and j-row in the two-dimensional array of the AEIC pertaining to the first embodiment illustrated in FIG. 1 and a planar pattern of the unit cell $X_{i,j}$. The unit cell $X_{i,j}$ serving as the AI semiconductor element of the first embodiment encompasses a fixed-potential electrode17o of a perforated hexagon (hexagonal ring) and a hexagonal floating electrode 17c, which is arranged concentrically and spaced from the fixed-potential electrode17o, inside a hexagonal opening made in the centre of the fixed-potential electrode17o. The lower electrode (17c, 17o) of the unit cell $X_{i,j}$ serving as the capacitive acoustic-element encompasses the fixed-potential electrode17o and the floating electrode 17c. However, the floating electrode 17c is electrically floating, and the fixed-potential electrode17ois connected to the first potential (ground potential). In addition, FIG. 1 and FIG. 2 illustrate a structure in which the fixed-potential electrode17o and the floating electrode 17c are divided into concentrically hexagonal shapes. However, a topology in which the lower electrode (17c, 17o) is divided into the fixed-potential electrode17o and the floating electrode 17c is not limited to the pattern exemplified in FIG. 1 and FIG. 2.

For example, a configuration is allowed in which a hexagon is divided into two by a diagonal line passing through the centre of the hexagon, and one of the divided portions opposite to each other is made to serve as the fixed-potential electrode17o, and the other is made to serve as the floating electrode 17c. In the divided configuration, a topology in which a concave portion (recessed pattern) is made near the centre of the side opposite to the fixed-potential electrode17oin the floating electrode 17c, and a protrusion formed near the centre of the counterpart latus to the fixed-potential electrode17o is inserted into the concave portion can be adopted. Moreover, the lower electrode can be divided into many areas of three or more divisions, and each of the divided lower electrodes is classified to serve as the fixed-potential electrodes 17o and the floating electrodes 17c, and the areas having the same function may be electrically connected to each other. Even in a topology that the lower electrode is divided into the many areas, the areas made to serve as the fixed-potential electrodes 17oare connected to the first potential (ground potential), and the areas made to serve as the floating electrodes 17c are set to be electrically floating.

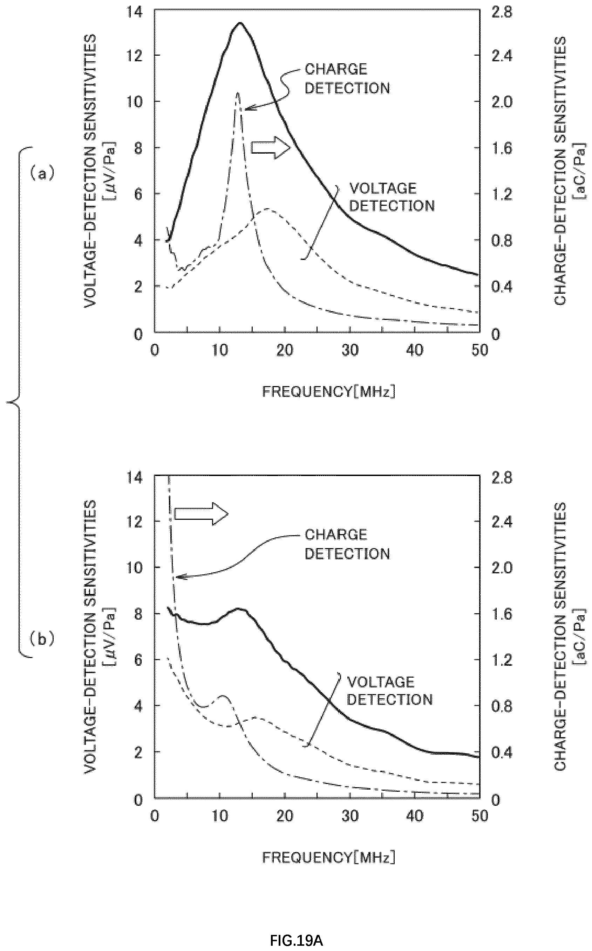

However, as will be described later by using FIG. 19A (a) and FIG. 19A (b) in the third embodiment, the topology in which the floating electrode 17c and the fixed-potential electrode 17o are concentrically arranged is preferable in the context of sensitivity characteristics. In view of the simulation-results illustrated in FIG. 19A (a) and FIG. 19A (b), even in the case of the divided patterns, a topology of double facing mirror-symmetric pattens which are generated by cutting the hexagon with the diagonal line passing through the centre of the hexagon, is preferable for the fixed-potential electrodes 17o. And, a concentric topology can be made, such that double mirror-symmetric concave portions, which are provided at each of the locations near the centre of mutual latera of the facing fixed-potential electrodes 17o, so that a pattern of an opening is formed by coupling of the double mirror-symmetric counterpart concave portions. And thereafter, the floating electrode 17cshall be inserted into the inside of the pattern of the opening formed by the mirror-symmetric concave portions.

In the unit cell $X_{i,j}$ as the AI semiconductor element of the first embodiment, the lower electrode (17c, 17o) having the topology of the divided electrodes is facing to the vibration electrode through a vibration-cavity 28 as illustrated in FIG. 3. Electric field between the fixed-potential electrode 17obuilding up the lower electrode (17c, 17o) and the vibration electrode keeps the predominant voltage at a transmission time and receiving mode of the unit cell $X_{i,j}$. On the other hand, as illustrated in double rectangles by concealed lines in FIG. 2, the first main-electrode region 15b and second main-electrode region 15a in the IG semiconductor element are arranged in which short latera are made facing to and separated from each other. The floating electrode 17c in the floating state, which implements the lower electrode (17c, 17o),acts to electro-statically control potentials in a channel region, generated at and in the surface of the channel-generating region 14 between the first main-electrode region 15b and the second main-electrode region 15a,by potentials generated via charges induced by the floating electrode 17c.

Two rectangular recesses (alcoves) are made as planar patterns around the outer circumference of the fixed-potential electrode 17odelineating the hexagonal ring, and the first and second contact plugs are arranged in each of the two recesses. That is, a first contact plug 24b is arranged inside the recess made on the right vertical latus of the planar pattern of the hexagon representing the outer circumference of the fixed-potential electrode 17o, and a second contact plug 24a is arranged inside the recess made on the left vertical latus. Moreover, a third contact plug is arranged on an oblique latus of the lower left portion of the hexagon representing the outer circumference of the fixed-potential electrode 17o, and a fourth contact plug is arranged on an oblique latus of the upper right portion.

As illustrated in FIG. 2, the first contact plug 24b is connected to the first main-electrode region 15b, configured to take out current signals from the first main-electrode region 15b implementing the IG semiconductor element in the unit cell $X_{i,j}$. Similarly to FIG. 1, the case is exemplified in which the current signals are read out in the scheme of source follower from the first main-electrode region (source region) 15b. However, a configuration is allowed in which the source region is grounded and the current signals are read out from the side of the second main-electrode region 15a (drain region) 15a. The second contact plug 24a is connected to the second main-electrode region 15a, to supply a power-supply voltage $V_{DD}$ from the power-supply interconnection $V_{DD}$ to the second main-electrode region 15a implementing the IG semiconductor element in the unit cell $X_{i,j}$. The third contact plug arranged on the oblique latus in the lower left portion supplies the high-frequency signal-line $V_{RFj}$ to the vibration electrode, when the bidirectional acoustic-element carries out an operation of a transmission mode. The fourth contact plug arranged on the oblique latus in the upper right portion supplies the DC bias $V_{bias}$ from the DC bias-supply line $V_{bias}$ to the vibration electrode, when the bidirectional acoustic-element carries out an operation of a receipt mode, and applies a desirable potential between the vibration electrode (upper electrode) and the lower electrode.

As illustrated in FIG. 3, the AI semiconductor element pertaining to the first embodiment encompasses the channel-generating region 14, which is made of a semiconductor region of first conductivity type (p-type) and set to the first potential (= ground potential GND), and the first main-electrode region 15b and the second main-electrode region 15a of second conductivity type (n-type) which are facing to and separated from each other on the surface of the channel-generating region 14. Moreover, the AI semiconductor element pertaining to the first embodiment encompasses gate-insulating films (12 and 16), which are laminated on the first main-electrode region 15b and the second main-electrode region 15a, and further laminated on the channel-generating region 14 sandwiched in between the first main-electrode region 15b and the second main-electrode region 15a, and the floating electrode 17c made of conductive layers formed as floating state, on the gate-insulating films (12 and 16), above the channel-generating region 14 sandwiched in between the first main-electrode region 15b and the second main-electrode region 15a, and accordingly implements IG semiconductor element. For the conductive layers used in the floating electrode 17c, conductive materials of low specific resistivity such as impurity-doped polycrystalline silicon (DOPOS) films, etc. can be employed. Regarding the gate-insulating film (12 and 16), a double-layer structure made of the first gate-insulating films 12 at a lower layer and the second gate-insulating film 16 at an upper layer is exemplified. However, the gate-insulating film (12 and 16) is not limited to the structure illustrated in FIG. 3.

And, as illustrated in FIG. 3, the fixed-potential electrode17o, which is arranged adjacently to the floating electrode 17c and separately from the floating electrode 17c and is made of the conductive layer set to the first potential GND, is further provided on the gate-insulating films (12 and 16). For the fixed-potential electrode17o, the same conductive layer as the floating electrode 17c, for example, DOPOS film of low specific resistivity can be adopted. The lower electrode (17c, 17o) of the divided structure is composed of the floating electrode 17c and the fixed-potential electrode17o. That is, the AI semiconductor encompasses a vibration-membrane 23, which is made of insulating film facing through the vibration-cavity to the lower electrode (17c, 17o) composed of the floating electrode 17c and the fixed-potential electrode17o, and a vibration electrode 25c, which is in contact with the top surface of the vibration-membrane 23. The vibration electrode 25faces to the floating electrode 17c and the fixed-potential electrode7othrough the vibration-cavity, and is set to second potential $V_{bias}$. As illustrated in FIG. 3, between the lower electrode (17c, 17o) and the vibration electrode 25c, a cavity-surrounding insulating-film 20 surrounding the vibration-cavity is formed in such a way that the vibration-cavity is enclosed as hermetically confined space. In the AI semiconductor element pertaining to the first embodiment, displacement of the vibration electrode 25c caused by ultrasonic waves Φ can be detected as the changes in the current flowing between the first main-electrode region 15b and the second main-electrode region 15a. In FIG. 3, a bidirectional white-arrow overlaid on the vibration electrode 25c mean the displacement of the vibration electrode 25c.

As illustrated in FIG. 3, a first surface-wiring layer 25b and a second surface-wiring layer 25 are laminated on the top surface of the vibration-membrane 23, in addition to the vibration electrode 25c. A first contact plug 24b, which penetrates through the vibration-membrane 23, the cavity-surrounding insulating-film 20, a second gate-insulating film 16 and a first gate-insulating film 12, is provided in a location between the first surface-wiring layer 25band the first main-electrode region 15b. Similarly, a second contact plug 24a, which penetrates through the vibration-membrane 23, the cavity-surrounding insulating-film 20, the second gate-insulating film 16 and the first gate-insulating film 12, is provided in a location between a second surface-wiring layer 25a and the second main-electrode region 15a. That is, the top end of the first contact plug 24b is metallurgically connected to the first surface-wiring layer 25b, and the bottom end of the first contact plug 24b is electrically connected to the first main-electrode region 15b. Thus, the first surface-wiring layer 25b and the first main-electrode region 15b are electrically connected through the first contact plug 24b.

Similarly, the top end of the second contact plug 24a is metallurgically connected to the second surface-wiring layer 25a, and the bottom end of the second contact plug 24a is electrically connected to the second main-electrode region 15a. Thus, the second surface-wiring layer 25a and the second main-electrode region 15a are electrically connected through the second contact plug 24a. The first surface-wiring layer 25b corresponds to the vertical output-signal line $R_i$ on the i-th column illustrated in FIG. 2. On the other hand, the second surface-wiring layer 25a corresponds to the power-supply interconnection $V_{DD}$ on the j-th row illustrated in FIG. 2. On the vibration-membrane 23, a vibration-electrode protection-film (first vibration-electrode protection -film) 26 such as silicon oxide film is coated so as to cover the vibration electrode 25c, the first surface-wiring layer 25b and the second surface-wiring layer 25. Moreover, a second vibration-electrode protection-film 34 such as polyimide film is stacked on the first vibration-electrode protection-film 26. The second vibration-electrode protection -film 34 closes a liquid-introduction canal 27 through which a removal liquid is introduced to selectively dissolve and remove a sacrificial layer by wet etching, at a step of forming the vibration-cavity 28. The liquid-introduction canal 27 penetrates the first vibration-electrode protection-film 26 and the vibration-membrane 23 and arrives at the vibration-cavity 28. After the liquid-introduction canal 27 is closed, the inside of the vibration-cavity 28 can be kept in an atmosphere of inert gas at reduced pressure.

FIG. 3 represents a capacitance as $C_1$, the capacitor $C_1$is provided between the vibration electrode 25c near the centre of the vibration-cavity 28 and the floating electrode 17cin the floating state. A capacitance value of the capacitor $C_1$ is a variable capacitor as illustrated in FIG. 4A and FIG. 4B, because the capacitor $C_1$changes depending on displacements of the vibration electrode 25c. Also, around the capacitor $C_1$, a couple of inter-electrode capacitances are expressed as $C_2$,the inter-electrode capacitors $C_2$ are generated between portions of the vibration electrode 25c, which surround the capacitor $C_1$ in hexagonal-ring as illustrated in FIG. 2, and counterpart portions of the fixed-potential electrode17oat ground potential (first potential) GND. Even the capacitors $C_2$arevariable capacitors as illustrated in FIG. 4A and FIG. 4B, because the capacitor $C_2$changes depending on the displacement of the vibration electrode 25c. To distinguish the two variable capacities, hereafter, the capacitor $C_1$ is called "a first variable capacitor $C_1$", and the capacitor $C_2$ is called "a second variable capacitor $C_2$". The second variable capacitor $C_2$ is a capacitance having a role equivalent to the capacitance being designed between the upper and lower electrodes in the earlier capacitive acoustic-elements, and the first variable capacitor $C_1$ is a specific capacitor that induces charges required to drive the IG semiconductor element specific to the AI semiconductor element of the first embodiment. Moreover, a gate-source capacitor $C_{gs}$ is indicated between the floating electrode 17c and the first main-electrode region 15b, a gate-drain capacitor $C_{gd}$ is indicated between the floating electrode 17c and the second main-electrode region 15a, and an insulating-film capacitor Cox is indicated between the floating electrode 17c and the channel-generating region 14.

Electric situation around the IG semiconductor element, the floating electrode 17c and the vibration electrode 25c, which implement the AI semiconductor element of the first embodiment illustrated in FIG. 3 can be expressed by equivalent circuits as illustrated in FIG. 4A and FIG. 4B when the above electric situation is expressed by equivalent electric circuits. A horizontal line illustrated on the top surface of the paper in FIG. 4A and FIG. 4B corresponds to the DC bias-supply line $V_{bias}$ for supplying the voltage of the second potential illustrated in FIG. 1 and FIG. 2. On the right of FIG. 4A, a series circuit of the first variable capacitor $C_1$ whose one end is connected to the DC bias-supply line $V_{bias}$ and an n-channel MOSFET (hereafter, called "nMOSFET") is illustrated. However, the nMOSFET is merely an exemplification, and various IG semiconductor elements explained in the beginning of the section of "Description of Embodiments" can be employed. In addition to the IG semiconductor elements explained in the beginning of the section of "Description of Embodiments", still another IG semiconductor elements such as a high electron mobility transistor (HEMT) can be employed. Because FIG. 4A is an equivalent circuit diagram when an output is taken out in the scheme of source follower, a source terminal of the nMOSFET is connected through a source resistor Rs to the first potential (ground potential), and a current output supplied from the source terminal is transmitted to the amplifier 81.

On the right in FIG. 4B, similarly to FIG. 4A, the series circuit encompassing the first variable capacitor $C_1$, whose one end is connected to the DC bias-supply line $V_{bias}$ of the second potential, and the nMOSFET is illustrated. However, the nMOSFET is merely an exemplification. Because FIG. 4B is an equivalent circuit diagram when current signals are taken out from a drain region side, a drain terminal of the nMOSFET is connected through the drain resistor $R_D$ to the power source $V_{DD}$, and a current output supplied from the drain terminal is transmitted to the amplifier 81. Both of FIG. 4A and FIG. 4B are implemented by parallel circuit respectively, each of the parallel circuits encompasses the second variable capacitor $C_2$ and the series circuit made of the first variable capacitor $C_1$ and the nMOSFET. The series circuit is connected in parallel to the second variable capacitor $C_2$, and the second variable capacitor $C_2$ is connected between the DC bias-supply line $V_{bias}$ of the second potential and the first potential (ground potential).

As explained in FIG. 4C, the gate-source capacitor $C_{gs}$, the gate-drain capacitor $C_{gd}$ and the insulating-film capacitor Cox are distributed around the floating electrode 17c, in the IG semiconductor element that is the nMOSFET. Roughly thinking, when approximation is performed under an assumption that a capacitor $C_3$ around the floating electrode 17c is Cox, the equivalent circuits in FIG. 4A and FIG. 4B can be expressed by using the capacitor $C_3$. A value of the capacitor $C_3$ that can be approximated by the insulating-film capacitor Cox, contrary to the first variable capacitor $C_1$ and the second variable capacitor $C_2$, is a fixed capacitor because the value of the capacitor $C_3$ is not changed even if the vibration electrode 25c is displaced. Thus, hereafter, the capacitor $C_3$ is called "a fixed capacitor $C_3$". For the sake of simplification, the fixed capacitor $C_3$ is assumed to be an inter-gate-substrate capacitance illustrated in FIG. 4C.

At first, in the series circuits of the first variable capacitor $C_1$ and the fixed capacitor $C_3$ which are illustrated in the equivalent circuits in FIG. 4A and FIG. 4B, voltages to be applied across the fixed capacitor $C_3$ are considered. When charges induced in the first variable capacitor $C_1$ are assumed to be $q_1$, voltages across the terminals of the first variable capacitor $C_1$ are assumed to be $V_1$, charges induced in the fixed capacitor $C_3$ are assumed to be $q_3$, and voltages across the terminals of the fixed capacitor $C_3$ are $V_3$, $q_1 = q_3 = q$ is established in the series circuit. Thus, a voltage $V_{bias}$ across the terminals of the series circuit made of the first variable capacitor $C_1$ and the fixed capacitor $C_3$ is expressed by the following Eq. (1).
[Equation 1]

$$V_{bias} = V_1 + V_3 = \frac{q}{C_1} + \frac{q}{C_3} = \frac{(C_1 + C_3)\, q}{C_1 C_3} \cdots\cdots\cdots (1)$$

In FIG. 3, an area of the floating electrode 17c is assumed to be S1, an inter-electrode distance between the vibration electrode 25c and the floating electrode 17c, which implement the first variable capacitor $C_1$, is assumed to be d, a thickness of the gate-insulating films (12 and 16) constructing the fixed capacitor $C_3$ is assumed to be tox (= $T_{OX1} + T_{OX2}$). Also, a vacuum permittivity is $\varepsilon_0$ and a dielectric constant of silicon oxide film is $\varepsilon_r$. Then, the following Eq. (2) and Eq. (3) are established.

$$C_1 = \varepsilon_0 S_1/d \qquad\qquad \cdots\cdots (2)$$

$$C_3 = \varepsilon_0 \varepsilon_r S_1/t_{OX} \qquad \cdots\cdots(3)$$

Thus, by calculating capacitive voltage division in the series circuit of the first variable capacitor $C_1$ and the fixed capacitor $C_3$, a voltage Vs across the fixed capacitor $C_3$ can be obtained according to the following Eq.(2).
[Equation 2]

$$\frac{V_3}{V_{bias}} = \frac{\dfrac{q}{C_3}}{\dfrac{(C_1 + C_3)\, q}{C_1 C_3}}$$

$$= \frac{C_1}{(C_1 + C_3)}$$

$$= \frac{\varepsilon_0 S_1 / d}{\varepsilon_0 S_1 / d + \varepsilon_0 \varepsilon_r S_1 / t_{OX}}$$

$$= \frac{1}{1 + d\,(\varepsilon_r / t_{OX})} \qquad \cdots\cdots (4)$$

For example, when the conditions that d = 10 nm, tox = 10 nm and a dielectric constant of silicon oxide film $\varepsilon_r$ = 4.0 are assumed, one-fifth of the DC bias-supply line $V_{bias}$ is applied to a voltage $V_3$ across the fixed capacitor $C_3$ can be understood from Eq. (4). Also, when the inter-electrode distance between the vibration electrode 25c and the floating electrode 17c is set to d = 10/4 nm = 2.5 nm, a half of the DC bias-supply line $V_{bias}$ is applied to the voltage Vsacross the fixed capacitor $C_3$ can be understood from Eq. (4). Thus, by selecting the DC bias-voltage $V_{bias}$ as a predetermined value, and accordingly adjusting a value of d( $\varepsilon_r/t_{OX}$) of Eq. (4)and then selecting the structure of the AI semiconductor element pertaining to the first embodiment, a gate voltage of the nMOSFET can be selected so that the nMOSFET is operated in a linear regime.

When the application of the ultrasonic waves will cause the vibration electrode 25c slightly displace, whether significant changes $\Delta V_3$ of the gate voltages of the nMOSFET, which correspond to minute displacements $\Delta$d of the vibration electrode 25c, can be obtained is an important concern, regarding the gate voltages of the nMOSFET. From Eq. (2), when the inter-electrode distance d between the vibration electrode 25c and the floating electrode 17c is displaced by infinitesimal displacement $\Delta$d, capacitance-variations $\Delta C_1$ of the capacitance value of the first variable capacitor $C_1$ can be expressed by Eq. (5).

$$C_1 + \Delta C_1 = \varepsilon_o S_1 / (d - \Delta d) \qquad (5)$$

On the other hand, Eq. (4) can be rewritten to the following Eq. (6).
[Equation 3]

$$V_3 = \frac{1}{1 + (\varepsilon_r / t_{OX})\, d}\, V_{bias} \qquad \cdots\cdots (6)$$

When a multivariable function of Eq. (6) is partially differentiated with respect to $\partial$ d, the following Eq. (7) can be obtained. Eq. (7) is the multivariable function having the variable d, the variable tox, etc. [Equation 4]

$$\frac{\partial V_3}{\partial d} = -\left(\frac{\varepsilon_r}{t_{OX}}\right)\frac{1}{\left(1 + (\varepsilon_r / t_{OX})\, d\right)^2}\, V_{bias} \qquad \cdots\cdots (7)$$

Taking note on the variable d, and when the inter-electrode distance d between the vibration electrode 25c and the floating electrode 17cchangesby infinitesimal displacement $\Delta$d, change $\Delta V_3$ of the gate voltage of the nMOSFET can be expressed by the form of the following Eq. (8).
[Equation 5]

$$\Delta V_3 = -\left(\frac{\varepsilon_r}{t_{OX}}\right)\frac{1}{\left(1 + (\varepsilon_r / t_{OX})\, d\right)^2}\, V_{bias}\, \Delta d \qquad \cdots\cdots (8)$$

From Eq. (8), for increasing the change $\Delta V_3$ of the gate voltage when the inter-electrode distance d is displaced by infinitesimal displacement $\Delta d$, it can be understood that the specific dielectric constant $\varepsilon_r$ of the gate-insulating film shall be made large, the film thickness tox of the gate-insulating film shall be made thin, and the inter-electrode distance d shall be made thin, and further, the DC bias-supply line $V_{bias}$ of the second potential shall be made large. In the earlier capacitive acoustic-elements, the changes of the charges that are extensive variables are detected. Therefore, ascribable to the extensive variable, because values per element will become smaller and smaller by element miniaturization, when the size of each element is reduced for realizing the high-definition imaging, the assembling difficulty is dogged by, incorporating the elements into a receiving circuit, with the earlier capacitive acoustic-elements.

On the other hand, according to the AI semiconductor element of the first embodiment as exemplified in FIG. 3, because changes of voltages that are intensive variables are generated in the floating electrode 17c, and the intensive variables are detected as represented by Eq. (8), even if the element size is reduced, the voltage values will not be made small. Because Eq. (8) represents the multivariable function, when a value of $\varepsilon_r/t_{OX}$ is looked at as a variable, the absolute value of Eq. (8) represents a function of convex upward to have a maximum value at $\varepsilon_r/t_{OX}$= 1/d. That is, because a functional value representing absolute values of Eq. (8) becomes maximum when the film thickness tox of the gate-insulating film has a value at which the inter-electrode distance d is multiplied by the specific dielectric constant ($\varepsilon_r$),the film thickness tox of the gate-insulating film shall be thinned depending on the inter-electrode distance d. However, in thinning the film thickness tox of the gate-insulating film and shortening the inter-electrode distance d, there are limitations in terms of manufacturing technology and physical property. In view of Eq. (8), for increasing the change $\Delta V_3$ of the gate voltages with respect to the change of $\Delta d$, it is known that increasing the DC bias-supply line $V_{bias}$ is effective.

Already, a situation is explained in which operational points of the gate voltages can be selected in such a way that the nMOSFET is operated in the linear regime by selecting the DC bias-voltage line $V_{bias}$ which is the second potential, by using Eq. (4). If the DC bias-voltage $V_{bias}$ is excessively increased from the request of Eq. (8), the operational point is outside the linear regime. Insulated-gate SIT such as MOSSIT, MISSIT, etc., which represents the drain voltage - drain current characteristics of vacuum tube triode, is preferred as the IG semiconductor element adopted for the AI semiconductor element pertaining to the first embodiment, because all the operational regimes belong to the linear regime.

With mutual conductance $g_m$ of MOSFET, changes $\Delta I_{ds}$ of drain current $I_{ds}$ can be expressed by the following Eq. (9).

[Equation 6]

$$\Delta I_{DS} = - g_m\left(\frac{\varepsilon_r}{t_{OX}}\right) \frac{1}{(1+(\varepsilon_r / t_{OX})d)^2} V_{bias} \Delta d \quad \cdots\cdots (9)$$

That is, according to the AI semiconductor element of the first embodiment, since the changes $\Delta V_3$ of the received voltages as indicated by Eq. (8) in each element is used as the changes of the gate voltages of the IG semiconductor element built in each element, it can be detected as large changes-of current by using an amplifying function of the IG semiconductor element. Thus, the high-definition imaging can be achieved by arraying the AI semiconductor elements, which are high in detection sensitivity, as minute unit cells.

By incorporating the IG semiconductor elements such as nMOSFET, etc., as the components of the AI semiconductor elements of the first embodiment, it is known that the IG semiconductor elements can be driven by the voltages determined by the capacitive voltage division at the floating electrode 17c, and the displacement of the vibration electrode 25c can be detected as the changes-of current $\Delta I_{ds}$. The values of the changes $\Delta V_3$ of the gate voltages of the nMOSFET indicated by Eq. (7) is a function that decreases when the inter-electrode distance d between the vibration electrode 25c and the floating electrode 17c is increased. Thus, the shorter distance is desirable for the inter-electrode distance d. On the other hand, as already explained, when the thickness tox of the gate-insulating film is elected as the variable and considered, the function representing the absolute value of Eq. (7) exhibits an upward convex curve, and the convex curve has a maximum value.

Thus, when the curve shapes of the multivariable functions representing the absolute values of the changes $\Delta V_3$ of the gate voltages are considered with the thicknesses tox as the variables, the optimal value based on the inter-electrode distance d exists in the thicknesses tox of the gate-insulating film. The breakdown voltage of the gate-insulating film becomes low, when the inter-electrode distance d becomes short and the thickness tox of the gate-insulating film as the optimal value is accordingly thinned. So, the existence of the optimal value of the thicknesses tox of the gate-insulating film, the thicknesses tox depend on the inter-electrode distances d, is explained. Namely, when the reciprocal of the thickness tox of the gate-insulating film that is normalized by the specific dielectric constant $\varepsilon_r$ of the gate-insulating film is taken as ($\varepsilon_r/t_{OX}$) = x, the multivariable function of Eq. (7) can be rewritten to the following Eq. (10). [Equation 7]

$$\frac{\partial V_3}{\partial d} = -x \frac{1}{(1+dx)^2} V_{bias} = -V_{bias} \frac{f(x)}{g(x)} \qquad \cdots\cdots (10)$$

In Eq. (10), f(x) = x and g(x) = ((1+dx)$^2$. When the derivative function of the differential of f(x) with respect to x is expressed by $f_x(x)$, and the derivative function of the differential of g(x) with respect to x is expressed by $g_x(x)$, the following Eq. (12) is established:

$$f_x(x) = 1 \qquad \cdots\cdots (11)$$

$$g_x(x) = 2d+2d^2x = 2d(1+dx) \qquad \cdots\cdots (12).$$

Then, the following Eq. (13) is established:

$$f_x(x)g(x)\text{-}f(x)g_x(x) = (1+dx)^2\text{-}2xd(1+dx)$$
$$= 1\text{-}d^2x^2 \qquad \cdots\cdots (13).$$

With Eq. (13), the result when the multivariable function of Eq. (7) is partially differentiated with respect to the variable x is expressed by the following Eq. (14).
[Equation 8]

$$\frac{\partial^2 V_3}{\partial x \partial d} = -\frac{1-d^2x^2}{(1+dx)^4} V_{bias} \qquad \cdots\cdots (14)$$

That is, the multivariable function representing the absolute values of the changes $\Delta V_3$ of the gate voltages indicated by Eq. (7) and Eq. (10) will have the maximum value at $d^2x^2 = 1$ when the variable x is noticed.

The $d^2x^2 = 1$ which makes the value of Eq. (14) zero is equivalent to dx = 1. Thus, the multivariable function representing the absolute values of the changes $\Delta V_3$ of the gate voltages represented by Eq. (7) and Eq. (10) has the maximum value when the following Eq. (15) is established:

$$d/\varepsilon_o = t_{OX}/\varepsilon_o\varepsilon_r \qquad \cdots\cdots (15).$$

Therefore, the function representing the absolute values of the changes $\Delta V_3$ of the gate voltages of the nMOSFET indicated by Eq. (7) has the maximum value, when the value of the first variable capacitor $C_1$ is equal to the value of the fixed capacitor $C_3$ as can be understood from Eq. (2) and Eq. (3).

FIG. 4C illustrates a large-signal equivalent circuit comparing with a general nMOSFET, with regard to a structure in which an n$^+$-type source region (first main-electrode region) 15b and an n$^+$-type drain region (second main-electrode region) 15a are arranged, facing to each other via the surface of the channel-generating region 14, on the surface of the p-type channel-generating region 14 illustrated in FIG. 3. Contrasting with the structure illustrated in FIG. 3, even FIG. 4C illustrates a structure in which on the channel region, the source region 15b and the drain region 15a, a floating electrode 17c serving as a gate electrode is stacked via a double-layer gate-insulating film (12 and 16) made of a first gate-insulating film 12 having a thickness of $t_{OX2}$ and a second gate-insulating film 16 having a thickness of $t_{OX1}$. A source electrode S is connected to the source region 15b, and a drain electrode D is connected to the drain region 15a, respectively.

Similarly to the general nMOSFET, as illustrated in FIG. 4C, the gate-source capacitor $C_{gs}$ exists between the floating electrode 17c serving as the gate electrode and the first main-electrode region 15b, the gate-drain capacitor $C_{gd}$ exists between the floating electrode 17c and the second main-electrode region 15a, and the gate-substrate capacitor Cox exists between the floating electrode 17c and the channel-generating region 14. Moreover, a source-substrate capacitor $C_{Bs}$ exists between the first main-electrode region 15b and the channel-generating region 14, and a drain-substrate capacitor $C_{Bd}$ exists between the second main-electrode region 15a and the channel-generating region 14.

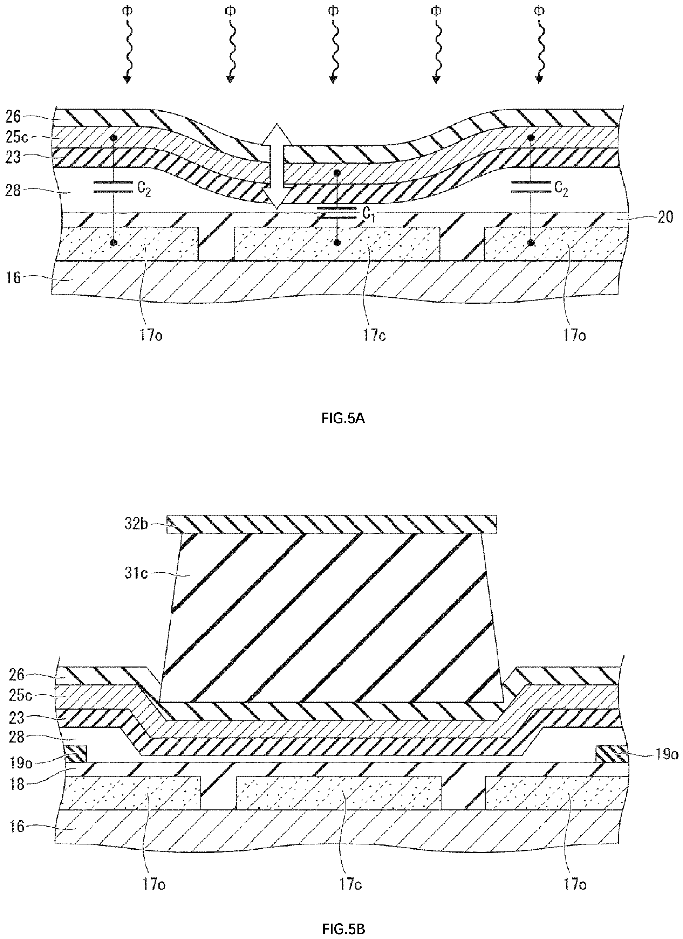

FIG. 5A illustrates the profiles of the deformed shapes of the vibration electrode 25c and the vibration-membrane 23 when the ultrasonic waves Φ are irradiated to the AI semiconductor element of the first embodiment. As already explained by using FIG. 28, in the structure of the AI semiconductor element, since the flexure of the vibration-membrane 23 is not uniform, the vibration electrode 25c and the floating electrode 17c come close to each other only within the range near the centre of the vibration-cavity 28. Therefore, as illustrated in FIG. 5A, an inter-electrode distance between the vibration electrode 25c and the fixed-potential electrode17o at the positions around the floating electrode 17c is wider than an inter-electrode distance between the vibration electrode 25c and the floating electrode 17c near the centre of the vibration-cavity 28.

In the structure illustrated in FIG. 5A, a capacitance, which is generated between the vibration electrode 25c near the centre of the vibration-cavity 28 and the floating electrode 17cin the floating state, is illustrated as $C_1$. And, around the first variable capacitor $C_1$, inter-electrode capacitances that are generated between the vibration electrode 25c of the second potential, which surround the first variable capacitor $C_1$ in hexagonal-ring as illustrated in FIG. 2, and the fixed-potential electrode17o of the ground potential (first potential) GND are expressed as $C_2$. In situation in which the ultrasonic waves $\Phi$ is irradiated, the flexures of the vibration-membrane 23 and the vibration electrode 25c are not uniform and are downward convex in shape Thus, an inter-electrode distance of the first variable capacitor $C_1$ is narrower than inter-electrode distances of the second variable capacitors $C_2$. As explained in FIG. 3, the second variable capacitors $C_2$arecapacitances having roles equivalent to the capacitance defined between the upper and lower electrodes in the earlier capacitive acoustic-elements. On the contrary, the first variable capacitor $C_1$ is a capacitor that induces the charges required to drive the IG semiconductor element specific to the AI semiconductor element of the first embodiment. In relationships of the inter-electrode distances as illustrated in FIG. 5A, when the ultrasonic waves $\Phi$ are irradiated, a magnitude ratio of the first variable capacitor $C_1$ to the second variable capacitor $C_2$ is not determined only by a ratio of an area of the floating electrode 17c to an area of the fixed-potential electrode17o.

FIG. 5B represents the ideally deformed shape of the vibration electrode 25c and the vibration-membrane 23 when the ultrasonic waves are supplied to the AI semiconductor element of the first embodiment, contrary to the real structure of the AI semiconductor element illustrated in FIG. 5A, In FIG. 5B, since the vibration-membrane 23 is displaced in flat manner, inter-electrode distances between parts of the vibration electrode 25c and the fixed-potential electrode17oat the positions around the floating electrode 17c are equal to an inter-electrode distance between the vibration electrode 25cand the floating electrode 17c near the centre of the vibration-cavity 28. In FIG. 5B, electric-field strengthened-layer 19made of silicon nitride film having high specific dielectric constant is inserted between both sides of the vibration-cavity 28. Moreover, stiffness-toughened lid 31cmade of silicon nitride film with high stiffness, is disposed on a vibration-electrode protection-film 26. However, as indicated by the following simulation result in FIG. 6A, it is known that the ideal shape of flat parallel plate capacitance cannot be obtained, in accordance with the schemes of the electric-field strengthened-layer 19 and the stiffness-toughened lid 31c, which are adopted exemplarily in FIG. 5B.

FIG. 6A illustrates profiles when the shapes of the deformations (flexures) of the vibration-membranes of the capacitive acoustic-elements with various structures are simulated. In the simulations, the centre of abscissa of FIG. 6A corresponds to the centre of the vibration-cavity serving as space in which the vibration-membranes are deformed. That is, FIG. 6A illustrates the results in which the spatial changes in the position coordinates in the vibration-cavity are simulated along $\pm$ directions in the scale unit of two micrometers, defining the centre of abscissa (= the centre of the vibration-cavity) as zero. Ordinate of FIG. 6A represents relative values such that, from the zero (no-displacement) defined at the top of ordinate, the displacement amounts of the vibration-membrane change downward to the position of the maximum displacement, which is labelled as "-1". FIG. 6A illustrates what the profiles of the flexural shapes of the vibration-membrane are, in a space widen by ten micrometers in left-right directions from the centre of the vibration-cavity indicated on abscissa, under the above conditions.

Dashed line in FIG. 6A illustrates the result when the deformed shape of the earlier vibration-membrane is simulated, and as already explained by using FIG. 28, the simulated result represents that the upper electrode and the lower electrode come close to each other only within a range of about 0.7 micrometre toward left-right directions from the centre of the vibration-cavity. FIG. 28 illustrates that the vibration-membrane is deflected within the vibration-cavity 28, toward a flat lower electrode 17B and the top surface of a cavity-bottom insulating-film 18 on the lower electrode 17B, in the profile in which an upper electrode 25B, which is sandwiched in between the vibration-electrode protection-film 26 and the vibration-membrane 23, is bent downward convex, at the same curvature as the simulation profile of the flexural shape indicated by dashed line in FIG. 6A. The second gate-insulating film 16 is illustrated under the lower electrode 17B in FIG. 28.

A dash-dotted line in FIG. 6A illustrates a simulation profile of a deformed shape of the vibration-membrane by a first improvement-scheme. In the first improved-scheme, silicon nitride film ( $\varepsilon_r$= 7.0 to 7.8) whose specific dielectric constant is higher than a specific dielectric constant $\varepsilon_r$= 3.9 to 4.5 of silicon oxide film is inserted into the cavity-surrounding insulating-film 20, which surrounds the vibration-cavity 28 as illustrated in FIG. 3,the cavity-surrounding insulating-film 20 establishes the hermetically confined space of the vibration-cavity 28 and the silicon oxide film is inserted as the electric-field strengthened-layer 19 in FIG. 6C. That is, dash-dotted line in FIG. 6A illustrates the simulated profile of the deformed shape of the vibration-membrane, in a case of a structure- -peripheral electric-field strengthening structure- - in which the electric-field strengths near sidewalls of the vibration-cavity 28 are increased by the first improvement-scheme. A structure illustrated in FIG. 6C,as will be described later, adopts a structure in which the electric-field strengthened-layer 19 by the first improvement-scheme is inserted between a cavity-sidewall top surface insulating-film 21o and the cavity-bottom insulating-film 18, considering the simulation results illustrated in FIG. 6A. Bidirectional arrow marks labelled on both sides of upper abscissa in FIG. 6A illustrate that, on both sides of the vibration-cavity, peripheral electric-field strengthening structures are adopted in a range of 3.12five micrometers from the side-edges of the vibration-cavity. Contrasting with the profile of the earlier structure represented by dashed line, there is a propensity that the curvature of the downward-convex curve becomes slightly small by adopting the first improvement-scheme.

A thin solid line in FIG. 6A represents a simulated profile of a deformed shape of the vibration-membrane by a second improvement-scheme. In a simulation in the second improvement-scheme, a stiffness-toughening structure is examined in which silicon nitride film is selected as a high-stiffness insulating-film that has high stiffness. And therefore, by the silicon nitride film, a stiffness-toughened lid 31c is stacked on a vibration-electrode protection-film 26 as illustrated in FIG. 6C, and further, the silicon nitride film is used even in the vibration-membrane 23 just under the vibration electrode 25c. Although the Young's modulus (longitudinal elastic modulus) of silicon oxide film is Y = 66 to 68 GPa, the Young's modulus of the silicon nitride film is Y = 210 to 310 GPa. Bidirectional arrow mark labelled on the centre of upper abscissa in FIG. 6A represents that the stiffness-toughened lid 31c with a width of ten micrometers is arranged in a range of ±five micrometers from the centre of the vibration-cavity. In addition, in the profile of the second improvement-scheme represented by the thin solid line, the peripheral electric-field strengthening structure is not adopted. Contrasting with the profile of the earlier structure represented by dashed line and the peripheral electric-field strengthening structure represented by dash-dotted line, the curvature of the downward-convex curve becomes further smaller value by adopting the second improvement-scheme. Especially, at both edges of the arranged position of the stiffness-toughened lid 31c, the position of which is indicated by bidirectional arrow mark labelled at the centre of upper abscissa in FIG. 6A, there are inflection points where the curvature of the downward-convex curve change.

"Moment I of inertia of area" is calculated by dividing the cross-section into many micro-sectional areas dA, and multiplying each of the above areas by the square $y^2$ of a distance from a specific rotational axis, and then adding them all together ($I = \sum (y^2 dA)$), and accordingly, the moment I has the dimension of the fourth power of length. When a bar is bent, the cross-section of the bar is slightly rotated, and then, "the specific rotational axis" means the central axis when the bar is bent. The moment of inertia of area represents the resistance against the deformation in geometric meaning, about bending. As well known, the moment of inertia of area in material having a cross-sectional shape of a rectangular parallelepiped of width b and thickness h is expressed by a form that is inversely proportional to the third power of the thickness h, as indicated by Eq. (16):

$$I = bh^3/12 \qquad \text{--- ---} (16).$$

On the other hand, the Young's modulus Y represents the resistance against the deformation in mechanical meaning.

The flexure (bending) of a beam is determined by using and calculating a neutral-plane radius $\rho$. When the neutral-plane radius is large, the flexure becomes small. Thus, the above means that the larger YI, the harder the beam is to be deflected. Therefore, the YI is also called a flexural rigidity of the beam:

$$K = YI = Ybh^3/12 \qquad \text{--- ---} (17).$$

The deformation of the flat plate is classified into flexure (bending), extension (compression) and shear (torsion). Even the flexural rigidity K in the case of the flat plate is expressed by the following expression (18), by using a Poisson's ratioó:

$$K = YI = Yh^3/12(1\text{-}ó^2) \qquad \text{--- ---} (18).$$

Thus, the rigidity K against a bending load is known to be proportion to the product of the Young's modulus Y and the three powers ($h^3$) of a plate thickness.

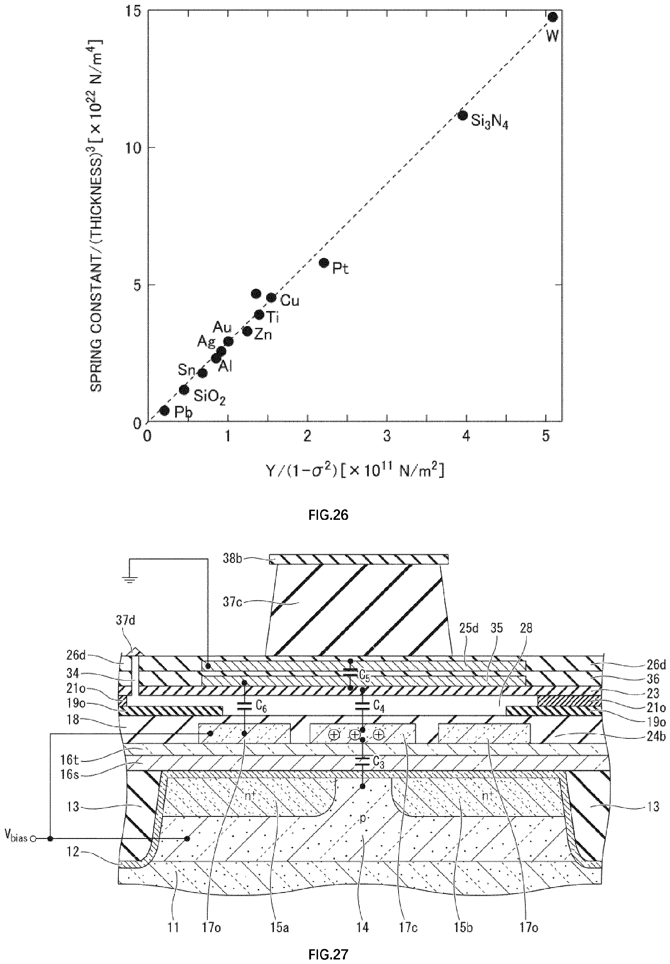

So, in FIG. 26,ordinaterepresentsa value ($k/h^3$) in which a spring constant k calculated from "(the maximum field $\times$ charges) / (the maximum displacement)" when the DC bias-voltage $V_{bias}$ is applied between the upper electrode (vibration electrode) and the lower electrode (fixed-potential electrode) is divided by $h^3$, and abscissa represents a value of $Y/(1\text{-}ó^2)$, and the calculated values are plotted for various materials. When $\alpha$ is defined as a proportional constant, for the various materials, the following Eq. (19) is established:

$$k/h^3 = \alpha (Y/(1\text{-}ó^2)) \qquad \text{--- ---} (19).$$

Then, FIG. 26 represents that the left-hand side and right-hand side of Eq. (19) are in proportional relationship. That is, FIG. 26 represents that the main component of the spring constant k on the vibration electrode side is the flexural rigidity represented by Eq. (18). The Poisson's ratio o can theoretically only take a value of 0.5 or less. As some examples are illustrated in Table 1, the Poisson's ratios óof many materials belong to a range between about 0.3 and 0.4. Thus, when the Poisson's ratioóis squared ($ó^2$), the squared value becomes about 0.09 to 0.16.

Therefore, the contribution of the factor of ($1-ó^2$) with respect to abscissa of FIG. 26 is relatively small contrasting with the Young's modulus Y. Thus, the materials having high Young's modulus Y are located on the upper right potion in FIG. 26. From FIG. 26, the silicon nitride film ($Si_3N_4$ film) having high Young's modulus Y will have a high flexural rigidity of flat plate.

[Table 1]

(Table 1)

| Material | Poisson's Ratio |
|---|---|
| W | 0.28 |
| Al | 0.345 |
| Cu | 0.343 |
| Pb | 0.44 |
| Au | 0.44 |
| Sn | 0.36 |
| $SiO_2$ | 0.2 |
| $Si_3N_4$ | 0.27~0.25 |

The thick solid line in FIG. 6A represents a simulated profile of the deformed shape of the vibration-membrane by a third improvement-scheme. FIG. 6A represents the simulated profile of the deformed shape of the vibration-membrane when the peripheral electric-field strengthening structure by the first improvement-scheme illustrated by dash-dotted line and the stiffness-toughening structure by the second improvement-scheme represented by the thin solid line are used together at the same time. By adopting the third improvement-scheme, the curvature of the downward-convex curve becomes further smaller, contrasting with the case of only the stiffness-toughening structure by the second improvement-scheme represented by the thin solid line. Even in the case of the third improvement-scheme, the positions at both edges of the stiffness-toughened lid31c, which is allocated at the position of ±five micrometers from the centre of the vibration-cavity, have the inflection points at which the curvatures of the downward-convex curves change.

The results of the simulations illustrated in FIG. 6A teaches that the ideal flat parallel-plate structure as illustrated in FIG. 5B cannot be realized practically. FIG. 6B is a cross-sectional view explaining a design configuration by which a capacitor structure is built by curved surfaces, with the lower electrode side as the curved surface, in accordance with the simulated results illustrated in FIG. 6A. The vertically measured dimensions of the curves indicating the flexures of the vibration-membrane 23 and the vibration electrode 25c in FIG. 6B is reduced from vertically measured dimension of the curvature illustrated in FIG. 6A. FIG. 6B illustrates that the vibration-membrane 23 and the vibration electrode 25c are deflected in the profile of downward convex within the vibration-cavity 28, at the same curvature as the simulated profile of the flexural shape in the third improvement-scheme indicated by the thick solid line in FIG. 6A, although the size along vertical direction is reduced.

In FIG. 6B, the floating electrode 17cand the fixed-potential electrode 17o are arranged on the curved surface depending on the flexure of the vibration electrode 25c, different from the structures illustrated in FIG. 5A and FIG. 5B. Even the top planar shape of the cavity-bottom insulating-film 10 in which the floating electrode 17c and the fixed-potential electrode17o are embedded is deflected in the downward convex profile at the same curvature as the simulated profile of the flexural shape in the third improvement-scheme illustrated by the thick solid line in FIG. 6A. In FIG. 6B, a capacitance generated between the vibration electrode 25c near the centre of the vibration-cavity 28 and the floating electrode 17c in floating state is $C_1$, and inter-electrode capacitances between the vibration electrode 25c of the second potential, which surrounds the central first variable capacitor $C_1$, on both sides of the first variable capacitor $C_1$, and the fixed-potential electrode17o of the ground potential (first potential) GND are $C_2$. Even in the structure illustrated in FIG. 6B, the second variable capacitors $C_2$are the capacitances having the role equivalent to the capacitance scheduled between the upper and lower electrodes in the earlier capacitive acoustic-elements. On the contrary, the first variable capacitor $C_1$ is the capacitor that induces the charges required to drive the IG semiconductor element unique to the AI semiconductor element of the first embodiment. In the structure illustrated in FIG. 6B, the inter-electrode distance between the first variable capacitor $C_1$ and the second variable capacitors $C_2$ are equal. Thus, the magnitude of the first variable capacitor $C_1$ and the second variable capacitors $C_2$ are determined by an area ratio between the floating electrode 17c and the fixed-potential electrode17o.

However, giving a consideration to the current technical-process level of microfabrication, achieving the configuration structure of the cavity-bottom insulating-film 10 and the fixed-potential electrode17o as illustrated in FIG. 6B is practically difficult. Thus, there is a limitation in improving the detection sensitivity by detecting voltages as the changes of the charges, which are accumulated in the capacitance between the upper and lower electrodes, in the architecture adapted for the earlier capacitive acoustic-elements. Therefore, in the AI semiconductor element and AEIC pertaining to the first embodiment, a new method is proposed which detects the changes in the capacitance as the changes in current, by integrating the IG semiconductor element as an inner structure for measuring the current, departing from the architecture of detecting the changes of charges in the earlier capacitive acoustic-elements as voltages.

FIG. 6C is a cross-sectional view explaining an AI semiconductor element pertaining to an improved structure of the first embodiment in which the third improvement-scheme explained in FIG. 6A is adopted. As illustrated in FIG. 6C, the AI semiconductor element pertaining to the improved structure of the first embodiment encompasses a channel-generating region 14, which is a semiconductor region of the first conductivity type (p-type) and set to the first potential (= ground potential GND), and a first main-electrode region 15b and second main-electrode region 15a of the second conductivity type (n-type). The first main-electrode region 15b and second main-electrode region 15a are arranged at and in the surface of the channel-generating region 14, facing to and spaced from each other. Moreover, the lower electrode (17c, 17o), having a topology of divided electrodes, is facing to the vibration electrode 25c via the vibration-cavity 28 as illustrated in FIG. 6C. The electric field between the fixed-potential electrode 17obuilding up the lower electrode (17c, 17o) and the vibration electrode 25c keeps the predominant voltage at transmission and receiving modes of the AI semiconductor element.

The floating electrode 17c in floating state building up the lower electrode (17c, 17o) acts to electro-statically control a potential-barrier height generated in the surface of the channel-generating region 14 between the first main-electrode region 15b and the second main-electrode region 15a via potential through the charges induced by the floating electrode 17c.When the potential barrier height changes, a current $I_{ds}$ flowing between the first main-electrode region 15b and the second main-electrode region 15 will change. Thus, in the AI semiconductor element of the improved structure of the first embodiment, the displacements of the portion electrode 25c caused by the ultrasonic waves applied to the vibration electrode 25c can be detected as the changes in the current $I_{ds}$ flowing between the first main-electrode region 15b and the second main-electrode region 15a.

Similarly to the structure illustrated in FIG. 3, the AI semiconductor element pertaining to the improved structure of the first embodiment encompasses an IG semiconductor element. The IG semiconductor element includes the gate-insulating films (12 and 16), which are laminated on the first main-electrode region 15b, the second main-electrode region 15a and the channel-generating region 14, which is sandwiched in between the first main-electrode region 15b and the second main-electrode region 15a. The AI semiconductor element further encompasses a floating electrode 17cmade of the conductive layers in floating state, on the gate-insulating films (12 and 16). The floating electrode 17c is disposed above the channel-generating region 14, at a site above a location sandwiched in between the first main-electrode region 15b and the second main-electrode region 15a. Regarding the gate-insulating film (12 and 16), the double-layer structure made of the first gate-insulating films 12 and the second gate-insulating film 16 is exemplified. However, a situation that the gate-insulating film is not limited to the structure illustrated in FIG. 6C is like the case explained in FIG. 3.

And, illustrated in FIG. 6C, a fixed-potential electrode17o is further provided on the gate-insulating films (12 and 16). The fixed-potential electrode 17o is arranged adjacently to the floating electrode 17c and separately from the floating electrode 17c. The fixed-potential electrode 17o is made of the conductive layer such as DOPOS film, and is set to the first potential GND. A lower electrode (17c, 17o) of the divided structure composed of the floating electrode 17c and the fixed-potential electrode17o is provided. That is, the AI semiconductor element further encompasses a vibration-membrane 23, which becomes of the insulating film facing via the vibration-cavity 28, to the lower electrode (17c, 17o) composed of the floating electrode 17c and the fixed-potential electrode17o, and a vibration electrode 25c, which is in contact with the top surface of the vibration-membrane 23 and facing via the vibration-cavity28 to the floating electrode 17c and the fixed-potential electrode17o. The vibration electrode 25c is set to the second potential $V_{bias}$. A cavity-bottom insulating-film 18 is stacked on the second gate-insulating film 16to cover the floating electrode 17c and the fixed-potential electrode17o.

As illustrated in FIG. 6C, a cavity-surrounding insulating-film (19o, 21o),in which the vibration-cavity 28 is enclosed as hermetically confined space, is cylindrically arranged between the lower electrode (17c, 17o) and the vibration electrode 25c. The cavity-surrounding insulating-film (19o, 21o) embraces an electric electric-field strengthened-layer 19o and a cavity-sidewall top surface insulating-film 21o. The electric electric-field strengthened-layer 19o is formed in hexagonal-ring to surround the space of the vibration-cavity 28 on the cavity-bottom insulating-film 18 as an opening. The cavity-sidewall top surface insulating-film 21o is laminated on the electric electric-field strengthened-layer 19oin hexagonal-ring. The electric electric-field strengthened-layer 19o is the insulating film whose specific dielectric constant is higher than the specific dielectric constant of the silicon oxide film $\varepsilon_r$= 3.9 to 4.5 which is explained in the first improvement-scheme. In addition to the silicon nitride film, alumina ($Al_2O_3$) whose specific dielectric constant $\varepsilon_r$ = about 9 can be employed. Moreover, haunium silicate ($HfSi_xO_y$) whose specific dielectric constant $\varepsilon_r$= about 11, lanthanum oxide ($La_2O_3$) whose specific dielectric constant $\varepsilon_r$= about 21, zirconia ($ZrO_2$) whose specific dielectric constant $\varepsilon_r$= about 25 and Hafnia ($HfO_2$) whose specific dielectric constant $\varepsilon_r$= about 26 can be employed.

As illustrated in FIG. 6C, a first surface-wiring layer 25b and a second surface-wiring layer 25 are disposed on the top surface of the vibration-membrane 23, in addition to the vibration electrode 25c. The first contact plug 24b, which penetrates through the vibration-membrane 23, the cavity-surrounding insulating-film (19o, 21o), the cavity-bottom insulating-film 18, the second gate-insulating film 16 and the first gate-insulating film 12, is provided in a location between the first surface-wiring layer 25b and the first main-electrode region 15b. Similarly, the second contact plug 24a, which penetrates through the vibration-membrane 23, the cavity-surrounding insulating-film (19o, 21o), the cavity-bottom insulating-film 18, the second gate-insulating film 16 and the first gate-insulating film 12, is provided in a location between the second surface-wiring layer 25a and the second main-electrode region 15a. That is, the top end of the first contact plug 24b is metallurgically connected to the first surface-wiring layer 25b, and the bottom end of the first contact plug 24b is electrically connected to the first main-electrode region 15b. That is, the first surface-wiring layer 25b and the first main-electrode region 15b are electrically connected through the first contact plug 24b.

Similarly, the top end of the second contact plug 24a is metallurgically connected to the second surface-wiring layer 25a, and the bottom end of the second contact plug 24a is electrically connected to the second main-electrode region 15b. Thus, the second surface-wiring layer 25a and the second main-electrode region 15a are electrically connected through the second contact plug 24a. The first surface-wiring layer 25b corresponds to the vertical output-signal line $R_i$ on the i-th column illustrated in FIG. 2. On the other hand, the second surface-wiring layer 25a corresponds to the power-supply interconnection $V_{DD}$ on the j-th row illustrated in FIG. 2. On the vibration-membrane 23, the vibration-electrode protection-film26 such as the silicon oxide film is coated so as to cover the vibration electrode 25c, the first surface-wiring layer 25b and the second surface-wiring layer 25.

Moreover, on the vibration-electrode protection-film 26, the stiffness-toughened lid 31c explained as the second and third improved-schemes in the simulation in FIG. 6A is locally disposed in a shape of truncated hexagonal pyramid to back up the vibration-membrane 23. As a high-stiffness insulating-film that is strong in rigidity and used in the stiffness-toughened lid 31c, in addition to the silicon nitride film used in the simulation in FIG. 6A, aluminium nitride (AlN) whose Young's modulus Y = about 320GPa, alumina whose Young's modulus Y = about 360 GPa, silicon carbide (SiC) whose Young's modulus Y = about 440 GPa, cermet (TiC-TiN) whose Young's modulus Y = about 440 GPa, sapphire whose Young's modulus Y = about 470 GPa and diamond whose Young's modulus Y = about 340 to 1050 GPa can be used. Moreover, those high-stiffness insulating-films may be used not only as the stiffness-toughened lid 31c to back up the vibration-membrane 23, but also as the material of the vibration-membrane 23 to enhance the rigidity of the vibration-membrane 23 itself.

There is also a method of not only enhancing the rigidity of the vibration-membrane 23 but also enhancing the rigidity of the vibration electrode 25c itself. As the improvement-scheme to enhance the rigidity of the vibration electrode 25c itself, it is allowed to use tungsten (W), molybdenum (Mo) and titanium nitride (TiN) of the metals having high stiffness as the material of the vibration electrode 25c. The Young's modulus of the aluminium (Al) used in the usual Al semiconductor element in many cases is Y = about 80 GPa. However, the Young's modulus of W is Y = about 403GPa, the Young's modulus of Mo is Y = about 327GPa, and the Young's modulus of TiN is Y = about 350 GPa. FIG. 26 illustrates that W has the high flexural rigidity as a flat plate.

As illustrated in FIG. 6C, a conical vacuum plug 31d is disposed on the vibration-electrode protection-film 26. At a time of a step of forming the vibration-cavity 28, it is necessary to introduce a removing solution (etching solution) for selectively solving a sacrificial layer by wet etching. The vacuum plug 31d closes a liquid-introducing hole 27 that is opened to introduce the removing solution. The liquid-introducing hole 27 penetrates through the vibration-electrode protection-film 26 and the vibration-membrane 23 and arrives at the vibration-cavity 28. By closing the liquid-introducing hole 27, the inside of the vibration-cavity 28 is kept in an atmosphere of inert gas at reduced pressure.

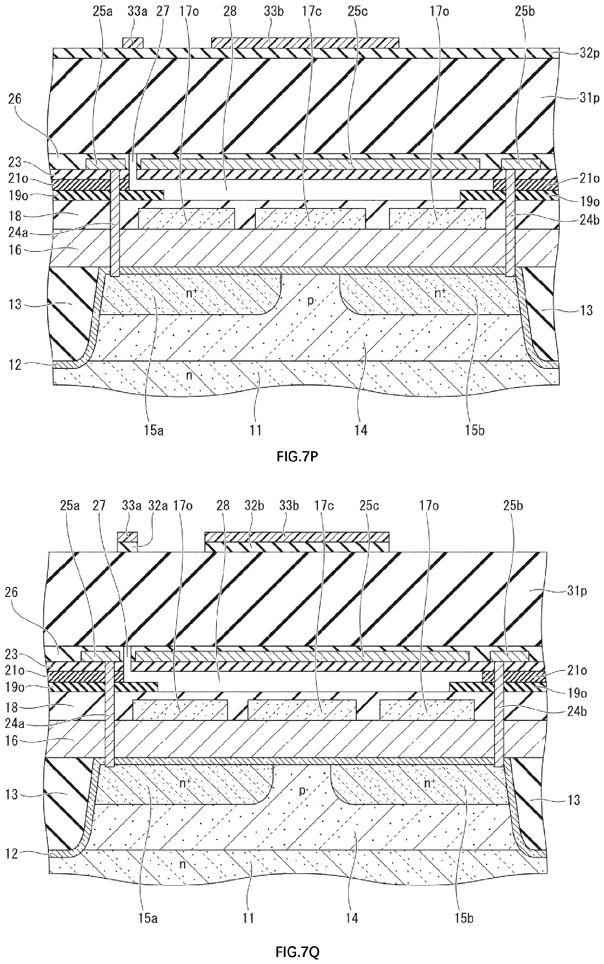

A vacuum-plug upward-pattern 32a, which is used as an etching mask when a stiffness-toughened lid 31pis delineated at a process step illustrated in FIG. 7Q and made of silicon oxide film, remains as cornice state on the stiffness-toughened lid 31c and hangs over. Although the silicon oxide film used as the etching mask exists even on the vacuum plug 31d, the silicon oxide film is lost because the pattern of the vacuum plug 31d is small contrasting with thickness, when wet etching is executed on the silicon nitride film as illustrated in FIG. 7P to FIG. 7R. In addition, FIG. 6C represents as if the liquid-introducing hole 27 and the vacuum plug 31dexist on the same cross-sectional plane as the first contact plug 24b and the second contact plug 24a. However, the liquid-introducing hole 27 and the vacuum plug 31d do not practically exist on the same cross-sectional plane as the first contact plug 24b and the second contact plug 24a.

FIG. 6C illustrates a capacitance of a capacitor as $C_1$, the capacitor $C_1$ is defined between the vibration electrode 25c near the centre of the vibration-cavity 28 and the floating electrode 17c in the floating state. Also, capacitances of capacitors are expressed as $C_2$, which sandwich the first variable capacitor $C_1$ in between. The capacitors $C_2$ are defined between parts of the vibration electrodes 25c, the parts are disposed at both sides of the first variable capacitor $C_1$, and the fixed-potential electrode 17o at the ground potential (first potential) GND. The two second variable capacitors $C_2$ indicated on both sides of the vibration-cavity 28 in FIG. 6C are the capacitors having the role equivalent to the capacitor defined between the upper and lower electrodes in the earlier capacitive acoustic-elements. As can be understood from the planar pattern of hexagonal ring illustrated in FIG. 2, the two second variable capacitors $C_2$ appeared superficially on both sides are practically the continuous same capacitance. On the contrary, the first variable capacitor $C_1$ at the centre of the vibration-cavity 28 is an intrinsic capacitance of the equivalent circuit, the intrinsic capacitance is specific to the AI semiconductor element of the first embodiment.

That is, the first variable capacitor $C_1$ at the centre of the vibration-cavity 28 is the capacitor that induces the charges to the floating electrode 17c, the charges are required to electro-statically drive the IG semiconductor element embedded in the AI semiconductor element. The first variable capacitor $C_1$ at the centre in FIG. 6C is the equivalent-circuit capacitance, which is implemented by the central hexagon accommodated in a window of the planar pattern of hexagonal ring illustrated in FIG. 2. As illustrated in FIG. 4C, the gate-source capacitor $C_{gs}$ is distributed between the floating electrode 17c and the first main-electrode region 15b, the gate-drain capacitor $C_{gd}$ is distributed between the floating electrode 17c and the second main-electrode region 15a, and the insulating-film capacitor Cox is distributed between the floating electrode 17c and the channel-generating region 14. However, in FIG. 6C, the above capacitors are collectively expressed as the fixed capacitor $C_3$. Even the fixed capacitor $C_3$ connected in series to the first variable capacitor $C_1$ is the equivalent-circuit capacitance, which is specific to the AI semiconductor element of the first embodiment. The fixed capacitor $C_3$ connected in series is the capacitance for electro-statically controlling the potential of a channel region generated at and in the surface of the channel-generating region 14, between the first main-electrode region 15b and the second main-electrode region 15a.

The structure illustrated in FIG. 6C includes the IG semiconductor element, such as MOS transistors, etc., has an amplifying function. Then, as the IG semiconductor element is driven by the voltage of the floating electrode 17c, which is defined by the capacitive voltage division, and the relative displacements of the vibration electrode 25c to the floating electrode 17c can be detected as changes-of current $\Delta I_{ds}$ in the IG semiconductor element as represented by Eq. (9). Especially, because values per element becomes smaller and smaller by element miniaturization as the charges are the extensive variables, the difficulty hangs around in incorporating the earlier capacitive acoustic-elements into a receiving circuit, for realizing the high-definition imaging. On the other hand, because the AI semiconductor element of the improved structure of the first embodiment can detect the changes in the voltages that are the intensive variables generated via the floating electrode 17c, even if the element sizes are reduced, the values are not made small. Therefore, according to the AI semiconductor elements of the improved structure of the first embodiment, the voltages detected by each of the AI semiconductor elements are used as the changes of the gate voltages in the IG semiconductor elements, which are built in each of the AI semiconductor elements. Hence, by arraying the AI semiconductor elements, each of which having high detection sensitivity, as minute unit cells, the high-definition imaging can be achieved.

= MANUFACTURING METHOD OF AI SEMICONDUCTOR ELEMENT=

A manufacturing method of the AI semiconductor element pertaining to the improved structure of the first embodiment exemplified in FIG. 6C is explained by using FIG. 7A to FIG. 7R. In addition, the following manufacturing method of the AI semiconductor element is merely one example, and if the structure prescribed by the claims can be realized, including variations, it is naturally possible for realizing the structure prescribed by the claims, by using the various manufacturing methods other than the following manufacturing method. Also, a nomenclature to which an ordinal number is combined such as "a first photoresist film" used in the following explanations, the nomenclature is based on rhetorical request to merely distinguish from other photoresist film, and does not mean the first photoresist film used in practical manufacturing steps.

(a) At first, a common base-body 11 is prepared, which is made of an n-type silicon substrate having a main-surface of (100) plane with about 0.1 ohm-centimetre to three ohm-centimetre. The common base-body 11 is thermally oxidized, and a buffer film made of silicon oxide film is formed to a thickness of about 50 nm. On the buffer film, silicon nitride film ($Si_3N_4$ film) is deposited to about 50 to 200 nm by deposition method of chemical vapor deposition (CVD), and an isolation-region defining insulating-film of double-layer structure of $SiO_2/Si_3N_4$ is deposited. And, a first photoresist film is coated on the isolation-region defining insulating-film, and a photolithography is used to delineate the first photoresist film. Concretely, in such a way that positions of patterns of element-isolation regions of each of unit cells $X_{i(j-1)}$, $X_{ij}$, $X_{i(j+1)}$, --- are two-dimensionally defined on the main-surface of the common base-body 11, the first photoresist film is exposed and developed to delineate the pattern of the element-isolation region. The first photoresist film is used as a first etching mask, and the isolation-region defining insulating-film is etched, and the surface of the common base-body 11 is further etched to about 0.8 to 1.1 micrometers and an element-isolation trench is cut.

After the removal of the first photoresist film and the isolation-region defining insulating-film, the entire surface of the common base-body 11 including the inside of the element-isolation trench is thermally oxidized to create a first gate-insulating film 12 made of silicon oxide film to about 15 to 25 nm. Next, an element-isolation insulating-film 13 made of silicon oxide film, etc., is deposited to a thickness of about 0.9 micrometre to 1.2 micrometers by deposition method of CVD, and the element-isolation insulating-film 13 is buried in the element-isolation trench. On the element-isolation insulating-film 13, the silicon nitride film is deposited to a thickness of about 40 to 60 nm by deposition method of CVD, and a stopper insulating film made of Silicon nitride film is deposited. And, a second photoresist film is coated on the stopper insulating film, and by using a photolithography, the second photoresist film is delineated by mask alignment.

That is, in such a way that patterns of active regions, which are surrounded by the element-isolation regions in each of the unit cells $X_{i(j-1)}$, $X_{ij}$, $X_{i(j+1)}$, ---, are position aligned with two-dimensional shape defined on the main-surface of the common base-body 11, the second photoresist film is exposed and developed to delineate the patterns of the active regions. With the second photoresist film as a second etching mask, the stopper insulating film is selectively etched to protect the top surface of the active region with the stopper insulating film. Next, chemical mechanical polishing (CMP) method is used to carry out planarization with the stopper insulating film as stopper. After the planarization, when the stopper insulating film is selectively etched with hot phosphoric acid ($H_2PO_4$), the first gate-insulating film 12 is laminated on the active region as illustrated in FIG. 7A, and a cross-sectional shape is obtained in which the periphery of the active region is surrounded by the element-isolation insulating-film 13 through the first gate-insulating film 12 of a sidewall.

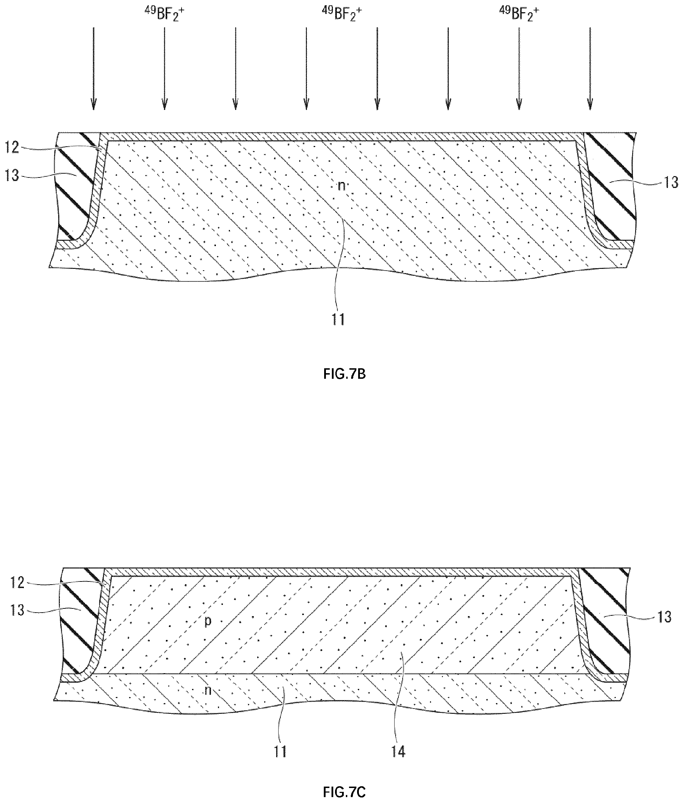

(b) Next, a third photoresist film is coated on the first gate-insulating film 12 and the element-isolation insulating-film 13, and the photolithography is used to delineate a pattern. That is, in such a way that the pattern of the active region is exposed in the third photoresist film, the third photoresist film is exposed and developed to delineate a first ion implanting mask in which the top surface of the active region is opened. The third photoresist film is used as the first ion implanting mask, and as illustrated in FIG. 7B, ions exhibiting a p-type conductivity such as $^{49}BF_2^+$ are ion-implanted at a predetermined acceleration energy. After the implantation of the ions exhibiting the p-type conductivity, when the third photoresist film is removed to perform the annealing, as illustrated in FIG. 7C, the p-type channel-generating region 14 is created in the top surface of the common base-body 11. In addition, the annealing just after the ion implantation may be omitted, the channel-generating region 14 illustrated in FIG. 7C is not still created at this stage when the annealing is omitted. "The p-type channel-generating region 14" has the function and structure equivalent to a p-well in the usual semiconductor integrated circuit.

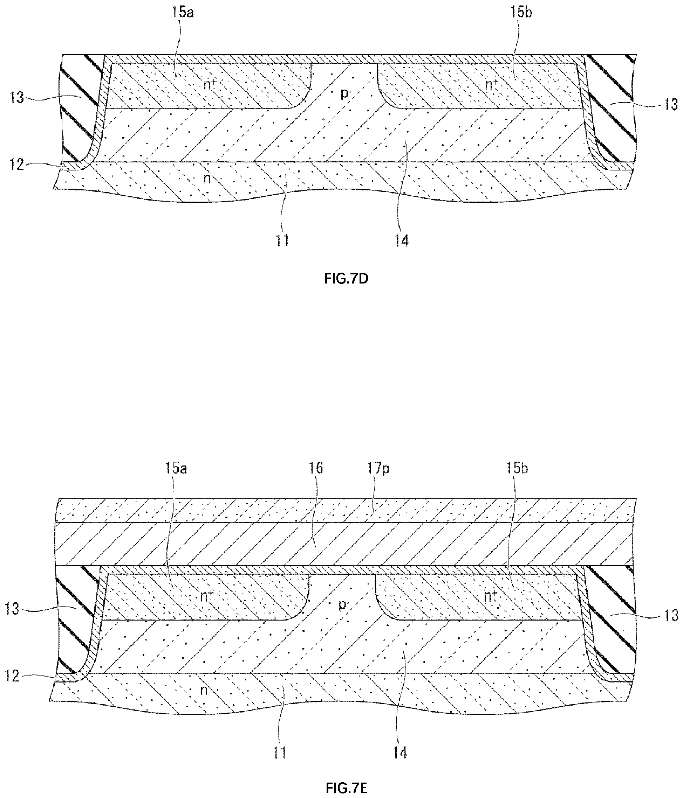

(c) Even when the annealing after the ion implantation of $^{49}BF_2^+$ is omitted, after the removal of the third photoresist film, a fourth photoresist film is coated on the first gate-insulating film 12 and the element-isolation insulating-film 13. And, by using the photolithography, the fourth photoresist film is exposed and developed to delineate a second ion implanting mask for source/drain region formation. By using the fourth photoresist film as the second ion implanting mask, ions exhibiting an n-type conductivity such as $^{75}A_s^+$ are ion-implanted at a predetermined acceleration energy. After the removal of the fourth photoresist film, when the annealing is conducted, as illustrated in FIG. 7D, a cross-sectional structure is realized in which the first main-electrode region 15b and the second main-electrode region 15a are created facing to each other in a top surface of the p-type channel-generating region 14. An example of a planar pattern of the first main-electrode region 15b and the second main-electrode region 15a is the rectangular pattern, for example, as illustrated in FIG. 2. After that, a step of ion-implantation for threshold control of MOSFET can be added, as necessary.

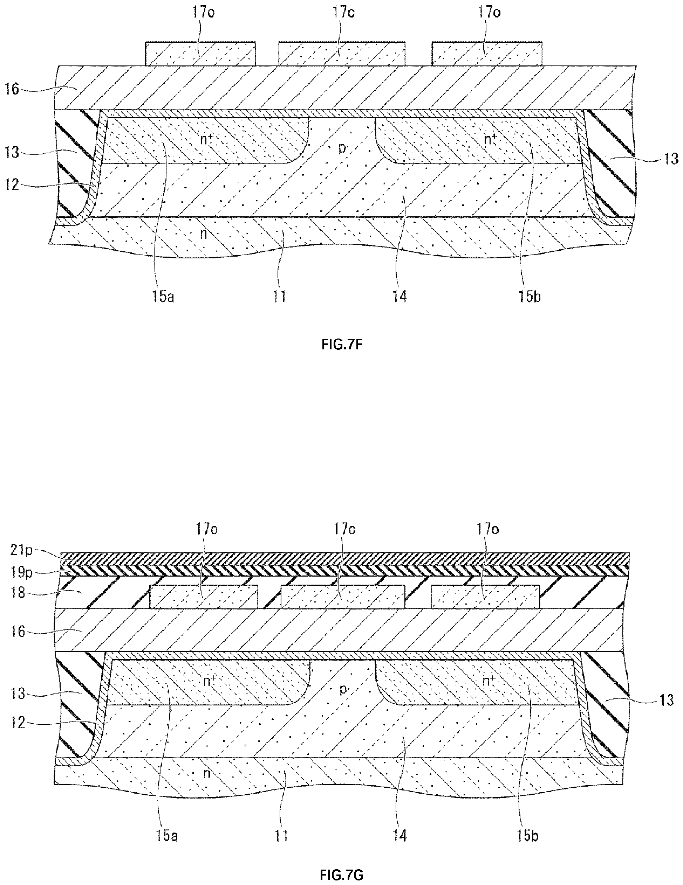

(d) After that, on the first gate-insulating film 12, a second gate-insulating film 16 made of silicon oxide film is deposited to a thickness of about 180 to 250 nm by using the deposition method of CVD. Moreover, as illustrated in FIG. 7E, on the second gate-insulating film 16, a first conductor film 17p made of conductive layer such as DOPOS film is deposited to a thickness of about 80 to 200 nm by deposition method of CVD. A fifth photoresist film is coated on the first conductor film 17p, and by using the photolithography, the fifth photoresist film is exposed and developed to delineate a third etching mask for defining a fixed-potential electrode. By using the third etching mask and a dry etching technology such as a reactive ion etching (RIE), as illustrated in FIG. 7F, the first conductor film 17p is selectively etched to delineate the pattern of the fixed-potential electrode 17o and the floating electrode 17c. As already explained by using FIG. 2, for example, a planar shape pattern of the hexagonal floating electrode 17cis defined in the hexagonal fixed-potential electrode 17o, in which a hexagonal window is opened at the centre.

(e) After that, by using the deposition method of CVD, a cavity-bottom insulating-film 18 is deposited on the second gate-insulating film 16 to perfectly cover the top surfaces of the fixed-potential electrode 17o and the floating electrode 17c. As the cavity-bottom insulating-film 18, for example, tetraethoxysilane (TEOS) film is deposited to a thickness of about 40 to 60 nm. As necessary, the polishing method such as the CMP method is used to planarize the top surface of the cavity-bottom insulating-film 18. Moreover, on the cavity-bottom insulating-film 18, a high dielectric constant layer 19p that is higher in specific dielectric constant $\varepsilon_r$ than the silicon nitride film or the silicon oxide film such as alumina film ($Al_2O_3$ film) is deposited to a thickness of about 40 to 60 nm. Moreover, as illustrated in FIG. 7G, by using the deposition method of CVD, on the high dielectric constant layer 19p, a cavity insulating film 21p made of silicon oxide film is deposited to a thickness of about 40 to 60 nm.

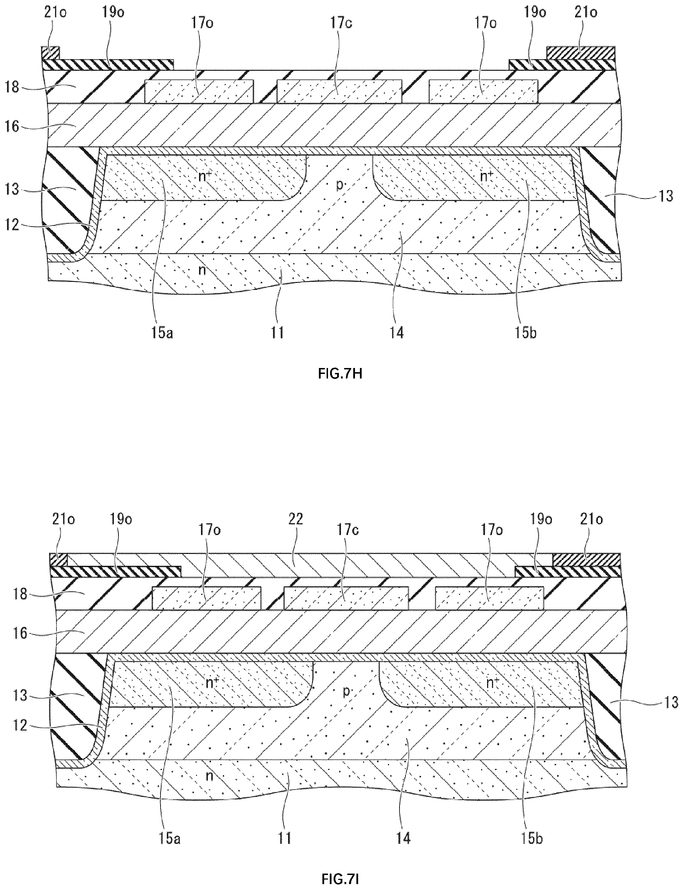

(f) And, a sixth photoresist film is coated on the cavity insulating film 21p, and by using the photolithography, the sixth photoresist film is exposed and developed to delineate a fourth etching mask for defining a vibration-cavity top surface. The fourth etching mask is used to selectively etch the cavity insulating film 21p, and the high dielectric constant layer 19p is exposed at a bottom of a window that is opened in the cavity insulating film 21p. After the removal of the sixth photoresist film, a seventh photoresist film is coated on the cavity insulating film 21p and the high dielectric constant layer 19p exposed at the bottom of the window of the cavity insulating film 21p. And by using the photolithography, the seventh photoresist film is exposed and developed to delineate a fifth etching mask for defining a vibration-cavity bottom. By using the fifth etching mask, a part of the high dielectric constant layer 19p exposed at the bottom of the window of the cavity insulating film 21p is selectively etched to establish a cross-sectional structure illustrated in FIG. 7H. Namely, an electric-field strengthened-layer 19o is defined as ring to surround the vibration-cavity bottom, and on the electric-field strengthened-layer 19o, the cavity-sidewall top surface insulating-film 21o is defined as ring to surround a vibration-cavity top surface, thereby the cross-sectional shape having steps is established as illustrated in FIG. 7H.

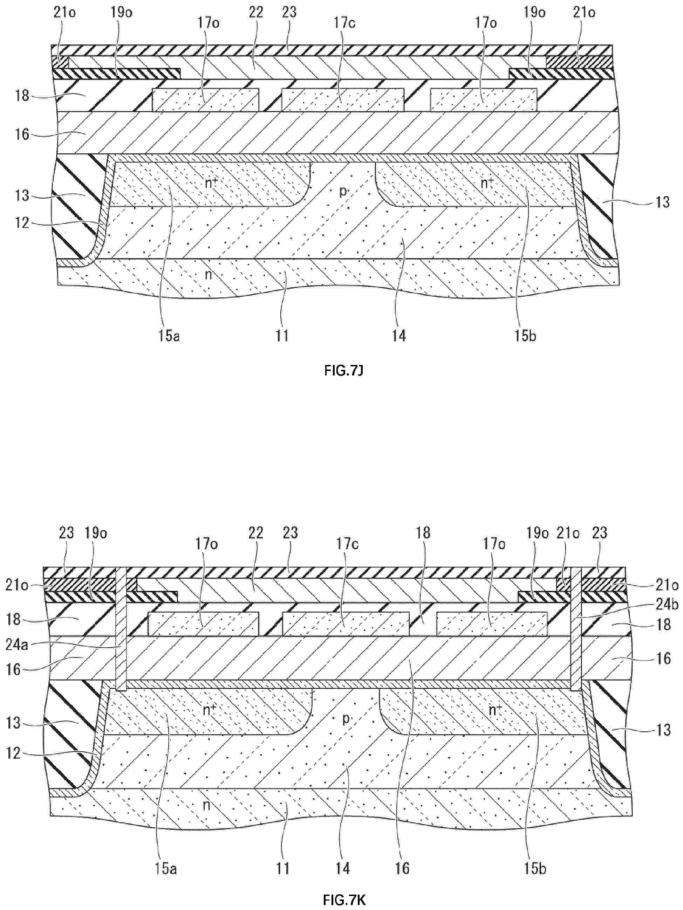

(g) And, by using a sputtering method, a vacuum evaporating method, or the deposition method of CVD, a sacrificial material film layer made of tungsten (W) is deposited on the entire surface. And, the CMP method is used to planarize, and as illustrated in FIG. 7I, a sacrificial layer 22 is buried in stepped shape into the vibration-cavity bottom and the vibration-cavity top surface. Moreover, by using the deposition method of CVD, as illustrated in FIG. 7J, on the sacrificial layer 22 and the cavity-sidewall top surface insulating-film 21o surrounding the sacrificial layer 22, the vibration-membrane 23 made of high-stiffness dielectric-film is deposited on the entire surface. As the high-stiffness dielectric-film, silicon nitride film, etc. can be adopted

(h) After that, an eighth photoresist film is coated on the vibration-membrane 23, and by using the photolithography, to arrive at the positions of the patterns of the first main-electrode region 15b and the second main-electrode region 15a, mask alignment is conducted to delineate a pattern for via-hole creation. By using the eighth photoresist film as a sixth etching mask, a first via-hole, which penetrates through the vibration-membrane 23, the cavity-sidewall top surface insulating-film 21o, the electric electric-field strengthened-layer 19o, the cavity-bottom insulating-film 18, the second gate-insulating film 16 and the first gate-insulating film 12 and arrives at the surface of the first main-electrode region 15b, is drilled by using the method of the RIE. At the same time, a second via-hole, which penetrates through the vibration-membrane 23, the cavity-sidewall top surface insulating-film 21o, the electric electric-field strengthened-layer 19o, the cavity-bottom insulating-film 18, the second gate-insulating film 16 and the first gate-insulating film 12 and arrives at the surface of the second main-electrode region 15a is drilled at the same step as the first via-hole.

After that, a refractory metal such as W, molybdenum (Mo), or titanium (Ti) is deposited as a second conductor film on the vibration-membrane 23 by using deposition method with excellent step-coverage behaviour such as reduced pressure CVD method. And, the second conductor film is etched back, and the second conductor film is buried into the via-holes, and as illustrated in FIG. 7K, the first contact plug 24b arriving at the first main-electrode region 15b is created, and the second contact plug 24a arriving at the second main-electrode region 15a is created. The second conductor film may be made of multi-level metallic layers of different kinds, and the first contact plug 24b and the second contact plug 24a may be made of multi-level metallic poles in a shape of concentric cylinders. When the first contact plug 24b and the second contact plug 24a are buried, the CMP method may be used as necessary.

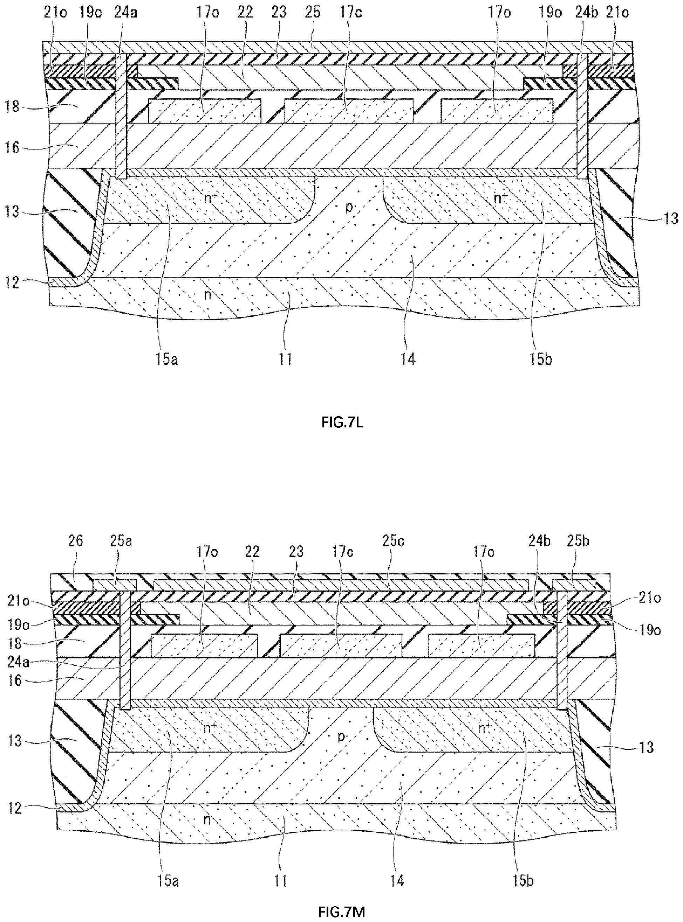

(i) And, on the vibration-membrane 23, a third conductive film made of aluminium (Al) or aluminium alloy is deposited on the entire surface by using the sputtering method, the vacuum evaporation method or the deposition method of CVD. Moreover, a ninth photoresist film is coated on the third conductive film, and the photolithography is used to delineate a pattern for defining a vibration electrode. By using the ninth photoresist film as a seventh etching mask, the third conductive film is selectively etched to delineate the patterns of the vibration electrode 25c, the first surface-wiring layer 25b and the second surface-wiring layer 25, as illustrated in FIG. 7L. The planar pattern of the vibration electrode 25c may be selected as the shape and dimension of the hexagon corresponding to the shape and size of the fixed-potential electrode17o, for example, illustrated in FIG. 2. On the other hand, the first surface-wiring layer 25b and the second surface-wiring layer 25a are delineated to required patterns, such that the first surface-wiring layer 25b is metallurgically connected to the first contact plug 24b, and the second surface-wiring layer 25a is metallurgically connected to the second contact plug 24a.

In addition, to reinforce the rigidity of the vibration electrode 25c, high-stiffness metal W can be adopted as the third conductive film. When W is used for the vibration electrode 25c, W film is delineated by damascene process. To perform the damascene process, at first, by using the deposition method of CVD, a damascene-insulating film made of silicon oxide film is deposited on the vibration-membrane 23. And, by using the photolithography and selectively etching the damascene-insulating film, the trench of the pattern of the vibration electrode 25c is cut in the damascene-insulating film, and W film is buried in the trench, and pattern of the vibration electrode 25c is delineated. The CMP or the like may be used at the damascene process of practically burying W film.

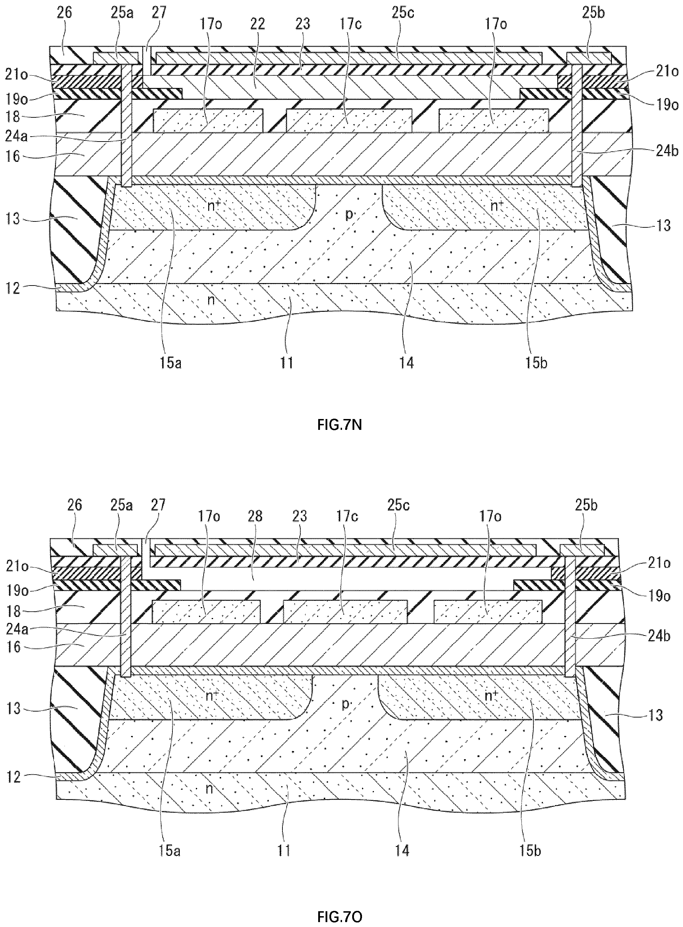

(j) Next, on the vibration-membrane 23, by using the deposition method of CVD, the vibration-electrode protection-film 26 made of silicon oxide film is deposited to cover the patterns of the vibration electrode 25c, the first surface-wiring layer 25b and the second surface-wiring layer 25, as illustrated in FIG. 7M. Moreover, a tenth photoresist film is coated on the vibration-electrode protection-film 26, and the photolithography is used to delineate a pattern for introducing a removing solution. By using the tenth photoresist film as an eighth etching mask, a liquid-introducing hole 27, which penetrates the vibration-electrode protection-film 26 and the vibration-membrane 23 and arrives at the sacrificial layer 22 is drilled as illustrated in FIG. 7N. FIG. 7N illustrates a cross-sectional shape after the tenth photoresist film as the eighth etching mask is removed.

(k) And, for example, heated hydrogen peroxide ($H_2O_2$) is introduced to the sacrificial layer 22 from the liquid-introducing hole 27. When the heated hydrogen peroxide is introduced from the liquid-introducing hole 27, the sacrificial layer 22 is selectively solved by wet etching. When the sacrificial layer 22 is solved, as illustrated in FIG. 7O, the vibration-cavity 28 is created at a position above the fixed-potential electrode17o. After that, by using the deposition method of CVD, the stiffness-toughened lid 31p made of high-stiffness material such as silicon nitride film is deposited to a thickness of about 400 to 600 nm on the vibration-electrode protection-film 26, and the liquid-introducing hole 27 is closed. When the process-step for closing the liquid-introducing hole 27 is conducted under atmosphere at a reduced pressure of about 1kPa, using inert gas such as helium (He) gas as main component of the atmosphere, the inside of the vibration-cavity 28 will become a reduced pressure that can be regarded as almost vacuum, confining the inert gas as the main component in the vibration-cavity 28.

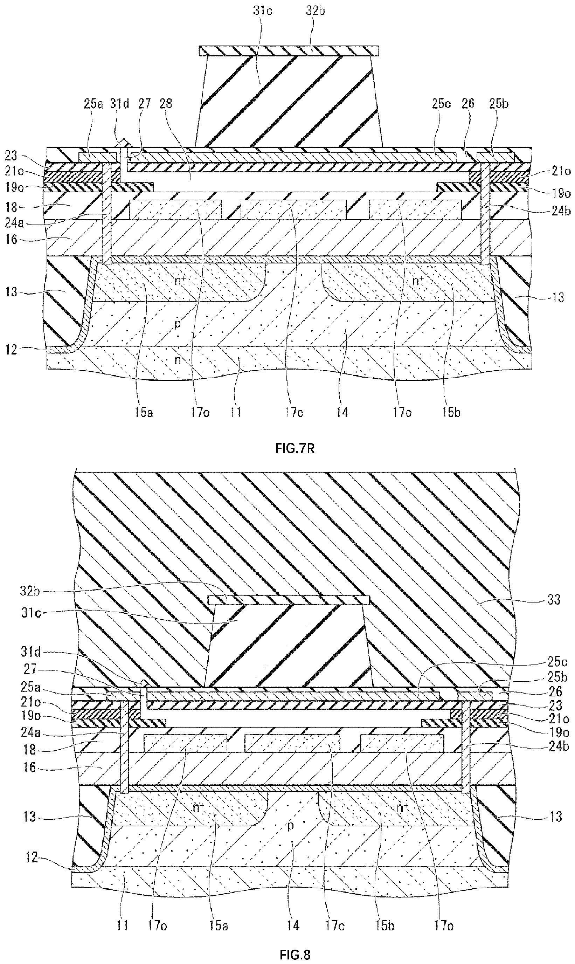

(l) Moreover, on the high-stiffness insulating-film 31p, by using the deposition method of CVD, a patterning-insulating film 32p made of silicon oxide film is deposited to a thickness of about 45 to 55 nm on the entire surface. And, an eleventh photoresist film is coated on the patterning-insulating film 32p, and patterns 33a and 33b for defining a stiffness-toughened lid are delineated by using the photolithography. By using the patterns 33a and 33b of the eleventh photoresist film as a ninth etching mask, the patterning-insulating film 32p is delineated as illustrated in FIG. 7P, and a vacuum-plug upward-pattern 32a and a cavity upward-pattern 32b are delineated. After the patterns 33a and 33b of the eleventh photoresist film are removed, when the stiffness-toughened lid 31p is etched by using the vacuum-plug upward-pattern 32a and the cavity upward-pattern 32b as a tenth etching mask, as illustrated in FIG. 7R, the conical vacuum plug 31d remains on the liquid-introducing hole 27. On the other hand, the stiffness-toughened lid31c having the shape of truncated hexagonal pyramid is created above the vibration electrode 25c. When the high-stiffness insulating-film 31p is made of silicon nitride film, as the high-stiffness insulating-film 31p is wet-etched with hot phosphoric acid, the planar shape of the AI semiconductor element pertaining to the improved structure of the first embodiment is obtained as illustrated in FIG. 7R.

## <<FIRST VARIATION OF FIRST EMBODIMENT>>

For realizing the ideally deformed shape of the vibration electrode 25c creating the flat parallel-plate capacitor with the lower electrode (17c, 17o)as exemplified in FIG. 5B, in view of the examination illustrated in FIG. 6A, a partial region of the vibration electrode 25c shall be locally backed up, in relation with the rigidities of the vibration electrode 25c and the vibration-membrane 23. Thus, as illustrated in FIG. 6C, the uniform protection-film in which the thickness of polyimide film is even, which is preferred in the earlier general capacitive acoustic-element, shall not to be used for an objective directed to realize a non-uniform planar distribution of stiffness, in which rigidities are localized. However, according to the AI semiconductor element of the improved structure of the first embodiment, the IG semiconductor element is incorporated in the AI semiconductor element, which enables the displacement of the vibration electrode 25c to be efficiently detected as the changes-of current $\Delta I_{ds}$ because the IG semiconductor element is driven by the voltages, which are defined by the capacitive voltage division in relation with the floating electrode17c as represented by Eq. (9). Hence, the scheme of avoiding the configuration, in which the uniform protection-film made of polyimide film with even thickness is stacked on the vibration electrode 25c, is not essential.

In short, as illustrated in FIG. 3, the second vibration-electrode protection-film 34 made of polyimide film may be stacked on the first vibration-electrode protection-film 26. In an AI semiconductor element pertaining to the variation (first variation) of the first embodiment illustrated in FIG. 8, similarly to the configuration illustrated in FIG. 3, a second vibration-electrode protection-film 33 made of polyimide film is uniformly stacked on the vibration-electrode protection-film (first vibration-electrode protection-film) 26 illustrated in FIG. 6C. Because the constitutional structures other than the second vibration-electrode protection -film 33 explained in the AI semiconductor element pertaining to the first variation of the first embodiment are on a similar level with the configurations illustrated in FIG. 6C, the duplicated explanations are omitted.

## <<SECOND VARIATION OF FIRST EMBODIMENT>>

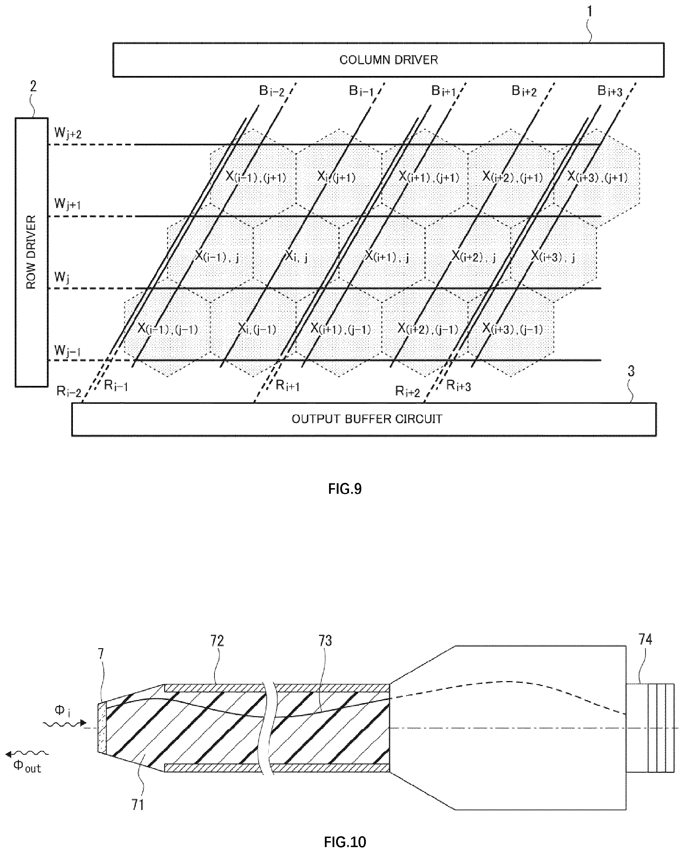

Although FIG. 1 exemplifies the case in which the two-dimensionally-arrayed AI semiconductor element are driven by units of columns, each of the AI semiconductor elements arrayed in a shape of matrix may be driven individually. In AEIC pertaining to the second variation of the first embodiment, as illustrated in FIG. 9, a column driver 1 is arranged on the upper side of the upper latus of an element array portion, and a row driver 2 is arranged on the left-hand side of the right-hand latus of the element array portion. Moreover, an output buffer circuit 3 is arranged on the lower side of the lower latus of the element array portion. Two-dimensional phased array operation is possible by arraying a large number of unit cells $X_{i,j}$ (i = 1 to m, j = 1 to n: each of m and n is positive integers of two or more) in a shape of two-dimensional matrix in the element array portion and constructing the irradiation and imaging area of a predetermined shape.

By word lines $W_{j+2}$, $W_{j+1}$, $W_j$, $W_{j-1}$ --- delivered from the row driver 2, respective cell rows in the element array portion are scanned by units of cell rows. That is, unit cells $X_{(i-1),(j+1)}$, $X_{i,(j+1)}$, $X_{(i+1),(j+1)}$, $X_{(i+2),(j+1)}$, $X_{(i+3),(j+1)}$, --- on the (j+1)-th row are scanned by a word line $W_{j+1}$ on a (j+1)-th row. Also, unit cells $X_{(i-1),j}$, $X_{i,j}$, $X_{(i+1),j}$, $X_{(i+2),j}$, $X_{(i+3),j}$, --- on a j-th row are scanned by a word line $W_j$ on the j-th row, and unit cells $X_{(i-1),(j-1)}$, $X_{i,(j-1)}$, $X_{(i+1),(j-1)}$, $X_{(i+2),(j-1)}$, $X_{(i+3),(j-1)}$, --- on a (j-1)-th row are scanned by a word line $W_{j-1}$ on the (j-1)-th row. Moreover, unit cells $X_{i1}$, $X_{i2}$, $X_{i3}$, --- --- -- -, $X_{i,(j-1)}$, $X_{ij}$, $X_{i(j+1)}$, --- --- ----- on a i-th row are scanned by a word line $W_j$ on the i-th row.

On the other hand, by bit lines $B_{(i-1)}$, $B_i$, $B_{(i+1)}$, $B_{(i+2)}$, $B_{(i+3)}$, --- delivered from the column driver 1, respective cell columns in the element array portion are sequentially scanned by units of cell columns. For example, by the bit line $B_{(i-1)}$ on the (i-1)-th column, the unit cells $X_{(i-1),(j+1)}$, $X_{(i-1),j}$, $X_{(i-1),(j-1)}$, --- on the (i-1)-th column. Also, by the bit line $B_i$ on the i-th row, the unit cells $X_{i,(j+1)}$, $X_{i,j}$, $X_{i,(j-1)}$, --- on the i-th column are scanned, and by the bit line $B_{i+1}$ on the (i+1)-th column, unit cells $X_{(i+1),(j+1)}$, $X_{(i+1),j}$, $X_{(i+1),(j-1)}$ --- on the (i+1)-th column scanned. Moreover, by the bit line $B_{i+2}$ on the (i+2)-th column, the unit cells $X_{(i+2),(j+1)}$, $X_{(i+2),j}$, $X_{(i+2),(j-1)}$ --- on the (i+2)-the column are scanned, by the bit line $B_{i+3}$ on the (i+3)-th column, the unit cells $X_{(i-1),(j+1)}$, $X_{(i-1),j}$, $X_{(i-1),(j-1)}$ --- on the(i+1)-th column are scanned.

Then, in the AEIC pertaining to the second variation of the first embodiment, by the word lines $W_{j+2}$, $W_{j+1}$, $W_j$, $W_{j-1}$ ---, the respective cell rows in the element array portion are scanned by units of cell rows, and by the bit lines $B_{(i-1)}$, $B_i$, B(i+1), B(i+2), B(i+3), ---, the respective cell columns are sequentially scanned by units of cell columns, and therefore, a two-dimensional array-irradiation of ultrasonic signals and a two-dimensional reading-operation of the cell signals are conducted. That is, the AEIC pertaining to the second variation of the first embodiment is organized such that the element array portion is scanned sequentially along a vertical direction at unit cells $X_{(i-1),(j+1)}$, $X_{i,(j+1)}$, $X_{(i+1),(j+1)}$, $X_{(i+2),(j+1)}$, $X_{(i+3),(j+1)}$, ---, $X_{(i-1),j}$, $X_{i,j}$, $X_{(i+1),j}$, $X_{(i+2),j}$, $X_{(i+3),j}$, ---, $X_{(i-1),(j-1)}$, $X_{i,(j-1)}$, $X_{(i+1),(j-1)}$, $X_{(i+2),(j-1)}$, $X_{(i+3),(j-1)}$, ---, $X_{i1}$, $X_{i2}$, $X_{i3}$, ---, $X_{i(j-1)}$, $X_{ij}$, $X_{i(j+1)}$, --- units on the respective cell rows and accordingly, the cell signals of unit cells $X_{(i-1),(j+1)}$, $X_{i,(j+1)}$, $X_{(i+1),(j+1)}$, $X_{(i+2),(j+1)}$, $X_{(i+3),(j+1)}$, ---, $X_{(i-1),j}$, $X_{ij}$, $X_{(i+1),j}$, $X_{(i+2),j}$, $X_{(i+3),j}$, ---, $X_{(i-1),(j-1)}$, $X_{i,(j-1)}$, $X_{(i+1),(j-1)}$, $X_{(i+2),(j-1)}$, $X_{(i+3),(j-1)}$, ---, $X_{i1}$, $X_{i2}$, $X_{i3}$, --- --- ---, $X_{i(j-1)}$, $X_{ij}$, $X_{i(j+1)}$, --- on the respective cell rows are read out by the vertical output-signal lines $R_{(i-2)}$, $R_{(i-1)}$, $R_i$, R(i+i), $R_{(i+2)}$, $R_{(i+3)}$, --- provided for each of the unit cells $X_{(i-1),(j+1)}$, $X_{(i-1),j}$, $X_{(i-1),(j-1)}$, ---,$X_{j,(j+1)}$, $X_{i,j}$, $X_{i(j-1)}$, ---, $X_{(i+1),(j+1)}$, $X_{(i+1),j}$, $X_{(i+1),(j-1)}$, ---, $X_{(i+2),(j+1)}$, $X_{(i+2),j}$, $X_{(i+2),(j-1)}$, ---, $X_{(i-1),(j+1)}$, $X_{(i-1),j}$, $X_{(i-1),(j-1)}$, --- on the respective cell columns.

The AEIC pertaining to the second variation of the first embodiment can be used for other applications such as underwater hydrophone, from the aspect of acoustic impedance, in addition to the application for medical field. That is, the AEIC explained in the second variation of the first embodiment has an acoustic impedance close to the intrinsic impedance of water, and represents excellent pulse-response characteristics. And, the AEIC of the second variation represents high sensitivity, wide band, and low output impedance characteristics, in addition to a feature that the AI semiconductor element does not require similar backing material as the earlier capacitive acoustic-elements. Then, the above characteristics and feature are suitable for the applications to the hydrophones.

An AEIC 7 pertaining to the second variation of the first embodiment is fixed to a tip of a resin horn illustrated as a shape of a truncated cone on the left of FIG. 10. A resin cylindrical portion 71 is continuous to the left side tip of the resin horn, the resin cylindrical portion 71 is thick in diameter and is opposing to the left side tip of the resin horn. As illustrated in FIG. 10, the main body (71, 72) of the hydrophone encompasses the resin horn, the resin cylindrical portion 71 continuous to the resin horn and a cylindrical enclosure 72 in which the resin cylindrical portion 71 is accommodated. The resin horn, the resin cylindrical portion 71 and the cylindrical enclosure 72 a remerged into a single body. According to the hydrophone illustrated in FIG. 10, the AEIC 7 detects ultrasonic waves $\Phi_1$ reflected from a not-inspection-target, and the AEIC 7irradiatesultrasonic waves $\Phi_2$ toward the not-inspection-target, and then, the ultrasonic waves $\Phi_1$ that are reflection waves from the not-inspection-target are generated.

The cylindrical enclosure 72 of the hydrophone illustrated in FIG. 10 has an outer terminal 74 on an end side opposite to tip side along an axial direction. The outer terminal 74 is a connector which is connected through a transmission line, such as a coaxial cable, etc., to an observation device whose illustration is omitted. Since the AEIC 7 and the outer terminal 74 are connected through a lead line 73, an output signals of the AEIC 7are directed to the outer terminal 74. The transmission line connected to the outer terminal 74 on the other end side of the main body (71, 72) and the observation device connected to the transmission line are included, which can observe two-dimensional signals of the ultrasonic waves $\Phi_1$ reflected from the not-inspection-target. In a case of underwater application, a pressure balance structure is required to avoid an influence of water pressure. Thus, it is very importance to optimize the thickness of the vibration membrane and the size of the vibration-cavity, depending on employed frequency. In underwater use, since the ultrasonic waves whose frequency is low compared with the medical application is used, the size of the vibration-cavity and the inter-electrode distance are made larger, thereby requiring a larger bias voltage.

## <<THIRD VARIATION OF FIRST EMBODIMENT>>

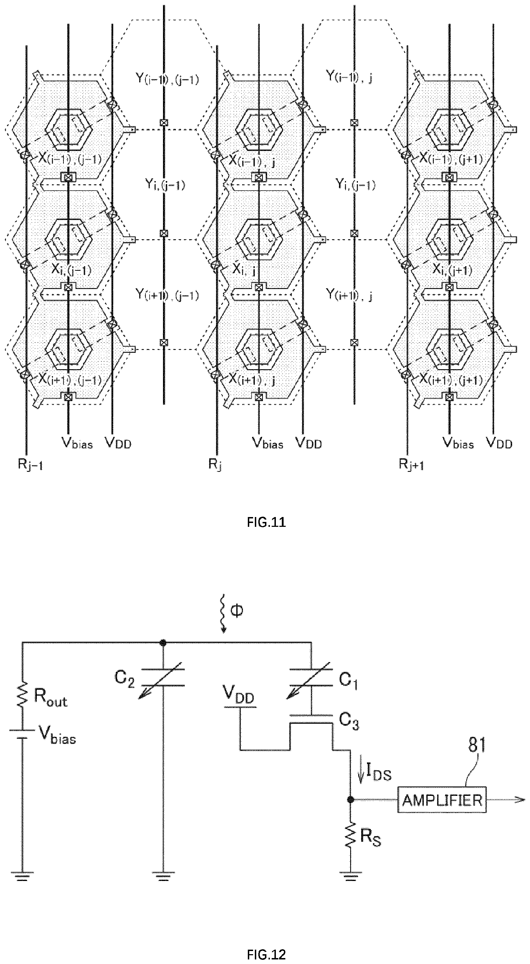

An AEIC pertaining to the third variation of the first embodiment of the present invention inheres in a feature such that the AEIC includes AI semiconductor elements dedicated to reception and capacitive acoustic-elements dedicated to transmission, the capacitive acoustic-elements are arranged independently of the AI semiconductor elements dedicated to reception. As illustrated in FIG. 11, transmitting cells $Y_{(i-1),(j-1)}$, $Y_{i,(j-1)}$, $Y_{(i+1),(j-1)}$, --- on a transmitting (j-1) column indicated as hollow white cells are arrayed between receiving cells $X_{(i-1),(j+1)}$, $X_{i,(j+1)}$, $X_{(i+1),(j+1)}$, --- on a receiving (j+1)-th column and receiving cells $X_{(i-1),j}$, $X_{i,j}$, $X_{(i+1),j}$, --- on a receiving j-th column, the receiving cells $X_{(i-1),(j+1)}$, $X_{i,(j+1)}$, $X_{(i+1),(j+1)}$ --- ;$X_{(i-1),j}$, $X_{i,j}$, $X_{(i+1),j}$, --- are illustrated by gray-color representation. Also, transmitting cells $Y_{(i-1),j}$, $Y_{i,j}$, $Y_{(i+1),j}$, --- on a transmitting j-column indicated as hollow white cells are arrayed between receiving cells $X_{(i-1),j}$, $X_{i,j}$, $X_{(i+1),j}$,on a receiving j-th column and receiving cells $X_{i,(j-1)}$, $X_{(i+1),(j-1)}$, --- on a receiving (j-1)-th column, the receiving cells $X_{(i-1),j}$, $X_{i,j}$, $X_{(i+1),j}$, ---; $X_{i,(j-1)}$, $X_{(i+1),(j-1)}$, --- are illustrated by gray-color representation.

FIG. 11 exemplifies a case in which the planar patterns of the receiving cells $X_{i,j}$ and the transmitting cells $Y_{i,j}$ are hexagonal. However, the planar patterns of the receiving cells $X_{i,j}$ and the transmitting cells $Y_{i,j}$ are not limited to be hexagonal, and various planar patterns such as rectangles, octagons, etc. can be adopted. Each of the transmitting cells $Y_{(i-1),(j-1)}$, $Y_{i,(j-1)}$, $Y_{(i+1),(j-1)}$, ---; $Y_{(i-1),j}$, $Y_{i,j}$, $Y_{(i+1),j}$, --- in the AEIC pertaining to the third variation of the first embodiment encompasses the same structure as the earlier capacitive acoustic-elements. Each of the receiving cells $X_{(i-1),(j+1)}$, $X_{i,(j+1)}$, $X_{(i+1),(j+1)}$, ---; $X_{(i-1),j}$, $X_{i,j}$, $X_{(i+1),j}$, ---; $X_{i,(j-1)}$, $X_{(i+1),(j-1)}$, --- arranged independently of the transmitting cells is separately defined as a channel-generating region, isolated from each other by the element-isolation insulating-film, similarly to the configuration already explained with reference FIG. 3, etc., and two-dimensionally arrayed in a top surface of the common substrate. And, similarly to the configuration illustrated in the cross-sectional view in FIG. 3, etc., the IG semiconductor elements, each of which having the first and second main-electrode regions, are arranged on the surface of the channel-generating region, and the vibration-cavities are provided in portions above the IG semiconductor elements.

Although the reference numerals 15a and 15b are omitted in the plan view in FIG. 11, two rectangles indicated by concealed lines inside the hexagons with gray-color representations indicate the first and second main-electrode regions making up the IG semiconductor elements for the respective receiving cells. The two rectangles whose long latera are defined along oblique latera, which slope downward to the left, and two rectangles are facing to each other, are arranged in each of the receiving cells $X_{(i-1),(j+1)}$, $X_{i,(j+1)}$, $X_{(i+1),(j+1)}$, ---; $X_{(i-1),j}$, $X_{i,j}$, $X_{(i+1),j}$, ---; $X_{i,(j-1)}$, $X_{(i+1),(j-1)}$, --- which are hexagonal with gray-color representations, to bite into the inside of the inner hexagon from the outer hexagon ring. According to the receiving cells in the AEIC pertaining to the third variation of the first embodiment, the changes of the capacitance are detected as the variations of current flowing between the first and second main-electrode regions, as represented by Eq. (9).

For each of the IG semiconductor elements in the two-dimensionally-arrayed receiving cells, vertical output-signal lines $R_{j-1}$, $R_i$, $R_{j+1}$, --- are connected to the respective receiving cells, configured to take out current signals from the first or second first main-electrode region. The vertical output-signal lines $R_{j-1}$, $R_i$, $R_{j+1}$, --- are connected to first contact plugs provided on the left vertical latus of each of the hexagonal receiving cells. For example, the vertical output-signal line $R_{j-1}$ on the (j-1)-th column for receiving is connected to the first contact plug provided on the left vertical latus of the hexagon of each of the receiving cells $X_{(i-1),(j-1)}$, $X_{i,(j-1)}$, $X_{(i+1),(j-1)}$, --- on the (j-1)-th column for receiving. Also, the vertical output-signal line $R_j$ on the j-th column for receiving is connected to the first contact plug provided on the left vertical latus of the hexagon of each of the receiving cells $X_{(i-1),j}$, $X_{i,j}$, $X_{(i+1),j}$, --- on the j-th column for receiving. Similarly, the vertical output-signal line $R_{i+1}$ on the (j+1)-th column for receiving is connected to the first contact plug provided on the left vertical latus of the hexagon of each of the receiving cells $X_{(i-1),(j+1)}$, $X_{i,(j+1)}$, $X_{(i+1),(j+1)}$, --- on the (j+1)-th column for receiving.

To drive the IG semiconductor elements provided in each of the channel-generating regions 14 in the respective receiving cells, the power-supply interconnection $V_{DD}$ is connected to the second main-electrode regions in the IG semiconductor elements. The power-supply interconnection $V_{DD}$ is connected to second contact plugs provided on the right vertical latus in each of the receiving cells of the hexagon, as illustrated in FIG. 11. For example, the power-supply interconnection $V_{DD}$ on the (j-1)-th column for receiving is connected to the second contact plugs provided on the right vertical latera of the hexagons in each of the receiving cells $X_{(i-1),(j-1)}$, $X_{i,j}$, $X_{(i+1),(j-1)}$, --- on the (j-1)-th column for receiving.

Also, the power-supply interconnection $V_{DD}$ on the j-th column for receiving is connected to the second contact plugs provided on the right vertical latera of the hexagons in each of the receiving cells $X_{(i-1),j}$, $X_{i,j}$, $X_{(i+1),j}$, --- on the j-th column for receiving. Moreover, the power-supply interconnection $V_{DD}$ on the (j+1)-th column for receiving is connected to the second contact plugs provided on the right vertical latera of the hexagons in each of the receiving cells $X_{(i-1),(j+1)}$, $X_{i,(j+1)}$, $X_{(i+1),(j+1)}$, --- on the (j+1)-th column for receiving.

On the other hand, for the receiving cell to carry out a receiving operation, between the vibration electrode and fixed-potential electrode in each receiving cell, a DC bias-supply line $V_{bias}$ for supplying a DC bias $V_{bias}$ of second potential is connected to a third contact plug provided on a lower horizontal latus of each of the receiving cells of the hexagons. That is, the DC bias-supply line $V_{bias}$ on the (j+1)-th column for receiving is connected to the third contact plug in each of the receiving cells $X_{(i-1),(j+1)}$, $X_{i,(j+1)}$, $X_{(i+1),(j+1)}$, --- on the (j+1)-th column for receiving. Also, the DC bias-supply line $V_{bias}$ on the j-th column for receiving is connected to the third contact plug in each of the receiving cells $X_{(i-1),j}$, $X_{i,j}$, $X_{(i+1),j}$, --- on the j-th column. Moreover, the DC bias-supply line $V_{bias}$ on the (j-1)-th column for receiving is connected to the third contact plug in each of the receiving cells $X_{i,(j-1)}$, $X_{(i+1),(j-1)}$, --- on the (j-1)-th column.

On the other hand, for the transmitting cells arranged independently of the receiving cells to carry out transmitting operations, a high-frequency signal-line is connected to a transmitting-cell contact-plug provided on a lower horizontal latus in each of the cells dedicated to transmission of the hexagons. That is, the high-frequency signal-line on the (j-1)-th column for transmitting is connected to the transmitting-cell contact-plug of each of the transmitting cells $Y_{(i-1),(j-1)}$, $Y_{i,(j-1)}$, $Y_{(i+1),(j-1)}$, --- on the (j-1)-th column for transmitting. Also, a high-frequency signal-line $V_{RFj}$ on the j-th column for transmitting is connected to the transmitting-cell contact-plug of each of the transmitting cells $Y_{(i-1),j}$, $Y_{i,j}$, $Y_{(i+1),j}$, --- on the j-th column for transmitting.

The AEIC pertaining to the third variation of the first embodiment encompasses the unit cells of the AI semiconductor elements dedicated to reception and the unit cells made of the capacitive acoustic-elements dedicated to transmission, which are arranged independently of the unit cells dedicated to reception. Thus, as illustrated in FIG. 11, the second main-electrode region is directly connected to the power-supply interconnection $V_{DD}$. The circuit configuration illustrated in FIG. 11 differs from the circuit configuration in which the second main-electrode region is connected to the power-supply interconnection $V_{DD}$ through a switching switch, as illustrated in FIG. 4A. That is, each cell implementing the AEIC pertaining to the first embodiment as illustrated in FIG. 1, etc., is assumed to be the transmittable/receivable bidirectional acoustic-element as a premise. Thus, although at a time of receiving, the operational voltage $V_{DD}$ is supplied from the power-supply interconnection $V_{DD}$, the switching switch is used so that the power-supply interconnection $V_{DD}$ is connected to the first potential (ground potential) at a time of transmitting. On the contrary, the AEIC pertaining to the third variation of the first embodiment is constructed to separately include the receiving cell $X_{i,j}$ and the transmitting cell $Y_{i,j}$ as illustrated in FIG. 11. Thus, the switching switch illustrated in FIG. 4A is unnecessary.

**(SECOND EMBODIMENT)**

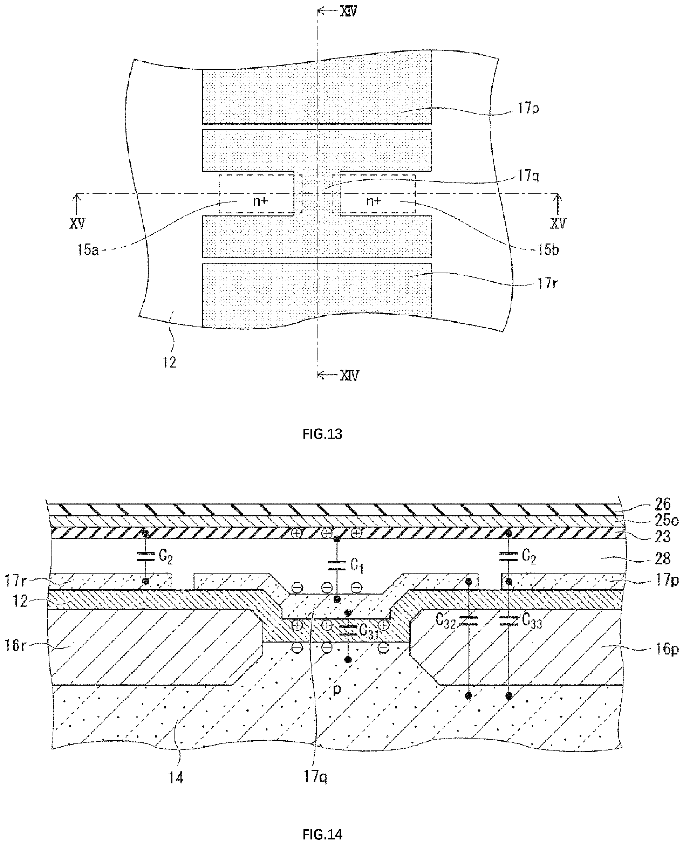

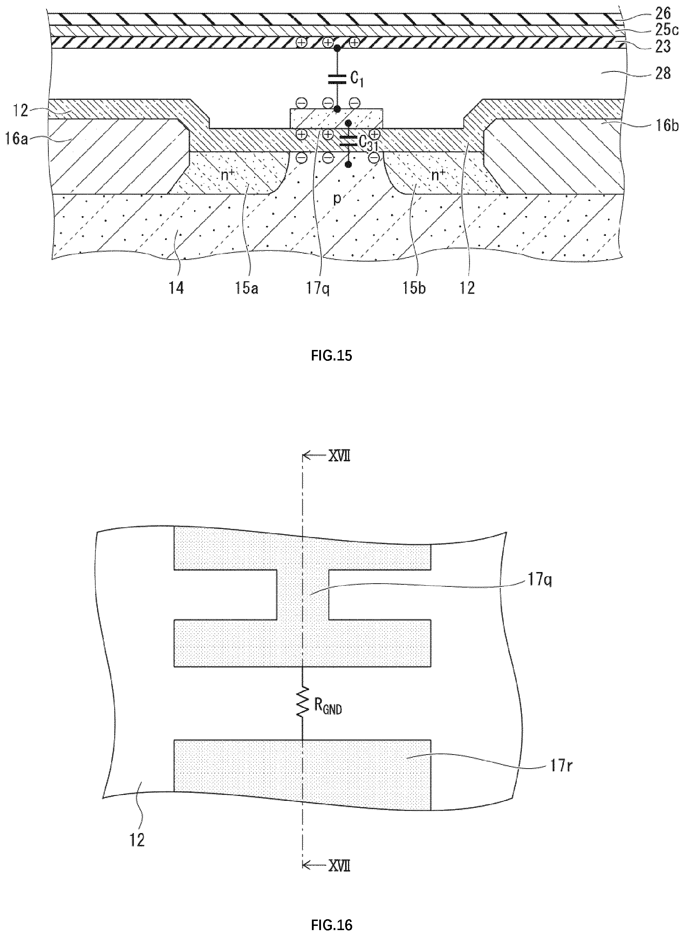

In the AI semiconductor elements pertaining to the first embodiment and the improved structures of the first embodiment, the gate-insulating films (12 and 16) made of the first gate-insulating film 12 and the second gate-insulating film 16 are uniform in thickness just under the floating electrode 17c. However, the structures illustrated in FIG. 3 and FIG. 6C, etc., are merely exemplifications. Thus, the thickness of the gate-insulating film just under the floating electrode 17c may be non-uniform such as the AI semiconductor elements pertaining to a second embodiment of the present invention, as illustrated in FIG. 13 and FIG. 15. Moreover, although FIG. 1 and FIG. 2 exemplify the hexagonal-annular fixed-potential electrode 17o, and the planar pattern in which the hexagonal floating electrode 17c is accommodated in the hexagonal opening made in the centre of the fixed-potential electrode 17o, it is not limited to the exemplified topology. For example, as illustrated in FIG. 13, the topology may be designed such that a floating electrode 17q is delineated in a pattern having 90 degree rotated-H-shaped constriction, and a width of a vertical bar of the rotated-H-shape defines a gate length, and a height of the vertical bar defines a gate width. That is, a pinch-off portion of the floating electrode 17q, corresponding to the vertical bar of the rotated-H-shape, implements "an effective gate electrode".

Focusing to the planar pattern illustrated in FIG. 13, a fixed-potential electrode $17_p$ on an upper side of paper and a fixed-potential electrode 17r on a lower side of the paper are arranged to sandwich the floating electrode 17q between the upper and lower sides. Because the first main-electrode region 15b and the second main-electrode region 15a in the IG semiconductor elements are arranged to sandwich the effective gate electrode, the gate-source capacitor $C_{gs}$ and the gate-drain capacitor $C_{gd}$ indicated in the equivalent circuit illustrated in FIG. 4C can be reduced. As can be understood from a cross-sectional view along a gate width direction illustrated in FIG. 14, only a first gate-insulating film 12 whose film thickness is thin exists in "the effective gate region" defined just under the effective gate electrode of the floating electrode 17q.

And, the thick second gate-insulating films 16 are arranged at a location separated from the effective gate region, and the first gate-insulating film 12 that extends from the effective gate region covers the top surfaces of the second gate-insulating films 16. In addition, contrary to the AI semiconductor elements of the first embodiment, in the AI semiconductor elements pertaining to the second embodiment, the first gate-insulating film 12 serves as an upper-layer gate-insulating film, and the second gate-insulating films 16 serve as low-layer gate-insulating films. And, the second gate-insulating films 16 serve as field insulating films. Similarly, even the cross-sectional view along the gate length direction illustrated in FIG. 15 illustrates that only the first gate-insulating film 12 whose film thickness is thin exists in the effective gate region just under the floating electrode 17q. Even in the cross-sectional view in the direction illustrated in FIG. 15, the second gate-insulating films 16 are arranged in a location separated from the effective gate region, and the first gate-insulating film 12 extending from the effective gate region covers the top surfaces of the second gate-insulating films 16. In addition, although FIG. 14 and FIG. 15 omit the illustration of the cavity-surrounding insulating-film in which the vibration-cavity is enclosed as the hermetically confined space on the floating electrode 17q, similarly to FIG. 3, as a matter of course, the configuration illustrated in FIG. 14 and FIG. 15 can include the cavity-surrounding insulating-film 20.

In the configuration illustrated in FIG. 15, a first variable capacitor $C_1$ is defined between the vibration electrode 25c and the floating electrode 17q of floating state. In defining the first variable capacitor $C_1$, cross-sectional view of the floating electrode 17q is wing shaped such that the centre is concave between protrusions of wings along a gate width direction, and a main capacitor $C_{31}$ is provided between the channel-generating regions 14 in the central effective gate region. The cross-sectional configuration of the concave-between-wings at the centre illustrated in FIG. 14 and FIG. 15 is preferable, because the concave-between-wings profile is prepared along the deformed shape, considering the profile of the deformed shape of the vibration electrode 25c illustrated in FIG. 6A. In the cross-sectional structure illustrated in FIG. 14 and FIG. 15, in auxiliary-floating electrode-portions of protrusions of wings that are positions on both side along the gate width direction surrounding the effective gate region, which corresponds to central concave, an auxiliary capacitor $C_{32}$ is defined even between the floating electrode 17 and the channel-generating region 14, and distributed capacitances are generated in which the main capacitor $C_{31}$ and the auxiliary capacitor $C_{32}$ are connected in parallel.

Also, similarly to the AI semiconductor elements of the first embodiment, a second variable capacitor $C_2$ is defined between the vibration electrode 25c at the second potential and the fixed-potential electrode 17r at the ground potential (first potential) GND. A peripheral capacitor $C_{33}$ can be defined between the fixed-potential electrode 17r and the channel-generating region 14 and between the fixed-potential electrode 17p and the channel-generating region 14. However, the peripheral capacitor $C_{33}$ is zero when the channel-generating region 14 is connected to the ground potential (first potential). In view of the profile of the deformed shape of the vibration electrode 25c illustrated in FIG. 6A, an inter-electrode distance between the periphery of the vibration electrode 25c and the fixed-potential electrodes 17p and 17r is longer than an inter-electrode distance in the concave portion allocated at the centre in the cross-sectional shape of the wings illustrated in FIG. 14. FIG. 14 illustrates that a first variable capacitor $C_1$ is defined between the vibration electrode 25c and the floating electrode 17q, and a main capacitor $C_{31}$ is defined between the main floating-electrode area of the floating electrode 17q and the channel-generating region 14.

Under conditions that a film thickness of the second gate-insulating films 16 is $T_{ox1}$ = 700 nm, and the dimension of the floating electrode 17q serving as the auxiliary-floating electrode-portions of two rectangles sandwiching the effective gate electrode serving as the main floating-electrode area in FIG. 13 is 30micrometers × 15 micrometers, the auxiliary capacitance becomes $C_{32}$ = 44fF. When the film thickness of the second gate-insulating films 16 is defined as $T_{ox1}$ = 800 nm under the same area, the auxiliary capacitance becomes $C_{32}$ = 39fF. Moreover, the dimension of the effective gate electrode (main floating-electrode) at the centre illustrated in FIG. 13 is defined as four micrometers × four micrometers, and an effective channel length defined in the effective gate region $L_{eff}$ = two micrometers, and a film thickness of the first gate-insulating film 12 $T_{ox2}$ = 100 nm, the main capacitance in the active region of a portion of the effective channel length $L_{eff}$ becomes $C_{31}$ = 2.8fF. When the width of the effective gate electrode is extended to the peripheral portions around the effective channel length $L_{eff}$ to four micrometers, the main capacitance becomes $C_{31}$ = 3.4fF.

Here, when voltages across the terminals of the first variable capacitor $C_1$ is defined as $V_1$ and voltages across the terminals of the parallel connection circuit of the main capacitor $C_{31}$ and the auxiliary capacitor $C_{32}$ is defined as $V_3$ the following Eq. (20) is established:

$$C_1 V_1 = (C_{31} + C_{32}) V_3 \qquad (20).$$

In a case that the first variable capacitor $C_1$ is slightly changed by $\Delta C_1$, when the voltage $V_1$ across the first variable capacitor $C_1$ is assumed not to be changed and a voltage $V_3$ across the main capacitor $C_{31}$ is assumed to be slightly changed by $\Delta V_3$, the following Eq. (21) is established:

$$(C_1 + \Delta C_1) V_1 = (C_{31} + C_{32}) (V_3 + \Delta V_3) \qquad \cdots \cdots (21).$$

If the assumption of Eq. (21) can be kept, the infinitesimal changes $\Delta V_3$ in the voltage $V_3$ across the main capacitor $C_{31}$ is calculated from the following Eq. (22):

$$\Delta V_3 = \Delta C_1 V_1 / (C_{31} + C_{32}) \qquad \cdots \cdots (22).$$

The DC bias-voltage $V_{bias}$ is assumed to be applied across the terminals of the series circuit composed of the main capacitor $C_{31}$- -more properly, be applied across the parallel connection circuit of the main capacitor $C_{31}$ and the auxiliary capacitor $C_{32}$- - and the first variable capacitor $C_1$, similarly to Eq. (4), a voltage Viacross the first variable capacitor $C_1$ is expressed by the following Eq. (23):

$$V_1 = (C_{31} + C_{32}) / (C_1 + C_{31} + C_{32}) \qquad \cdots \cdots (23).$$

When the voltage Viacross the first variable capacitor $C_1$ in Eq. (23) is substituted into Eq. (22), the infinitesimal changes $\Delta V_3$ in the voltage $V_3$ across the main capacitor $C_{31}$ is calculated from the following Eq. (24):

$$\Delta V_3 = \Delta C_1 V_{bias} / (C_1 + C_{31} + C_{32}) \qquad \cdots \cdots (24).$$

When the second line on the right-hand side of Eq. (4) is rewritten by using the main capacitor $C_{31}$ and the auxiliary capacitor $C_{32}$, the second line can be expressed by the following Eq. (25).
[Equation 9]

$$
\begin{aligned}
\frac{V_3}{V_{bias}} &= \frac{C_1}{(C_1 + C_{31} + C_{32})} \\
&= 1 - \frac{(C_{31} + C_{32})}{(C1 + C_{31} + C_{32})} \\
&= F(C_1, C_{31}) \qquad \cdots \cdots (25)
\end{aligned}
$$

The sensitivity with regard to the changes of the capacitance in the first variable capacitor $C_1$ that is one variable of the multivariable function $F(C_1, C_{31})$ in Eq. (25) can be expressed by the following Eq. (26).
[Equation 10]

$$\frac{\partial F(C_1, C_{31})}{\partial C_1} = \frac{(C_{31} + C_{32})}{(C1 + C_{31} + C_{32})^2} \qquad \cdots \cdots (26)$$

The sensitivity characteristics indicated by the multivariable function in Eq. (26) is the upward convex curve, when looking at the changes in the main capacitor $C_{31}$ that is the other variable.

Thus, when the multivariable function in Eq. (26) is partially differentiated with respect to $\partial C_{31}$ by paying attention to the value of the main capacitor $C_{31}$ as a variable, the following Eq. (27) is established.
[Equation 11]

$$
\begin{aligned}
\frac{\partial^2 F(C_1, C_{31})}{\partial C_{31} \partial C_1} &= \frac{1}{(C_1 + C_{31} + C_{32})^2} - \frac{2(C_{31} + C_{32})}{(C_1 + C_{31} + C_{32})^3} \\
&= \frac{C_1 - (C_{31} + C_{32})}{(C_1 + C_{31} + C_{32})^3} \qquad \cdots \cdots (27)
\end{aligned}
$$

Regarding the multivariable function indicated by Eq. (27), when the differentiated values in the partial differentiation with the values of the main capacitor $C_{31}$ as the variables is zero, the sensitivity indicated by Eq. (26) becomes the maximum value.

A case that a value of the partial differentiation in Eq. (27) is zero is a time that the value of the main capacitor $C_{31}$ as the variable of the partial differentiation is equal to a value at which a value of the auxiliary capacitor $C_{32}$ is subtracted from a value of the first variable capacitor $C_1$. That is, when the following Eq. (28) is established, the infinitesimal

changes $\Delta V_3$ in the voltage $V_3$ across the main capacitor $C_{31}$ is known to become the maximum:

$$C_{31} = C_1 \cdot C_{32} \qquad\qquad \text{--- --- (28)}.$$

Eq. (28) means that, if the value of the auxiliary capacitor $C_{32}$ is set large as $C_{32} \fallingdotseq C_1$, the value of the main capacitor $C_{31}$ can be small ($C_{31} << C_{32}$). In short, according to the structure of the AI semiconductor elements pertaining to the second embodiment illustrated in FIG. 14 and FIG. 15, etc., the film thickness $T_{ox1}$ of the first gate-insulating film 12 can be larger. However, the value of the auxiliary capacitor $C_{32}$ is required to be approximately equal to the value of the first variable capacitor $C_1$. Thus, there is a limitation of the value of the film thickness $T_{ox2}$ of the second gate-insulating films 16, by which the value of the auxiliary capacitor $C_{32}$ is determined.

Although the second gate-insulating films 16 are serving as the field insulating film, the values of the film thickness $T_{ox2}$ cannot be too large, without estimating the values of the first variable capacitor $C_1$. Thus, there is a limit of the values of the film thickness $T_{ox2}$ of the second gate-insulating films 16. As represented by Eq.(8) etc., the inter-electrode distances d are requested to be smaller. Therefore, the values of the first variable capacitor $C_1$ shall be larger, and even the values of the auxiliary capacitor $C_{32}$ are required to be larger values close to the values of the first variable capacitor $C_1$. When the value of the auxiliary capacitor $C_{32}$ is desired to be large, depending on the value of the first variable capacitor $C_1$, high dielectric constant films, such as $HfSi_xO_y$ film ( $\varepsilon_r$ = 11), $La_2O_3$ film ( $\varepsilon_r$ = 21), $ZrO_2$ film ( $\varepsilon_r$ = 25), $HfO_2$ film ( $\varepsilon_r$ = 26) and the like, may be used for at least a part of the second gate-insulating films 16.

When the value of the auxiliary capacitor $C_{32}$ is desired to be large, by using the $SiO_2$ film, having excellent interface characteristics with the Si surface, for the first gate-insulating film 12, carrier mobilities in the IG semiconductor elements can be increased, which can improve values of mutual conductance $g_m$ of the IG semiconductor elements. When high dielectric constant film is adopted for at least a part of the second gate-insulating films 16, the electric-field strength will also be increased around the periphery of the vibration-cavity 28. Thus, an effectiveness of improving the profile can be achieved as exemplified in FIG. 6A, such that the profile of the vibration electrode 25c is shaped to a topology, in which only the centre of the vibration electrode 25c is deflected. In view of the effectiveness of improving the deformed profile of the vibration electrode 25c, a cross-sectional shape may be used in which the film thickness $T_{ox1}$ of the first gate-insulating film 12 made thick as the film thickness $T_{ox2}$ of the second gate-insulating films 16 so that the top surface can be planarized, not employing the cross-sectional shape of the wings in which the centre is concave as illustrated in FIG. 14 and FIG. 15, etc. By making the film thickness $T_{ox1}$ of the first gate-insulating film 12 thick, dielectric break-down voltage can be improved.

Here, an inter-electrode distance between the vibration electrode 25c and the floating electrode 17q is assumed to be d = 100 nm, and the first variable capacitor between the vibration electrode 25c and the floating electrode 17q is assumed to be $C_1$ = 79.6fF $\fallingdotseq$ 80fF. Moreover, when the inter-electrode distance between the vibration electrode 25c and the floating electrode 17q is assumed to be changed by infinitesimal displacement $\Delta d$ = 10 nm and the value of the first variable capacitor $C_1$ changes by $\Delta C_1$ = 10%, $\Delta C_1 \fallingdotseq 8fF$. When the above-mentioned values such as the auxiliary capacitor $C_{32}$ = 44fF and the main capacitor $C_{31}$ = 2.8fF $\fallingdotseq$ 3fF are substituted into Eq. (24), the following Eq. (29) is obtained:

$$\Delta V_{3} = 8/(44+3+80)V_{bias} = (8/127)V_{bias} \qquad\qquad \text{--- --- (29)}.$$

When the DC bias-voltage at the second potential is $V_{bias}$ = ten volts, the infinitesimal changes in the voltage $V_3$ across the main capacitor $C_{31}$ becomes $\Delta V_3$ = $V_{gs}$ = 0.63 volt.

With a channel width W, an effective channel length $L_{eff}$, a mobility $\mu_n$ of electrons in a semiconductor region and a capacitor $C_{ox}$ per unit area of a MOS capacitor, a mutual conductance $g_m$ of MOSFET is expressed by the following Eq. (30):

$$g_m = \mu_n C_{ox} W / L_{eff} \qquad\qquad (30).$$

In view of the structure of the AI semiconductor element pertaining to the second embodiment which has the above-mentioned dimension, for example, an approximate value of $g_m$ = 3.45 $\times$ 10$^{-6}$ is obtained from Eq. (30). Thus, according to the AI semiconductor element pertaining to the second embodiment, even if the source-drain voltage is $V_{ds}$ = one volt, by using the above-mentioned gate-source voltage $V_{gs}$ = 0.65 volt, the changes of $I_{ds}$ = 2.17 $\mu$A can be obtained.

(THIRD EMBODIMENT)

In an AI semiconductor element pertaining to a third embodiment of the present invention, as illustrated in FIG. 16 to FIG. 18, a structure is explained in which the floating electrode 17q of the AI semiconductor element of the second embodiment is connected to the first potential (ground potential) through a delay resistor $R_{GND}$ selected to a predetermined value. As will be described later, when the first variable capacitor is $C_1$ = 40 to 100 fF, the delay resistor may be selected within a range of $R_{GND}$ = 0.3mega-ohms to ten giga-ohms, for a frequency of ultrasonic waves of 1 to 10 MHz. Because the values of the changes $\Delta V_3$ in the gate voltages of the nMOSFET indicated in the already-explained Eq. (7) are functions that decrease when the inter-electrode distance d between the vibration electrode and the floating electrode is increased, the inter-electrode distance d shall be smaller. Also, for the structure indicated in the AI semiconductor elements pertaining to the first embodiment, to maximize the changes $\Delta V_3$ in the gate voltages $V_3$ from Eq. (14), a request is generated in which the film thickness $T_{ox}$ of the gate-insulating film is desired to be thinned depending on the inter-electrode distance d. Moreover, for the structure of the AI semiconductor element in the second embodiment, as represented by Eq. (28), a request is generated in which the value of the main capacitor $C_{31}$ is equal to a value at which the value of the auxiliary capacitor $C_{32}$ is subtracted from the value of the first variable capacitor Ciso that the infinitesimal changes $\Delta V_3$ in the voltage $V_3$ across the main capacitor $C_{31}$ can be increased to maximum.

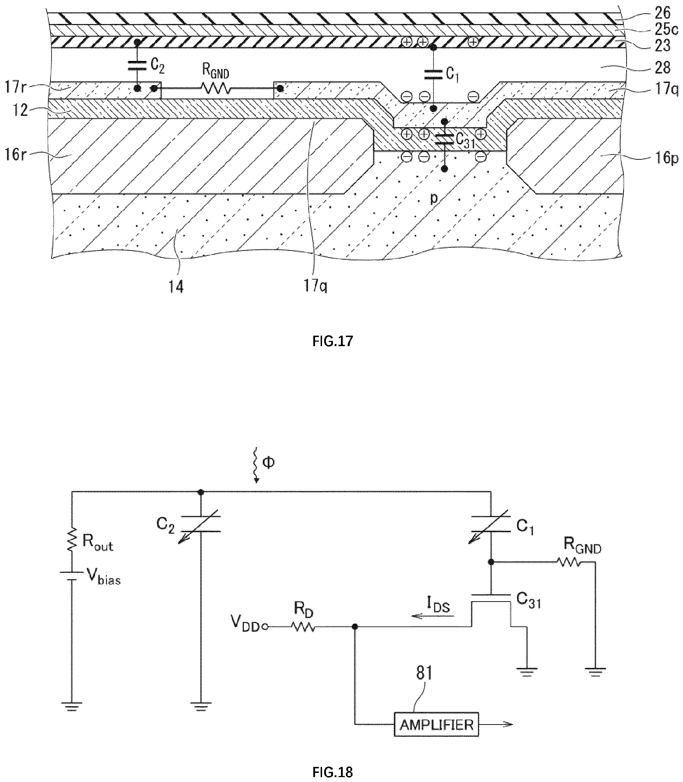

An area of the floating electrode 17c is defined $S_1$, an inter-electrode distance between the vibration electrode 25c and the floating electrode 17q, establishing the first variable capacitor $C_1$, is defined as d, a thickness of the gate-insulating film establishing the main capacitor $C_{31}$ is defined as $T_{ox1}$ and a thickness of the gate-insulating film establishing the auxiliary capacitor $C_{32}$ is defined as $T_{ox2}$. In addition, although the illustration of the cavity-surrounding insulating-film constructing the vibration-cavity on the floating electrode 17q is omitted in the cross-sectional view in FIG. 17, the cavity-surrounding insulating-film surrounds the vibration-cavity as the hermetically confined space, the cavity-surrounding insulating-film 20 can be included like the configuration illustrated in FIG. 3, as a matter of course. When an area of a main floating-electrode area constructing the main capacitor $C_{31}$ is defined as $\alpha S_1$ and an area of an auxiliary-floating electrode-portion constructing the auxiliary capacitor $C_{32}$ is defined as $\beta S_1$, ($\alpha + \beta = 1$), similarly to the calculation of Eq. (7), the following Eq. (31) and Eq. (32) are established:

$$C_{31} = \varepsilon_r \varepsilon_0 \alpha S_1 / T_{ox1} \qquad \text{--- --- (31)}$$

$$C_{32} = \varepsilon_r \varepsilon_0 \beta S_1 / T_{ox2} \qquad \text{--- --- (32)}$$

The $\alpha$ and $\beta$ in Eq. (31) and Eq. (32) determine a distribution rate of an electrode area of the main floating-electrode area constructing the main capacitor $C_{31}$ and an electrode are of the auxiliary-floating electrode-portion constructing the auxiliary capacitor $C_{32}$, ($\beta$ = 1- $\alpha$).

When Eq. (2), Eq. (31) and Eq. (32) are substituted into the first line on the right-hand side of Eq. (25), the following Eq. (33) can be expressed.

[Equation 12]

$$\frac{V_3}{V_{bias}} = \frac{\varepsilon_0 S_1 / d}{\varepsilon_0 S_1 / d + \varepsilon_0 \varepsilon_r S_1 \alpha / t_{0X1} + \varepsilon_0 \varepsilon_r S_1 \beta / t_{0X2}}$$
$$= \frac{1}{1 + \varepsilon_r (\alpha / t_{0X1} + \beta / t_{0X2}) d} \qquad \text{········ (33)}$$

Eq. (33) can be rewritten to the following Eq. (34).

[Equation 13]

$$V_3 = \frac{1}{1 + \varepsilon_r (\alpha / t_{0X1} + \beta / t_{0X2}) d} V_{bias} \qquad \text{········ (34)}$$

When Eq. (34) is differentiated with respect to $\partial$ d, the following Eq. (35) is understood to be established.

[Equation 14]

$$\frac{\partial V_3}{\partial d} = - \frac{\varepsilon_r (\alpha / t_{0X1} + \beta / t_{0X2})}{(1 + \varepsilon_r (\alpha / t_{0X1} + \beta / t_{0X2}) d)^2} V_{bias} \qquad \cdots\cdots (35)$$

In Eq. (35), by focusing to the value of $\alpha \varepsilon_r / T_{ox1}$ as a variable, let's speculate an optimal film thickness $T_{ox1}$ of the gate-insulating film in the effective gate region, which is required for increasing the changes $\Delta V_3$ in the gate voltages.

Eq. (35) is the multivariable function of the same form as Eq. (26). Thus, the partial differentiation with respect to the $\partial T_{ox1}$ in Eq. (35) is equivalent to the partial differentiation with respect to the $\partial C_{31}$ of the multivariable function in Eq. (26). Thus, similarly to Eq. (28), when the following Eq. (36) is established, Eq. (35) has the maximum value.

[Equation 15]

$$\frac{\alpha \varepsilon_r}{t_{0X1}} = \frac{1}{d} - \frac{\beta \varepsilon_r}{t_{0X2}} \qquad \cdots\cdots (36)$$

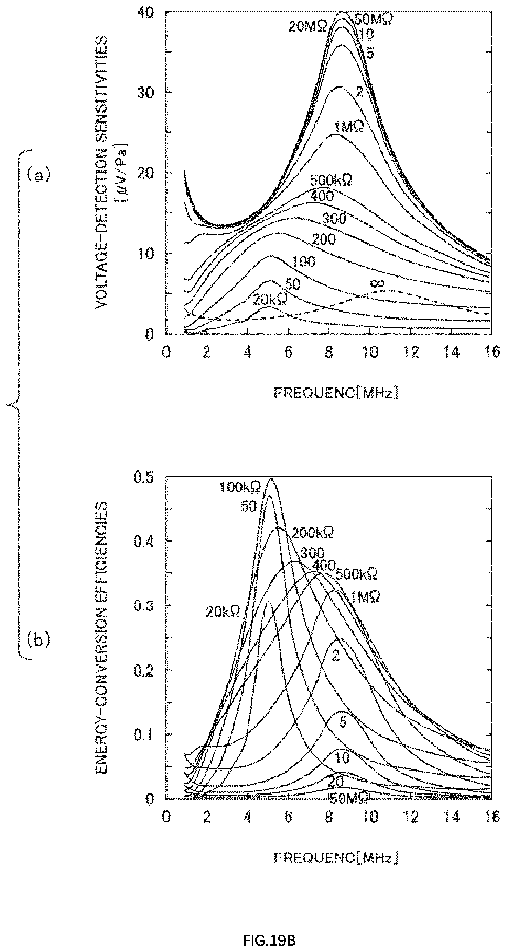

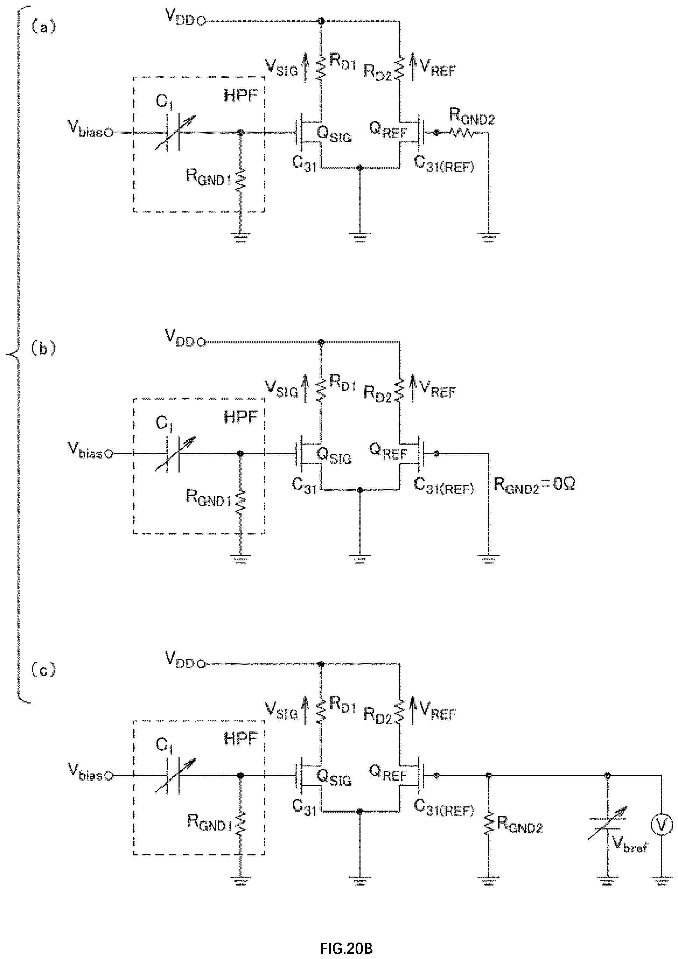

However, when the above requests are considered, a case occurs in which the thickness $T_{ox1}$ of the gate-insulating film for the effective gate region is insufficient with respect to the desired voltage $V_3$, in view of the break-down voltage. Therefore, in a case of the first variable capacitor $C_1$ = 40 to 100 fF, a value of a delay resistor $R_{GND}$ of about 0.3mega-ohms to one giga-ohm is selected, and the floating electrode 17q is grounded through the delay resistor $R_{GND}$. Then, the values of the delay resistor $R_{GND}$ directed to higher sensitivity may be determined by speculating capacitance values of the first variable capacitor $C_1$, which are addressed to signal processing, in such a way that an RC time-constant $\tau$ of a high pass filter (HPF) exemplified in FIG. 20B, which will be described later, complies with a frequency required by target-specification. As can be understood from sensitivity curves illustrated in FIG. 19B (a), which will be explained later, values of the delay resistor $R_{GND}$ may be selected to about one mega-ohms to one giga-ohm, in view of the relations with the RC time-constant $\tau$. The delay resistor $R_{GND}$ for signal processing can be implemented by, for example, an buried layer embedded in the channel-generating region 14, a surface impurity-doped layer (surface-buried layer) diffused in the top surface at the surface of the channel-generating region 14, or a surface-wiring layer such as DOPOS film or metallic thin film laminated on top surface of the first gate-insulating film 12.

When ultrasonic intensity is $1 W/m^2$, water particles of velocity v are expressed by the following Eq. (37):

$$v = (2 \times 1 W/m^2/(1.5 \times 10^6 kg/m^2/s)^{1/2}$$
$$= 1.16 \times 10^{-3} m/s$$
$$= 1.16 mm/s \qquad \cdots\cdots (37).$$

When the particle velocity v in Eq. (37) is converted into displacement $\Delta d$ at 1MHz, the displacement $\Delta d$ is expressed by the following Eq. (38):

$$\Delta d = 1.16 \times 10^{-3} m/s/(2\pi \times 10^6/s)$$
$$= 1.7 \times 10^{-10} m$$
$$= 0.17 nm \qquad \cdots\cdots (38).$$

Eq. (7) and Eq. (35), which corresponds to Eq. (7), explain that the inter-electrode distance d between the vibration electrode 25c and the floating electrode 17q shall be smaller, for increasing the sensitivity caused by the variation of displacement $\Delta d$. Here, when the inter-electrode distance d is assumed as d = 100 nm, Eq. (38) means the displacement $\Delta d$ of the inter-electrode distance d is 0.17%.

Similarly to the AI semiconductor element of the second embodiment, with the inter-electrode distance d = 100 nm, when the first variable capacitor $C_1$ = 79.6fF is assumed to be generated between the vibration electrode 25c and the floating electrode 17q, $\Delta C_1$ = 0.1361F is acquired by the displacement $\Delta d$ = 0.17%. Moreover, similarly to the AI semiconductor element of the second embodiment, under conditions that a film thickness $T_{ox1}$ of the second gate-insulating films 16 is 700 nm, a dimension of the floating electrode 17q sandwiching the effective gate electrode is 30 micrometers $\times$ 15 micrometers, a film thickness $T_{ox1}$ of the second gate-insulating films 16 is 800 nm, a gate width W of the effective gate electrode is four micrometers, an effective channel length $L_{eff}$ is two micrometers and a film thickness $T_{ox2}$ of the first gate-insulating film 12 is 100 nm, the first variable capacitor becomes $C_1$ = 80fF, the auxiliary capacitance becomes $C_{32}$ = 44fF, and the main capacitance becomes $C_{31}$ = 3fF. Thus, from Eq. (18), for the variation

of $\Delta C_1$ = 0.136fF, the voltage-variation $\Delta V_3$ in the voltage $V_3$ across the main capacitor $C_{31}$ is represented by Eq. (39), and the voltage-variation $\Delta V_3$ depends on the DC bias-voltage $V_{bias}$ at the second potential:

$$\Delta V_3 = 0.136/(44+3+80)V_{bias}$$
$$= 1.07 \times 10^{-3}V_{bias} \qquad \text{--- ---}(39).$$

From the following Eq. (39), when the DC bias-voltage is $V_{bias}$ = 100 volts, the variation of the voltage $V_3$ across the main capacitor $C_{31}$ becomes $V_3 = \Delta V_{gs}$ = 0.11 volt:

As can be understood from the first line on the right-hand side of Eq. (25), the voltage $V_3$ across the main capacitor $C_{31}$ depends on the DC bias-voltage $V_{bias}$. Similarly to Eq. (39), when the first variable capacitor is assumed as $C_1$ = 80F, the auxiliary capacitance is assumed as $C_{32}$ = 44fF and the main capacitance is assumed as $C_{31}$ = 3fF, the first line on the right-hand side of Eq. (25) is expressed as the following Eq. (40), which represents a dependent form on the DC bias-voltage $V_{bias}$:

$$V_3 = 80V_{bias}/(44+3+80)$$
$$= (80/127)V_{bias} \qquad \text{--- ---} (40)$$

Eq. (40) means that, when the DC bias-voltage $V_{bias}$ = 100 volts is applied across both terminals of the series circuit composed of the main capacitor $C_{31}$ and the first variable capacitor $C_1$, the voltage across the main capacitor $C_{31}$ implementing an insulated gate (IG) type capacitor becomes $V_3$ = 63 volts. Therefore, a problem of break-down voltage in a film thickness $T_{ox2}$ = 100 nm occurs, regarding the first gate-insulating film 12 implementing the IG type capacitor. In view of the problem of break-down voltage, in the AI semiconductor elements of the third embodiment, when the first variable capacitor $C_1$ = 40 to 100 fF, the delay resistor $R_{GND}$ having values of about 0.3mega-ohms to one giga-ohms is connected between the floating electrode 17q and the fixed-potential electrode17r, as illustrated in FIG. 16 to FIG. 18. Since the fixed-potential electrode17r is set at ground potential (first potential), the floating electrode 17q is connected to the first potential (ground potential) through the delay resistor $R_{GND}$ of about 0.3mega-ohms to one giga-ohms, and the break-down voltage against the DC voltage across the first gate-insulating film 12 is guaranteed.

For example, when the delay resistor $R_{GND}$ of about one mega-ohms is connected to the floating electrode 17q serving as the first variable capacitor of $C_1$ = 100 fF, a RC time-constant is $\tau$ = about $1 \times 10^{-7}$s. With RC time-constant, since HPF cutoff-frequency becomes $f_{cHP}$ = $1/(2\pi\tau)$ = 1.6 MHz, lower frequency signals of MHz order or less and DC signal cannot be applied to the floating electrode 17q. Thus, the floating electrode 17q becomes in a pseudo-floating state of high impedance for the lower frequency signals of MHz order or less and the DC signal. Additionally, in the explanations of the AI semiconductor elements of the third embodiment, electric states that are in high-impedance states (pseudo-floating states) for lower frequency signals of targeted frequency bands or less and DC signal are called "at least pseudo-floating states". "At least pseudo-floating states" are concepts inclusively including "floating states" used in the explanations of the AI semiconductor elements pertaining to the first and second embodiments. That is, "at least pseudo-floating states" means states of perfectly floating states or states connected to the first potential with the delay resistor $R_{GND}$ determined by a selected cutoff-frequency. When a reference cutoff-frequency $f_{refe}$ is defined for a specific frequency of a specification addressing to a predetermined purpose, a minimum delay resistor $R_{GNDmin}$ for realizing the pseudo-floating state can be determined. When the minimum delay resistor $R_{GNDmin}$ is determined for a desired frequency band, in higher frequency bands, for example, of MHz order or more, conditions for which the voltage $V_3$ across the main capacitor $C_{31}$ can be changed at high sensitivity by capacitance capacitance-variations $\Delta C_1$ in the first variable capacitor $C_1$ are determined, while keeping the break-down voltages of the first gate-insulating film 12. For example, in a state in which a DC bias-voltage $V_{bias}$ = 100 volts is applied across both terminals of the series circuit composed of the main capacitor $C_{31}$ and the first variable capacitor $C_1$, ultrasonic waves at 1 MHz are irradiated with strength of 1W/m_2, the inter-electrode distance d is displaced by infinitesimal displacement $\Delta d$ = 0.17nm as represented by Eq. (38). Within the meaning of DC, because the floating electrode 17q is grounded in the pseudo-floating state, all the DC bias-voltages $V_{bias}$ are applied between the floating electrode 17q and the upper electrode 25b. Therefore, contrasting with a state which lacks the delay resistor $R_{GND}$- -namely, the state of perfectly floating- -,the upper electrode 25b is more attracted, and the sensitivity is improved in the pseudo-floating state, which will be explained later.

At inter-electrode distance d = 100 nm, when $V_1$ = 100 volts is applied between the vibration electrode 25c and the floating electrode 17q, and the first variable capacitor defined by the vibration electrode 25c and the floating electrode 17q is assumed as $C_1$ = 80 fF, variations $\Delta q$ of the charges in the first variable capacitor $C_1$ caused by the displacement $\Delta d$ = 0.17 nm is expressed by the following Eq. (41):

$$\Delta q = \Delta C_1 \cdot V_1$$
$$= 80 \text{ fF} \times 0.17\% \times 100 \text{ volts}$$
$$= 13.6 \text{fQ} \qquad \text{--- --- (41).}$$

Similarly to the structure illustrated in FIG. 14, the charges $\Delta q$ in Eq. (41) are induced in the parallel capacitor$(C_{31}+C_{32})$ = 47 fF made of the auxiliary capacitor $C_{32}$ and the main capacitor $C_{31}$even in the AI semiconductor elements of the third embodiment. Therefore, the surface potential of the channel-generating region 14 illustrated in FIG. 17 in the AI semiconductor elements of the third embodiment changes by$\Delta q/(C_{31}+C_{32})$ = 0.29 volt, and the IG semiconductor element can operate.

A solid line in FIG. 19A (a) plots simulated results of voltage-detection sensitivities by a concentric-separated pattern with floating and fixed-potential electrodes. Voltage changes in the floating electrode in a divided-gate structure having the concentric-separated patterns are represented as the voltage detection sensitivities on left-hand ordinate in FIG. 19A (a), and frequency changes are represented on abscissa. In the simulation, "Piezo-phenomena and Ultrasonic-simulation Software PZFlex" is used which is firstly developed by U.S. Weidlinger Associates Corporation. In the simulation, both mechanical vibrations of the vibration-cavity associated with entered ultrasonic waves and the electric vibrations associated with the changes of the capacitance in the first variable capacitor $C_1$ by the mechanical vibration are considered.

Target substantially like the topology illustrated in FIG. 2, in which concentric-separated patterns encompassing an inner circular floating electrode and an outer fixed-potential electrode, is simulated. The circular floating electrode is arranged in a circular window cut in a centre of a circular pattern of the outer fixed-potential electrode, and the circular floating electrode is spaced apart from the fixed-potential electrode. A diameter of the circular floating electrode is 18.75 micrometers$^\Phi$, and a diameter of the outer fixed-potential electrode 17 is 60 micrometers$^\Phi$. An inner diameter of the fixed-potential electrode and an outer diameter of the floating electrode is separated from each other by 1.875 micrometers in a diameter direction. In the simulation, the central floating electrode is grounded through a resistor of one mega-ohms. The solid line in FIG. 19A (a) represents results for a case that the operation of the IG semiconductor element in the AI semiconductor element of the third embodiment is out of consideration, and the voltage changes of the gate electrode in the IG semiconductor elements are simulated as the voltage-detection sensitivities.

Dashed line in FIG. 19A (a) represents the voltage changes in the fixed-potential electrode, with respect to the left-hand ordinate as the voltage-detection sensitivities, for a case of a single pattern made of a uniform circular plate, and does not have the divided-gate structure such as the AI semiconductor element of the third embodiment. Dashed line in FIG. 19A (a) corresponds to a structure in which the entire region including the area of the corresponding portion of the fixed-potential electrode is set as the uniform floating electrode by omitting the pattern of the outer fixed-potential electrode, in contrast to the concentric-separated pattern embracing the floating electrode and fixed-potential electrode in the AI semiconductor elements of the third embodiment. The fixed-potential electrode targeted by the simulation corresponding to the plot by dashed line in FIG. 19A (a), is connected to the first potential (ground potential) through a resistor of 200 kilo-ohms, which is much smaller than the case of the divided-gate structure represented by solid line. Even dashed line in FIG. 19A (a) illustrates results in which the voltage changes of the gate electrode in the IG semiconductor elements are simulated as the voltage-detection results, in a case that the operation of the IG semiconductor element in the AI semiconductor element of the third embodiment is out of consideration, and the pattern of the outer fixed-potential electrode is omitted.

A dash-dotted line in FIG. 19A (a) plots charge-detection sensitivities measured by the fixed-potential electrode according to the earlier architecture on the right-hand ordinate, with respect to the frequency changes on abscissa, comparing the curves represented by the solid and dashed lines. In the simulation represented by dash-dotted line, similarly to the simulation represented by dashed line, the fixed-potential electrode is set to the uniform single pattern lacking the divided-gate structure. The structure in the periphery of the vibration-cavity 28 in the simulations represented by solid, dashed and dash-dotted lines in FIG. 19A (a) is substantially same as the structure of the AI semiconductor element pertaining to the improved structure of the first embodiment illustrated in FIG. 6C, except for the configuration that the IG semiconductor element is not provided. That is, in place of the IG semiconductor element, the fixed-potential electrode is delineated on the Si substrate as a pattern having the same radius as the vibration-cavity 28, in such a way that the patten of the bottom of the vibration-cavity is project onto the Si substrate.

And, on the fixed-potential electrode and the Si substrate around the fixed-potential electrode, the gate-insulating film (12 and 16) made of $SiO_2$having a thickness of 250 nm is stacked on the entire surface. The concentric-separated pattern represented by solid line in FIG. 19A (a) encompasses the floating electrode 17q and fixed-potential electrode 17o, which are made of DOPOS film having a thickness of 50 nm and are delineated on the gate-insulating film (12 and 16).In the cases of the uniform single pattern represented by dashed and dash-dotted lines in FIG. 19A (a), the fixed-potential electrodes made of the uniform DOPOS films, each having a thickness of 50 nm, are stacked on the gate-insulating film (12 and 16). An area outside of the fixed-potential electrode17o, a dummy layer made of DOPOS film having the same thickness of 50 nm as the fixed-potential electrode is stacked on the gate-insulating film (12 and 16).

Differently from the structure illustrated in FIG. 6C, in the simulation, the electric-field strengthened-layer 19 made of $Si_3N_4$ film is not used, and similarly to the structure illustrated in FIG. 3, the cavity-surrounding insulating-film 20made of $SiO_2$ film having a thickness of 100 nm, which surrounds the vibration-cavity 28, is stacked on the dummy layer of DOPOS film. And, the height of the vibration-cavity 28 is set to 100 nm in the simulation. Furthermore, a vibration-membrane 23 made of $Si_3N_4$ film having a thickness of 50 nm is laminated on the entire surfaces of the vibration-cavity 28 and the cavity-surrounding insulating-film 20 surrounding the vibration-cavity 28.On the vibration-membrane 23, a vibration electrode 25c made of AI film having a thickness of 50 nm is provided as a localized pattern to cover just above the vibration-cavity 28. In the practical simulation, analysis is conducted mainly in a state in which the vibration electrode 25c approaches the floating electrode 17q up to 50 to 60 nm that is equal to about half of the height of the vibration-cavity 28.

A $SiO_2$film having a thickness of50 nm, which is equal to the vibration electrode 25c,is provided as a donut-shaped pattern surrounding the vibration electrode 25c on the vibration-membrane 23, the donut-shaped pattern is disposed in a region surrounding the periphery of the vibration electrode 25c made of the localized pattern. On the vibration electrode 25c and the donut-shaped pattern made of $SiO_2$ film surrounding the vibration electrode 25c, a vibration-electrode protection-film 26 made of $Si_3N_4$ film having a thickness of 1155 to 1330 nm is stacked on the entire surface. Differently from the structure illustrated in FIG. 6C, the pattern of the stiffness-toughened lid 31c which is the localized pattern and made of $Si_3N_4$ film is not used in the simulation. In the simulation, a thick layer of water is assumed to further exist on the vibration-electrode protection-film 26. Also, the simulation is conducted such that a DC bias $V_{bias}$ of 130 volts as second potential is applied between the vibration electrode 25c and the fixed-potential electrode.

As can be understood from the simulation results represented by solid line in FIG. 19A (a), sensitivities of several microvolts/Pa to 13microvolts/Pa can be expected in a frequency band of 2 to 50 MHz, for the divided-gate structure in which the fixed-potential electrode is separated into the concentric pattern composed of the floating electrode and the fixed-potential electrode. On the other hand, as represented by dashed line in FIG. 19A (a), sensitivities of one microvolts/Pa to five microvolts/Pa can be expected in a frequency band of 2 to 50 MHz when the fixed-potential electrode is a uniform flat plate. The sensitivities of several microvolts/Pa are values same as the most widely used piezoelectric probe for medical diagnosis at present. Also, the above values are understood to have the sensitivities like a case of a charge-amplifier, which has a feedback capacitance of about 1pF being connected to an earlier cMUT of charge-detection architecture, to convert into voltages. Since the practical powers of ultrasonic waves are about kPa to MPa, solid and dashed lines in FIG. 19A (a) indicate that voltage variations from several milli-volts to several volts, or ten volts or more are obtained.

Additionally, attention shall be paid to a situation that the sensitivities of several microvolts/Pa obtained in the simulation in FIG. 19A (a) are results when the amplifying operation of the IG semiconductor element is out of consideration. Since the AI semiconductor element of the third embodiment encompasses the IG semiconductor element, it is necessary to consider an amplification factor of the IG semiconductor element such as the mutual conductance $g_m$ of the MOSFET represented by Eq.(9) and Eq.(30). When the amplification factor of the IG semiconductor element is considered, in the AI semiconductor element of the third embodiment, dramatically high sensitivities will be obtained compared with a case that the charge-amplifier is connected to the earlier cMUT of charge-detection architecture.

A comparison between the curves of solid and dashed lines in FIG. 19A (a) signifies that the voltage-detection sensitivities of the divided-gate structure, in which the fixed-potential electrode is separated into the concentric pattern, are higher than the voltage-detection sensitivities of the fixed-potential electrode implemented by the uniform flat plate. The reason why the voltage-detection sensitivities of the divided-gate structure are higher than the voltage-detection sensitivities of the uniform flat plate can be understand by the profile of the deformed shape of the vibration electrode as illustrated in FIG. 6A. As mentioned above, the profile illustrated in FIG. 6A represent the results of the simulation, in which only of the portion near the centre of the vibration electrode 25c is locally deflected, and the portion near the centre approaches the floating electrode 17q up to 50 to 60 nm. Therefore, the interval between the electrodes is not uniform because the flexure of the vibration electrode 25c becomes deep only in the centre, and the effective positions, at between the vibration electrode 25c and the floating electrode 17q or at between the vibration electrode 25c and the fixed-potential electrode17o, where the electric-field strengths become higher are distributed to be localized near the centre of the floating electrode 17q. Then, the reason why the voltage-detection sensitivities of the divided-gate structure are higher lies in the situation that the effective positions are localized near the centre of the floating electrode 17q.However,considering from reversed perspective, the situation that the effective positions are localized near the centre of the floating electrode 17q means that the pattern of the outer fixed-potential electrode17o may be omitted, in the concentric-separated pattern composed of the floating electrode 17q and the fixed-potential electrode17o, although the sensitivities will become lower. That is, attention shall be paid to a subject matter that the structure of the divided-electrode in which the fixed-potential electrode is separated into the floating electrode 17 and the fixed-potential electrode17o is not essential, in the present invention.

The curve represented by dash-dotted line in FIG. 19A (a) has a sharp resonant peak near a frequency of 12.5 MHz, although the curve of dash-dotted line illustrates the frequency dependence of the sensitivities by the charge-detection scheme of the earlier architecture for comparison. And, the curve represented by solid line in FIG. 19A (a) illustrates the frequency dependence of the sensitivities by the voltage-detection scheme, and the curve of solid line represents a resonant peak near a frequency of 12.5 MHz, and a full width at half maximum (FWHM) of the resonant peak of the voltage-detection scheme is extremely wide compared with the charge-detection scheme of dash-dotted line. Although the curve represented by dashed line in FIG. 19A (a) illustrates the frequency dependence of the sensitivities by the voltage-detection scheme, there is a resonant peak near a frequency of 17.5 MHz in the curve by dashed line. The FWHM of the resonant peak represented by dashed line in FIG. 19A (a) is extremely wide compared with dash-dotted line, and therefore, the voltage-detection schemes are known to be wider in frequency band compared with the charge-detection scheme.

In FIG. 19A (b), the simulation results represented by the curves of solid, dashed and dash-dotted lines correspond to the cases of the same line kinds as the curves of solid, dashed and dash-dotted lines illustrated in FIG. 19A (a), respectively. The targeted structures of the simulations in FIG. 19A (b) have respectively the common portions with the targeted structures of the simulations in FIG. 19A (a). However, in each of the targeted structures of the simulations in FIG. 19A (a), the vibration-electrode protection-film 26 made of $Si_3N_4$ film is in contact with the water layer. On the contrary, each of the targeted structures of the simulations in FIG. 19A (b) differs from the structures illustrated in FIG. 19A (a) in a feature such that a second vibration-electrode protection-film 33, which is made of epoxy resin having a thickness of four micrometers, is uniformly coated on the entire surface of the vibration-electrode protection-film 26 made of $Si_3N_4$ film, similarly to the structure illustrated in FIG. 8. Since the other features are equal to the structure explained in the simulation in FIG. 19A (a), the duplicated explanations are omitted. And, the second vibration-electrode protection-film 33 is in contact with the water layer. The situation that the simulation is conducted by applying the DC bias $V_{bias}$ of 130 volts between the vibration electrode 25c and the fixed-potential electrode is same as the situation of DC bias $V_{bias}$ illustrated in FIG. 19A (a).

Solid line in FIG. 19A (b) represents the voltage changes of the floating electrode in the divided-gate structure having the concentric-separated pattern composed of the floating electrode and the fixed-potential electrode are plotted as the voltage-detection sensitivities on ordinate, with respect to the frequency changes on abscissa. In the simulation, the central floating electrode is grounded through the resistor of one mega-ohms, similarly to FIG.19A (a). Solid line in FIG. 19A (b) represents the voltage changes of the gate electrode in the IG semiconductor element, which are simulated as the voltage-detection sensitivities, under a situation that the amplifying operation of the IG semiconductor element in the AI semiconductor element of the third embodiment is out of consideration.

Dashed line in FIG. 19A (b) represents results for a uniform single pattern lacking the divided-gate structure, and the voltage changes of the fixed-potential electrode are plotted as the voltage-detection sensitivities on ordinate. The fixed-potential electrode, which is targeted by the simulation corresponding to the plot by dashed line in FIG. 19A (b), is connected through a resistor of 200 kilo-ohms to the first potential (ground potential). Even dashed line in FIG. 19A (b) illustrates the results when the voltage changes of the gate electrode in the IG semiconductor element are simulated as the voltage-detection sensitivities, under a situation that the amplifying operation of the IG semiconductor element in the AI semiconductor element of the third embodiment is out of consideration, and that the pattern of the outer fixed-potential electrode is omitted. Dash-dotted line in FIG. 19A (b) plots charge-detection sensitivities for the earlier architecture on the right-hand ordinate, with respect to the frequency changes on abscissa. In the simulation curve represented by dash-dotted line, similarly to the simulation curve represented by dashed line, the uniform single pattern is elected as a target electrode of simulation, in which the fixed-potential electrode does not have the divided-gate structure.

As can be understood from the simulation results represented by solid line in FIG. 19A (b), for the divided-gate structure in which the fixed-potential electrode is separated into the concentric pattern composed of the floating electrode and the fixed-potential electrode, the sensitivities of 0.5 microvolts/Pa to six microvolts/Pa can be speculated in a frequency band of 2 to 50 MHz. However, the sensitivities are lower than FIG. 19A (a). On the other hand, in a case that the fixed-potential electrode is the uniform flat plate as represented by dashed line in FIG. 19A (b), the sensitivities of one microvolts/Pa to five microvolts/Pa can be speculated in a frequency band of 2 to 50 MHz. However, the sensitivities are lower than FIG. 19A (a). The sensitivities of several microvolts/Pa obtained by the simulation illustrated in FIG. 19A (b) exhibits the results when the amplifying operation of the IG semiconductor element is out of consideration. When the amplification factor of the IG semiconductor element is considered, in the AI semiconductor element of the third embodiment, dramatically high sensitivities are understood to be obtained compared with the earlier charge-detection scheme, even if the second vibration-electrode protection-film 33 made of epoxy resin is uniformly coated on the entire surface. In view of a situation that the practical powers of ultrasonic waves are kPa to MPa, solid and dashed lines in FIG. 19A (b) indicate that the voltage variations of several milli-volts to several volts can be obtained.

From the comparison between the curves represented by solid and dashed lines in FIG. 19A (b), voltage-detection sensitivities by the divided-gate structure are higher than the fixed-potential electrode with the uniform flat plate. However, considering from reversed perspective, the subject matter that voltage-detection sensitivities of the divided-gate structure are higher than the uniform flat plate means that, the pattern of the outer fixed-potential electrode may be omitted in the concentric-separated pattern, although the sensitivities may decrease. That is, in the present invention, the structure of the divided-electrode in which the fixed-potential electrode is separated into the floating electrode and the fixed-potential electrode is not essential.

The curve represented by dash-dotted line in FIG. 19A (b) illustrates the frequency dependence of the sensitivities by the scheme of the charge-detection method. However, curve represented by dash-dotted line has sharp resonant peaks at a low frequency side of 3 MHz or less and at near a frequency of 11 MHz. Even the curve represented by solid line in FIG. 19A (b) for the voltage-detection scheme has the resonant peak near the frequency of 12.5 MHz approximately equal to FIG. 19A (a). The FWHM of the peak of the voltage-detection scheme represented by solid line in FIG. 19A (b) is slightly wide than the FWHM of the peak of the charge-detection scheme represented by the curve represented by dash-dotted line near the frequency of 11 MHz. Although the curve represented by dashed line in FIG. 19A (b) is the voltage-detection scheme, there is the resonant peak near a frequency of 17 MHz approximately equal to the curve represented by dashed line in FIG. 19A (a). The FWHM of the peak represented by dashed line in FIG. 19A (b) is wider than dash-dotted line. Thus, frequency band of the voltage-detection scheme is wider than the charge-detection scheme.

To explain values of the delay resistor $R_{GND}$ used in the AI semiconductor element of the third embodiment, FIG. 19B (a) represents dependences of voltage-detection sensitivities of the floating electrode on the values of the ground resistor, which are simulated by the Piezo-phenomena and Ultrasonic-simulation Software PZFlex. That is, FIG. 19B (a) illustrates changes of the voltage-detection sensitivities as the voltage changes at the floating electrode, when the values of the delay resistor $R_{GND}$ serving as the ground resistor change, for the case of the divided-gate structure having the concentrically separated pattern composed of the floating electrode and the fixed-potential electrode, the divided-gate structure is represented by solid line in FIG. 19A (a). Similarly to the case of FIG. 19A (a), a diameter of the inner floating electrode is set to be18.75 micrometers$^{\Phi}$, and a diameter of the outer fixed-potential electrode is set to be 60 micrometers$^{\Phi}$. And, the simulation is conducted under a condition that an inter-electrode distance between the vibration electrode and the floating electrode is set as d = 134nm. Solid line in FIG. 19A (a) represents the simulated results of the voltage-detection sensitivities when the central floating electrode is grounded through the delay resistor $R_{GND}$ of one mega-ohms in the frequency band of 2 to 50 MHz. However, FIG. 19B (a) illustrates sensitivity-curve focusing to the frequency band of 1 to 16 MHz in the band out of 2 to 50 MHz. According to the sensitivity-curve in FIG. 19B (a), as the values of the delay resistor $R_{GND}$ through which the floating electrode is grounded is decreased such as the delay resistor $R_{GND}$ = 500 kilo-ohms→ 400 kilo-ohms→300 kilo-ohms→200 kilo-ohms- 100 kilo-ohms, the peak values of the resonant peaks appearing in the frequency dependence of the voltage-detection sensitivities may decrease compared with the sensitivity-curve for the delay resistor $R_{GND}$ = one mega-ohms. From a group of sensitivity-curves illustrated in FIG. 19B (a), the case of the delay resistor $R_{GND}$ = one mega-ohms can be judged to be the minimum delay resistor $R_{GND}$ relative to the structure exemplified in the AI semiconductor element of the third embodiment.

That is, when the value of the first variable capacitor $C_1$ is about 40 fF, employing values of the minimum delay resistor by which the floating electrode is grounded as $R_{GNDmin}$ = one mega-ohms or less is judged to be undesirable for acquiring higher sensitivities. Also, as the values of the delay resistor $R_{GND}$ are decreased such as the delay resistor $R_{GND}$ = 500 kilo-ohms →400 kilo-ohms→300 kilo-ohms→200 kilo-ohms- 100 kilo-ohms, the central frequencies of the resonant peaks appearing in the frequency dependence characteristics move to a lower frequency regime of about 5 MHz from the value of about 8.3 MHz with the minimum delay resistor $R_{GNDmin}$ = one mega-ohms. On the other hand, the sensitivity-curve in FIG. 19B (a) illustrates that, as the resistance values of the delay resistor $R_{GND}$ are increased such as the delay resistor $R_{GND}$ = two mega-ohms-five mega-ohms→ 10mega-ohms→20mega→ohms→ 20mega-ohms → 50mega-ohms, the peak values of the resonant peaks appearing in the frequency dependence characteristics of the voltage-detection sensitivities are increased compared with the case of the minimum delay resistor $R_{GNDmin}$ = one mega-ohms.

However, the increase in the peak values of the voltage-detection sensitivities begins to exhibit a tendency to saturation from the delay resistor $R_{GND}$ = about five mega-ohms. Thus, a condition that the values of the delay resistor $R_{GND}$ is set to be one to five mega-ohms or more is enough for keeping the voltage-detection sensitivities at high values, when the value of the first variable capacitor $C_1$ is about 40 fF. The condition that the values of the delay resistor $R_{GND}$, through which the floating electrode is grounded, are set to be one mega-ohms or more means that setting to a slightly larger values of the delay resistor $R_{GND}$ is preferable, compared with the resistance value defining the RC time-constant $\tau$ corresponding to the reference cutoff-frequency $f_{refe}$ predetermined from a design specification. Also, when the values of the delay resistor increase such as $R_{GND}$ = two mega-ohms-five mega-ohms→ 10mega-ohms→20mega-ohms→20mega→ohms→50mega-ohms, the central frequency of the resonant peaks appearing in frequency dependence characteristics are very slightly moved to higher frequency regime up to about 8.7 MHz from the value of about 8.3 MHzat the minimum delay resistor $R_{GNDmin}$ = one mega-ohms. In a case of the delay resistor $R_{GND}$ = 50mega-ohms, the voltage-detection sensitivities of 40 microvolts/Pa is obtained at 8.7 MHz. Although illustration is omitted because curves overlap with each other, the voltage-detection sensitivity becomes 40.05 microvolts/Paat the peak of 8.8 MHz for the delay resistor $R_{GND}$ = 100 mega-ohms, which is slightly increased from the peak value at the delay resistor $R_{GND}$ = 50mega-ohms, and it can be considered that the increase of the voltage-detection sensitivity is substantially saturated around the delay resistor $R_{GND}$ = 50 mega-ohms.

Moreover, in FIG. 19B (a), simulated results of frequency dependence of voltage-detection sensitivities for the delay resistor $R_{GND}$ = ∞, namely, in a case that the floating electrode is not grounded, is represented by the sensitivity-curve of dashed line. The peak value of the resonant peak appearing in the sensitivity-curve when the floating electrode is not grounded is about six microvolts/Pa, which is a small value similar to the case when the floating electrode is grounded through delay resistor $R_{GND}$ = 50 kilo-ohms. The peak value of the sensitivity-curve for the delay resistor $R_{GND}$ = ∞ is about one-sixth of the value of about 36 microvolts/Pa when the floating electrode is grounded through the delay resistor $R_{GND}$ = five mega-ohms. Also, the central frequency of the resonant peak of the sensitivity-curve when the floating electrode is not grounded is about 10.8 MHz. Thus, the central frequency of the resonant peak moves by about 2 MHz to higher frequency regime from the central frequency of the resonant peak when the floating electrode is grounded through the delay resistor $R_{GND}$ = five mega-ohms.

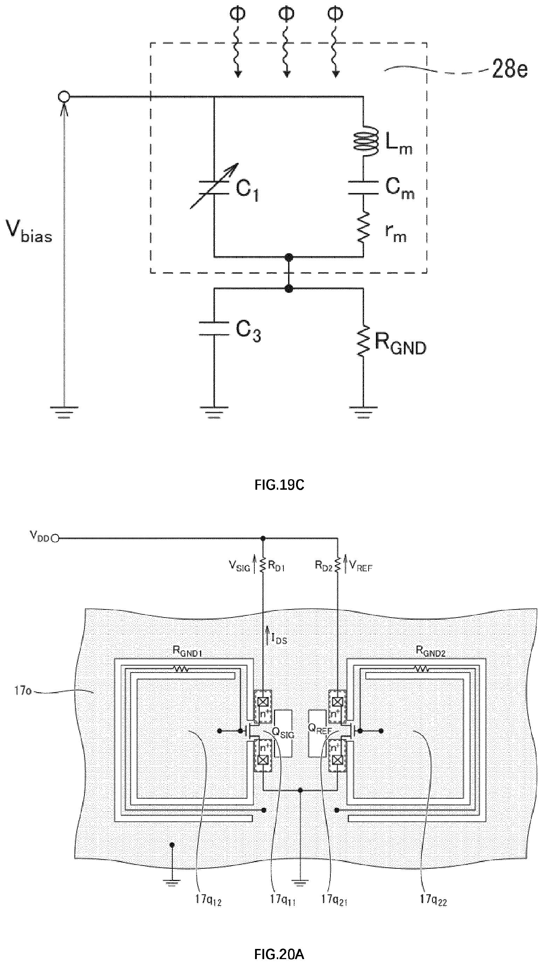

Comparing the sensitivity-curve of the floating electrode which is not grounded (delay resistor $R_{GND}$ = ∞) with a group of the sensitivity-curves of the floating electrodes which are grounded through various values of the delay resistors $R_{GND}$ as illustrated in FIG. 19B (a), voltage-detection sensitivities for floating electrodes, which are grounded through the delay resistors $R_{GND}$ of suitable values, are higher thana case when the floating electrode is not grounded. FIG. 19B (a) illustrates that sensitivities for the cases in which the floating electrode is grounded through the delay resistors $R_{GND}$ of the suitable values become higher, because the floating electrode can pull the vibration electrode more firmly. FIG. 19C illustrates an equivalent circuit model by electro-mechano-acoustical (EMA) analogy in which mechanical vibration system of mechanical-acoustics is treated in unified frame work with an equivalent circuit used in alternating current(AC) circuit-theory. A model of equivalent series inductance $L_m$ illustrated in FIG. 19C corresponds to a mass of a vibration electrode or a vibration membrane serving as a vibrating portion in the mechanical vibration system.

An equivalent series capacitor $C_m$ illustrated by using a graphic symbol in the AC circuit-theory in FIG. 19C is the mechanical compliance of the capacitive acoustic-element corresponding to the elasticity of the vibration electrode or vibration membrane in mechanical motion. An equivalent series resistor $r_m$ illustrated in FIG. 19C is lost components of vibration energy. The lost components of the vibration energy may include an inner friction when the vibration electrode is vibrated, a mechanical loss in the supporting system such as the cavity insulating film surrounding the vibration cavity of the capacitive acoustic-element, and an acoustic loss. An annular-enclosed vibration-cavity region 28esurrounded by a rectangular dashed line in FIG. 19C illustrates the vibration electrode (upper electrode) 25c and the lower electrode (17c, 17o), which sandwich the vibration-cavity 28 along up and down directions, and the region such as the cavity-surrounding insulating-film 20 surrounding the vibration-cavity 28horizontally, when the topology illustrated in FIG. 19C is taken as the cross-sectional view illustrated in FIG. 3.

The portion of the annular-enclosed vibration-cavity region 28e in the equivalent circuit by the EMA analogy, which is represented by the rectangular dashed line in FIG. 19C, is a well-known equivalent circuit in a field of mechanical vibration system such as the analysis of quartz crystal oscillator. In analogy with LCR resonant circuit by the AC circuit-theory, with the graphic symbols of the equivalent circuit in FIG. 19C, the annular-enclosed vibration-cavity region 28e can be expressed as a resonant circuit, which has a series resonant frequency $f_r$ represented by the following Eq. (42a) and a parallel resonant frequency (anti-resonant frequency) $f_a$ represented by the following Eq. (42b), which are known in the case of the crystal oscillator. The parallel resonant frequency $f_a$ is the resonant frequency that makes the impedance of the equivalent circuit in the portion of the annular-enclosed vibration-cavity region 28e infinite, and the series resonant frequency $f_r$ is the resonant frequency that makes the impedance infinitesimal.

[Equation 16]

$$f_r = \frac{1}{2\pi\sqrt{L_m C_m}} \quad \cdots\cdots (42a)$$

$$f_a = \frac{1}{2\pi\sqrt{L_m\left(\dfrac{C_m C_1}{C_m + C_1}\right)}} \quad \cdots\cdots (42b)$$

When the value of the mechanical compliance $C_m$, corresponding to the elasticity of the vibration electrode or vibration membrane illustrated in FIG. 19C, is sufficiently smaller than the value of the first variable capacitor $C_1$, namely, when the following Eq. (43):

$$C_m << C_1 \qquad\qquad \text{--- ---} (43)$$

is established, the following Eq. (44) can be established from Eq. (42a) and Eq. (42b):

$$F_r < f_a \qquad\qquad \text{--- ---} (44).$$

That is, the simulated results illustrated in FIG. 19B (a) signifies that, when the delay resistor $R_{GND}$ is large (impedance is large),the resonant peak becomes close to the anti-resonant frequency (parallel resonant frequency) $f_a$ on the higher frequency regime and when the delay resistor $R_{GND}$ is small (impedance is small), the resonant peak becomes close to the series resonant frequency $f_r$ on the lower frequency regime. In the example illustrated in FIG. 19B (a), there are a lower-resonant frequency $f_1$ of about 5 MHz and a higher-resonant frequency $f_2$ of about 8.7 MHz. The lower-resonant frequency $f_1$ is considered to correspond to the series resonant frequency $f_r$. Furthermore, as represented by dashed line in FIG. 19B (a), the resonant peak can be observed at frequency of about 10.8 MHz in the higher frequency regime, corresponding to the state when the floating electrode is not grounded - -in a case of a perfectly floating state- -. The resonant peak for the perfectly floating state - -open state- -represented by dashed line is considered to correspond to the parallel resonant frequency $f_a$.

FIG. 19B (a) illustrates that sensitivity-curve indicating voltage-detection sensitivities, for wide-band delay-resistors $R_{WBGND}$ = 300 kilo-ohms to 500 ohms, changes along a profile that is enlarged in a wide band. Namely, in a regime of the wide-band delay-resistors $R_{WBGND}$ = 300 kilo-ohms to 500 ohms, which gives the RC time-constant $\tau$ equivalent to the reference cutoff-frequency $f_{refe}$ of HPF, owing to the contributions from both of the higher-resonant frequency $f_2$ and the lower-resonant frequency $f_1$, the wide-band sensitivity-characteristic is established. To give one example, the reference cutoff-frequency $f_{refe}$ may be defined as a value close to the lower-resonant frequency $f_1$ or the higher-resonant frequency $f_2$, as represented by the following Eq. (45):

$$f_r = f_1 < f_{refe} < f_2 < f_a \qquad\qquad \text{--- ---} (45).$$

The values of the parallel resonant frequency $f_a$ and the series resonant frequency $f_r$ represented by Eq. (45) depend on the values of the equivalent series inductance $L_m$ and the mechanical compliance $C_m$ of the capacitive acoustic-element, and furthermore, the first variable capacitor $C_1$. The equivalent series inductance $L_m$ corresponds to the mass of the vibration electrode or vibration cavity represented in FIG. 19C, and the mechanical compliance $C_m$ corresponds to the elasticity of the vibration electrode or vibration cavity.

Although illustration is omitted, the simulation is executed even in a case that the values of the first variable capacitor $C_1$becomes smaller. That is, the simulation is performed even in a case that a diameter of the inner floating electrode is set as12.5 micrometers$\Phi$, a diameter of the outer fixed-potential electrode is set as 40 micrometers$\Phi$, a gap along a diameter direction between the fixed-potential electrode and the floating electrode is set as1.25 micrometers and an inter-electrode distance between the vibration electrode and the floating electrode is set as d = 180 nm. When the value of the first variable capacitor $C_1$becomes small, the resonant peak of the perfectly floating state ($R_{GND} = \infty$) becomes 9.4 MHz. Thus, a result is obtained in which the parallel resonant frequency $f_a$ moves to the lower frequency regime from 10.8 MHz illustrated in FIG. 19B (a). The shift of the parallel resonant frequency $f_a$ is ascribable to the contributions of the decrease in the values of the first variable capacitor $C_1$ in Eq. (42b). A result is obtained in which even the higher-resonant frequency fsslightly moves to the lower frequency regime, such as about 7.5 MHz, compared with a case illustrated in FIG. 19B (a) - - movement amount is small compared with the changes of the parallel resonant frequency $f_a$- -. The series resonant frequency $f_1$at lower frequency regime becomes about 4.3 MHz, which moves to the lower frequency regime as compared to the case illustrated in FIG. 19B (a). By considering Eq. (42a), the shift of the series resonant frequency $f_1$is ascribable to the changes in the equivalent series inductance $L_m$ and the mechanical compliance $C_m$. Also, the wide-band delay-resistor indicating the wide-band sensitivity-characteristics becomes $R_{WBGND}$ = one to two mega-ohms, which are larger values than the case illustrated in FIG.19B (a). The increase of $R_{WBGND}$ may be ascribable to a relatively larger contribution of the compensations of the decreases of values of the first variable capacitor $C_1$, which determine the RC time-constant $\tau$ ,by the increase of the wide-band delay-resistor $R_{WBGND}$, with respect to the changes in the reference cutoff-frequency $f_{refe}$.

The parallel resonant frequency $f_a$ corresponds to a mechanical resonant frequency for a situation that electromechanical coupling is small, the higher-resonant frequency $f_2$ corresponds to the resonant frequency for a situation that a positive feedback by the electromechanical coupling is weak. And the series resonant frequency $f_1$ (= $f_r$) at lower frequency regime corresponds to a frequency in which the elasticity of the vibration electrode becomes apparently soft due to the action of the positive feedback by the electromechanical coupling. Although illustration is omitted, the series resonant frequency $f_r$ allocated in the lower frequency regime is confirmed by the simulation to move toward the periphery of resonant frequency of the higher frequency regime which is not coupled to electricity when the DC bias-voltage $V_{bias}$ is decreased. For example, each of the series resonant frequencies $f_r$ admitted in the cases of the delay resistors $R_{GND}$ = one kilo-ohms and five kilo-ohms moves to the side of the parallel resonant frequency $f_a$ of 13 MHz from 5 MHz in the case of the DC bias-voltage $V_{bias}$ = 130 volts, when the DC bias-voltage is gradually decreased such as $V_{bias}$ = 130 volts→127 volts →123 volts→116 volts→109 volts→82 volts→58 volts. From reversed perspective, when the DC bias-voltage $V_{bias}$ is increased, the positive feedback by the electromechanical coupling is strengthen, which causes the resonant frequency to move to the side of the series resonant frequency $f_r$ of the lower frequency regime.

FIG. 19B (b) illustrates conversion-efficiency curves representing frequency dependences of energy-conversion efficiencies from ultrasonic waves to electricity. For the delay resistor $R_{GND}$ = 100 kilo-ohms, the energy-conversion efficiency at a frequency of 5 MHz is 0.5, which is the highest. However, when the values of the delay resistor $R_{GND}$ is increased such as the delay resistor $R_{GND}$ = 200 kilo-ohms→300 kilo-ohms→400 kilo-ohms→500 kilo-ohms-one mega-ohms, the central frequency of the peak of the conversion-efficiency curve changes toward 8.3 MHz and the peak value of the conversion-efficiency curve is also decreased. Moreover, when the delay resistor $R_{GND}$ is increased such as two mega-ohms-five mega-ohms→10mega-ohms →20mega-ohms→50mega-ohms, the central frequency of the peak of the conversion-efficiency curve changes toward 8.7 MHz, and the peak values are also decreased. Although illustration is omitted, under the conversion-efficiency curve of the delay resistor $R_{GND}$ = 50mega-ohms in FIG. 19B (b), at a level of about two-thirds of the peak value of the conversion-efficiency curve of the delay resistor $R_{GND}$ = 50mega-ohms, the conversion-efficiency curve of the delay resistor $R_{GND}$ = 100mega-ohms exists with the central frequency as common.

However, when the delay resistor $R_{GND}$ is increased such as the delay resistor $R_{GND}$ = 200 kilo-ohms→300 kilo-ohms→400 kilo-ohms→500 kilo-ohms, the FWHMs of the peaks of the conversion-efficiency curves spread. From FIG. 19B (b), the conversion-efficiency curves indicating the changes in energy-conversion efficiencies of the wide-band delay-resistors $R_{WBGND}$ = 300 kilo-ohms to 500 kilo-ohms are known to lie in a wide region. That is, when the values of the delay resistors $R_{GND}$ are the values of the wide-band delay-resistors $R_{WBGND}$ = 300 kilo-ohms to 500 kilo-ohms, integrated values in areas occupied by the conversion-efficiency curves are large compared with other cases of the delay resistors $R_{GND}$, and the energy-conversion efficiencies from the ultrasonic waves to the electricity are high. For wide-band delay-resistors $R_{WBGND}$ = 300 kilo-ohms to 500 kilo-ohms, because vibration energies, which will become cause of ringing phenomena, are absorbed as electric energy, frequency bands for electroacoustic conversion are enlarged. Moreover, FIG. 19B (b) illustrates that, when the values of the delay resistor $R_{GND}$ becomes larger than the minimum delay resistor $R_{GNDmin}$ = one mega-ohms or more, the energy-conversion efficiencies from the ultrasonic waves to the electricity are decreased.

From FIG. 19B (a) and FIG. 19B (b), in a case that the first variable capacitors $C_1$ = 40 to 100 fF are used and the ultrasonic waves of about 8 MHz is detected, the following two subject matters are known to be preferable:

(a) For the higher sensitivities, the minimum delay resistors $R_{GNDmin}$ = one mega-ohms or more is preferred.
(b) For the wider band, the wide-band delay-resistors $R_{WBGND}$ = 300 kilo-ohms to 500 kilo-ohms are preferred.

The wide-band sensitivity-characteristics represented by the curves of the wide-band delay-resistors $R_{WBGND}$ = 300 kilo-ohms to 500 kilo-ohms illustrated in FIG. 19B (a) and FIG. 19B (b) become extremely important for technical specification for imaging a high-definition image, in a case that the AEIC in which the AI semiconductor elements pertaining to the third embodiment are merged as unit cells on a common substrate is used for medical purpose.

## « FIRST VARIATION OF THIRD EMBODIMENT »

In an AI semiconductor element of a first variation of the third embodiment in the present invention, as illustrated in FIG. 20A and FIG. 20B (a), a signal-IG semiconductor element $Q_{SIG}$ for signal processing and a referential-IG semiconductor element $Q_{REF}$ for referential operation are arranged symmetrically in parallel sandwiching a fixed-potential electrode 17oin between. The signal-IG semiconductor element $Q_{SIG}$ and the referential-IG semiconductor element $Q_{REF}$ are designed such that they are identical to each other in dimensions, physical structures, and characteristics. However, as represented in an equivalent circuit in FIG.20B (a), the signal-IG semiconductor element $Q_{SIG}$ and the referential-IG semiconductor element differ from each other in configuration in which, although the first variable capacitor $C_1$ is connected to a gate electrode of the signal-IG semiconductor element $Q_{SIG}$, a capacitance corresponding to the first variable capacitor $C_1$ is not connected to a gate electrode of the referential-IG semiconductor element $Q_{REF}$.

Although illustration is omitted in FIG. 20A, for example, a shield plate made of W, etc., connected to the first potential (ground potential) is stacked at a level above a referential-main floating-electrode $17q_{21}$ serving as an effective gate electrode and a rectangular referential-auxiliary floating-electrode $17q_{22}$ which extends from the side of the referential-main floating-electrode $17q_{21}$ to a right direction. On the contrary, a vibration-cavity is disposed at a level above a signal-main floating-electrode $17q_{11}$ serving as an effective gate electrode and a rectangular signal-auxiliary floating-electrode $17q_{12}$ which extends from the side of the signal-main floating-electrode $17q_{11}$ to a left direction, and a gap of the vibration-cavity serves as the first variable capacitor $C_1$. As illustrated in FIG. 20A, by making an area of the referential-auxiliary floating-electrode $17q_{22}$ extending from the side of the referential-main floating-electrode $17q_{21}$ of the referential-IG semiconductor element $Q_{REF}$ to the right direction equal to an area of the signal-auxiliary floating-electrode $17q_{12}$ extending from the signal-main floating-electrode $17q_{11}$ of the signal-IG semiconductor element $Q_{SIG}$ to the left direction, the capacitive properties of the referential-IG semiconductor element $Q_{REF}$ and the signal-IG semiconductor element $Q_{SIG}$ are identical to each other.

The rectangular signal-auxiliary floating-electrode $17q_{12}$ extending from the signal-main floating-electrode $17qn$ constructing the signal-IG semiconductor element $Q_{SIG}$ illustrated on the left in FIG. 20A to the left direction is connected through signal-delay resistors $R_{GND1}$ of about 0.3mega-ohms to one giga-ohms to the first potential (ground potential). That is, as illustrated in an equivalent circuit in FIG. 20B (a), a high pass filter (HPF) made of the first variable capacitor $C_1$ and the signal-delay resistor $R_{GND1}$ is connected to the input circuit of the gate electrode of the signal-IG semiconductor element $Q_{SIG}$. On the other hand, the referential-auxiliary floating-electrode $17q_{22}$ extending from the referential-main floating-electrode $17q_{21}$ constructing the referential-IG semiconductor element $Q_{REF}$ illustrated on the right-hand side in FIG. 20A to the right direction is connected through a reference-delay resistors $R_{GND2}$ of one mega-ohms to one giga-ohms (hereafter, abbreviated as "about one mega-ohms or more") to the first potential (ground potential). As explained in Eq. (40), for example, when a DC bias-voltage $V_{bias}$ = 100 volts is applied across a signal-main capacitor $C_{31}$ and a signal first variable capacitor $C_1$, voltages across the terminals of the signal-main capacitor $C_{31}$ constructing an IG type capacitor is $V_3$ = 63 volts.

On the contrary, since the capacitance corresponding to the first variable capacitor $C_1$ is not connected to the gate electrode of the referential-IG semiconductor element $Q_{REF}$, a voltage is not electro-statically induced across the referential main capacitor $C_{31}$ implementing the IG type capacitor. Moreover, as illustrated in FIG. 20A, a signal-delay resistor $R_{GND1}$ having values of one mega-ohms or more is connected between an upper-right corner of the signal-auxiliary floating-electrode $17q_{12}$ and the fixed-potential electrode $17o$ surrounding the signal-auxiliary floating-electrode $17q_{12}$, the signal-auxiliary floating-electrode $17q_{12}$ extends from the signal-main floating-electrode $17q_{11}$ to the left direction. The fixed-potential electrode $17o$ encompasses a central strip-shaped pattern sandwiched between the signal-IG semiconductor element $Q_{SIG}$ and the referential-IG semiconductor element $Q_{REF}$, and peripheral patterns surrounding the signal-auxiliary floating-electrode $17q_{12}$ and the referential-auxiliary floating-electrode $17q_{22}$. The peripheral patterns are connected to the central strip-shaped pattern.

The signal-delay resistors $R_{GND1}$ can be implemented by an buried layer, a surface impurity-doped layer and a surface-wiring layer such as DOPOS film, metallic thin film, etc. The signal-delay resistors $R_{GND1}$ is curved at right angles along a C-shape, the C-shape surrounds the upper, left and lower latera of the signal-auxiliary floating-electrode $17q_{12}$. Namely, the C-shape extends from the upper-right corner of the signal-auxiliary floating-electrode $17q_{12}$, and connected to the fixed-potential electrode $17o$ near the lower-right corner of the signal-auxiliary floating-electrode $17q_{12}$. Also, a reference-delay resistor $R_{GND2}$ having values of about one mega-ohms or more is connected between the upper-left corner of the referential-auxiliary floating-electrode $17q_{22}$ extending from the referential-main floating-electrode $17q_{21}$ and the fixed-potential electrode $17o$ surrounding the referential-auxiliary floating-electrode $17q_{22}$. The reference-delay resistor $R_{GND2}$ can be implemented by a buried layer in such a way that the buried layer has the same impurity concentration, same depth, and same length as the signal-delay resistor $R_{GND1}$. Or alternatively, the reference-delay resistor $R_{GND2}$ can be implemented by a surface impurity-doped layer in such a way that the surface impurity-doped layer has the same impurity concentration and same depth, and same length as the signal-delay resistor $R_{GND1}$. Further alternatively, the reference-delay resistor $R_{GND2}$ can be implemented by a surface-wiring layer such that the surface-wiring layer is made of DOPOS film having the same impurity concentration and thickness, or such that the surface-wiring layer is made of metallic thin film having the same material and thickness, which are set to the same length as the signal-delay resistor $R_{GND1}$.

The reference-delay resistors $R_{GND2}$ is curved at a right angle in an inversed C-shape surrounding the upper, right and low latera of the referential-auxiliary floating-electrode $17q_{22}$ from the upper-left corner of the referential-auxiliary floating-electrode $17q_{22}$, and connected to the fixed-potential electrode $17o$ near the low left corner of the referential-auxiliary floating-electrode $17q_{22}$. The fixed-potential electrode $17o$ is set at ground potential (first potential). Thus, the signal-main floating-electrode $17q_{11}$ and the referential-main floating-electrode $17q_{21}$ are connected to the first potential (ground potential) through the signal-delay resistor $R_{GND1}$ and the reference-delay resistor $R_{GND2}$ each of which has the resistances of one mega-ohms or more, respectively. Then, the break-down voltage is guaranteed for the DC voltages of the signal first gate-insulating film and the referential first gate-insulating film.

A HPF illustrated in FIG. 20B (a) can cut off the signals of the lower frequency regime corresponding to RC

time-constants $\tau$ = 1 × 10$^{-7}$ s or less, by connecting the signal-delay resistor $R_{GND1}$ of about one mega-ohms to the signal-main floating-electrode 17q$_{11}$ in T-shape to construct the HPF. Thus, by the HPF, only a high frequency signals of MHz order or more are supplied to the signal-main floating-electrode 17q$_{11}$, and the DC bias-voltage $V_{bias}$ at a high voltage is grounded through the signal-delay resistor $R_{GND1}$. Similarly, by connecting the reference-delay resistor $R_{GND2}$ of about one mega-ohms to the referential-main floating-electrode 17q$_{21}$, HPF exhibits frequency characteristics of RC time-constant $\tau$ = about 1×10$^{-7}$s. Thus, the lower frequency signals of MHz order or less, or the DC signal cannot be transmitted to the referential-main floating-electrode 17q$_{21}$. Thus, the signal-main floating-electrode 17q$_{11}$ is in pseudo-floating state for the lower frequency signals of MHz order or less, or DC signal. That is, by the capacitance variations $\Delta C_1$ of the signal-first variable capacitor $C_1$ of MHz order, the voltagesVs across the signal-main capacitor $C_{31}$ can be changed while the break-down voltage of the signal-first gate-insulating film is kept. Similarly, the referential-main floating-electrode 17q$_{21}$ is in pseudo-floating state for the lower frequency signals of MHz order or less, or DC signal. Since the capacitance corresponding to the first variable capacitor $C_1$ is not connected to the gate electrode of the referential-IG semiconductor element $Q_{REF}$, the voltage $V_3$ across the referential main capacitor $C_{31}$implementing the IG type capacitor is not changed.

Even in the AI semiconductor element of the first variation in the third embodiment, the charges $\Delta q$ in Eq. (41) are induced in the parallel capacitor $(C_{31}+C_{32})$ of the signal-auxiliary capacitor $C_{32}$ and the signal-main capacitor $C_{31}$, similarly to the structure illustrated in FIG. 14. Therefore, the surface potential of the channel-generating region 14 in the signal-IG semiconductor element $Q_{SIG}$ changes by $\Delta q/(C_{31}+C_{32})$, and the signal-IG semiconductor element $Q_{SIG}$ can operate. Since the capacitance corresponding to the first variable capacitor $C_1$ is not connected to the gate electrode of the referential-IG semiconductor element $Q_{REF}$, the referential-IG semiconductor element $Q_{REF}$ keeps an initially-set constant operational state.

As illustrated in FIG. 20B (a), a voltage-signal output $V_{SIG}$ is taken out through an output resistor $R_{D1}$ from a second main-electrode region of the signal-IG semiconductor element $Q_{SIG}$, and a referential signals $V_{REF}$ is taken out through an output resistor $R_{D2}$ from a second main-electrode region of the referential-IG semiconductor element $Q_{REF}$. The referential signals $V_{REF}$ corresponds to the voltage-signal output $V_{SIG}$ when the signal-IG semiconductor element $Q_{SIG}$ is unloaded, namely, ultrasonic-wave signals are not entered to the signal-IG semiconductor element $Q_{SIG}$. To take out the referential signals $V_{REF}$ from the referential-IG semiconductor element $Q_{REF}$ always set to an unloaded state, both signal-IG semiconductor element $Q_{SIG}$ and the referential-IG semiconductor element $Q_{REF}$ may be designed as depression-mode MOSFETs that are equal in characteristics.

As illustrated in FIG. 20A, the signal-IG semiconductor element $Q_{SIG}$ and the referential-IG semiconductor element $Q_{REF}$, which are equal in characteristics and geometrical patterns, are arranged in parallel in mirror-symmetry, and by comparing the voltage-signal output $V_{SIG}$ and the reference signal $V_{REF}$, the noises of same phase can be removed, and cancel out the performance inconsistencies caused by process-steps of manufacturing the IG semiconductor elements. Especially, according to the AI semiconductor element of the first variation in the third embodiment, the influences of the potential fluctuations caused by the vibrations of the vibration electrode 25c can be cancelled out, which can increase the voltages applied to the vibration electrode 25c. By cancelling out the influences of the potential fluctuations caused by the vibrations, the height of the vibration-cavity can be reduced, and furthermore, the capacitance values of the first variable capacitor $C_1$ can be increased. When the capacitance values of the first variable capacitor $C_1$are increased, the infinitesimal changes $\Delta V_3$ of the voltage $V_3$ across the main capacitor $C_{31}$are increased as represented by Eq. (22). Thus, according to the AI semiconductor element of the first variation in the third embodiment, the capacitance values of the main capacitor $C_{31}$can be increased, which can increase the infinitesimal changes $\Delta V_3$ of the voltage $V_3$ applied to the first variable capacitor $C_1$ and can improve the detection sensitivities.

**« SECOND VARIATION OF THIRD EMBODIMENT »**

An AI semiconductor element pertaining to a second variation of the third embodiment of the present invention is assumed to have a structure in which similarly to the plan view illustrated in FIG. 20A, the signal-IG semiconductor element $Q_{SIG}$ for signal processing and the referential-IG semiconductor element $Q_{REF}$ for referential operation are arranged closely and parallel to each other, as a premise. The configuration in which the HPF made of the first variable capacitor $C_1$ and the signal-delay resistor $R_{GND1}$ is connected to the gate electrode of the signal-IG semiconductor element $Q_{SIG}$ is like the equivalent circuit illustrated in FIG. 20B (a). However, as exemplified in FIG. 20B (b), a situation that the reference-delay resistor $R_{GND2}$ is not connected to the gate electrode of the referential-IG semiconductor element $Q_{REF}$ differs from the equivalent circuit exemplified in FIG. 20B (a).

As described in the AI semiconductor element of the first variation in the third embodiment, the purpose of the configuration in which in the AI semiconductor element of the second variation, the signal-IG semiconductor element $Q_{SIG}$ and the referential-IG semiconductor element $Q_{REF}$ are arranged closely and parallel to each other is to remove the dark noise in the unloaded state (the case in which the ultrasonic waves are not irradiated) generated inside the signal-IG semiconductor element $Q_{SIG}$ and cancel out the performance inconsistencies caused by process steps in manufacturing the IG semiconductor elements.

Similarly to the AI semiconductor element pertaining to the first variation, even in the AI semiconductor element of the second variation, the shield plate made of W, etc., connected to the ground potential is arranged at a level above the referential-main floating-electrode $17q_{21}$ and the referential-auxiliary floating-electrode $17q_{22}$. That is, even in the AI semiconductor element of the second variation, the first variable capacitor $C_1$ is designed not to act for the referential-main floating-electrode $17q_{21}$ and the referential-auxiliary floating-electrode $17q_{22}$. Thus, there is no problem regarding the break-down voltage of the referential-IG semiconductor element $Q_{REF}$. In the equivalent circuit of the first variation illustrated in FIG. 20B (a), the gate electrode of the referential-IG semiconductor element $Q_{REF}$ is connected through the reference-delay resistor $R_{GND2}$ to the ground potential (first potential). However, for a purpose of setting the gate electrode to the ground potential, the reference-delay resistor $R_{GND2}$ is also unnecessary. Thus, in the second variation, the reference-delay resistor $R_{GND2}$ is removed, thereby simplifying the circuit configuration.

**« THIRD VARIATION OF THIRD EMBODIMENT »**

Although the illustration of a plan view is omitted, even in an AI semiconductor element pertaining to a third variation of the third embodiment of the present invention in which an equivalent circuit is illustrated in FIG. 20B (c), a signal-IG semiconductor element $Q_{SIG}$ for signal processing and a referential-IG semiconductor element $Q_{REF}$ for referential operation are arranged closely and parallel to each other. The situation that the purpose is to remove the dark noise in the unloaded state generated inside the signal-IG semiconductor element $Q_{SIG}$ and cancel out the performance inconsistencies caused by process steps in manufacturing the IG semiconductor elements is like the AI semiconductor elements pertaining to the first and second variations in the third embodiment. The configuration in which the HPF made of the first variable capacitor $C_1$ and the signal-delay resistor $R_{GND1}$ is connected to the gate electrode of the signal-IG semiconductor element $Q_{SIG}$ is like the equivalent circuit illustrated in FIG. 20B (a).

Although illustration is omitted, similarly to the configuration described in the AI semiconductor elements pertaining to the first and second variations, the shield plate made of W, etc., connected to the ground potential is arranged at a level above the referential-main floating-electrode $17q_{21}$ and the referential-auxiliary floating-electrode $17q_{22}$. Since the arrangement of the shield plate disables the first variable capacitor $C_1$ to act for the referential-main floating-electrode $17q_{21}$ and the referential-auxiliary floating-electrode $17q_{22}$, a high voltage is not generated in an effective gate region of the referential-IG semiconductor element $Q_{REF}$. In the equivalent circuit in the second variation illustrated in FIG. 20B (b), the reference-delay resistor $R_{GND2}$ is removed, and the gate electrode is directly grounded. However, in the equivalent circuit in the third variation illustrated in FIG. 20B (c), a predetermined reference gate voltages $V_{bref}$ is applied to the gate electrode of the referential-IG semiconductor element $Q_{REF}$. That is the first variable capacitor $C_1$ acts for the signal-main floating-electrode $17q_{11}$ so that a voltage equivalent to a voltage generated in the gate electrode of the signal-IG semiconductor element $Q_{SIG}$ can be applied to the gate electrode of the referential-IG semiconductor element $Q_{REF}$.

In the AI semiconductor element of the third variation in the third embodiment illustrated in FIG. 20B (c), the values of the reference gate voltages $V_{bref}$ can be adjusted while the voltages applied to the gate electrode of the referential-IG semiconductor element $Q_{REF}$ are being monitored with a voltmeter. As results, in the AI semiconductor element of the third variation, by controlling the gate voltages applied to the respective gate electrodes of the signal-IG semiconductor element $Q_{SIG}$ and the referential-IG semiconductor element $Q_{REF}$ which are arranged closely and parallel to each other to approximately the same value so that the operation characteristics of the signal-IG semiconductor element $Q_{SIG}$ and the referential-IG semiconductor element $Q_{REF}$ can be matched with each other. Since the gate voltages are controlled to approximately the same degree, an action for removing dark noises in the unloaded state and cancelling out the variations in the characteristics of the IG semiconductor elements can be realized with higher precision, compared with the AI semiconductor elements pertaining to the first and second variations.

**«FOURTH VARIATION OF THIRD EMBODIMENT»**

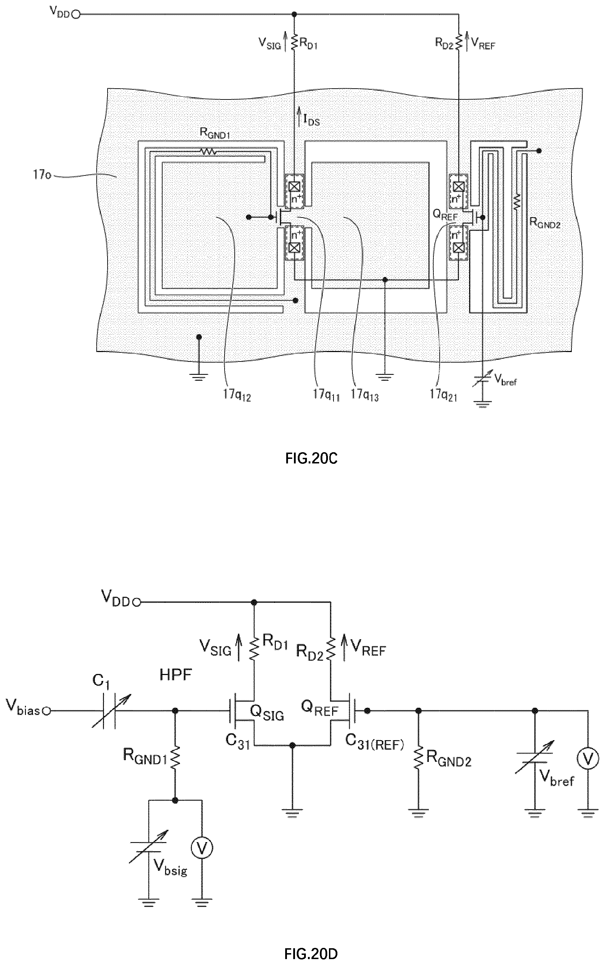

The IG semiconductor elements pertaining to the first to third variations in the third embodiment is organized such that the ultrasonic waves are not irradiated by arranging the shield plate made of W, etc., connected to the ground potential above the referential-main floating-electrode $17q_{21}$ and the referential-auxiliary floating-electrode $17q_{22}$, arranging the signal-IG semiconductor element $Q_{SIG}$ for signal processing and the referential-IG semiconductor element $Q_{REF}$ for referential operation closely and in parallel. In an AI semiconductor element pertaining to a fourth variation of the third embodiment of the present invention, a configuration illustrated in FIG. 20C, in which only a signal-IG semiconductor element $Q_{SIG}$ is arranged in the vibration-cavity and a referential-IG semiconductor element $Q_{REF}$ is arranged outside of the vibration-cavity will be explained, without arranging the shield plate. Since the referential-IG semiconductor element $Q_{REF}$ is arranged outside of the vibration-cavity, influences of the potential fluctuations due to the mechanical vibrations of the vibration electrode 25c,and influences of characteristics unevenness on the signal-IG semiconductor element $Q_{SIG}$ can be cancelled out by the signals from the referential-IG semiconductor element $Q_{REF}$.

For cancelling out the performance inconsistencies caused by planar distribution in process step of manufacturing the signal-IG semiconductor element $Q_{SIG}$, the signal-IG semiconductor element $Q_{SIG}$ and the referential-IG semiconductor element $Q_{REF}$ are preferred to be arranged at positions as close as possible. In a configuration that only the signal-IG semiconductor element $Q_{SIG}$ is arranged in the vibration-cavity and the referential-IG semiconductor element is arranged outside of the vibration-cavity, the signal-IG semiconductor element $Q_{SIG}$ must be arranged in the periphery of the vibration-cavity, under a requirement that the signal-IG semiconductor element $Q_{SIG}$ and the referential-IG semiconductor element $Q_{REF}$ shall be arranged closely to each other. However, as explained by FIG. 6A and FIG. 28, the capacitive acoustic-element has the problem in which the central portion of the vibration electrode is deeply deflected. The reason of the situation that voltage-detection sensitivity of the divided-gate structure in which the floating electrode is arranged at the centre of separated concentric patterns is higher than the case of the fixed-potential electrode of uniform flat plate as explained in FIG. 19A (a),depends on the non-uniform characteristics in the deformed shape of the vibration electrode.

Thus, giving priority to the voltage-detection sensitivity, when the planar pattern is designed, an arrangement in which a position of the signal-main floating-electrode $17q_{11}$ is selected to the centre of the vibration cavity and the referential-main floating-electrode $17q_{21}$ is arranged at a position spaced apart from the centre as illustrated in FIG. 20C must be selected. A layout in which the signal-main floating-electrode $17q_{11}$ illustrated in FIG. 20C is arranged at the centre of the vibration cavity exhibits a butterfly-shaped topology, in which a first signal-auxiliary floating-electrode $17q_{12}$ extends to the left of the signal-main floating-electrode $17q_{11}$ and a second signal-auxiliary floating-electrode $17q_{13}$ extends to the right of the signal-main floating-electrode $17q_{11}$. And, the referential-main floating-electrode $17q_{21}$ is arranged at a position that is allocated outside of the right-hand vibration cavity of the second signal-auxiliary floating-electrode $17q_{13}$.

However, since the planar layout illustrated in FIG. 20C is merely an exemplification, the planar layout is not limited to the exemplification illustrated in FIG. 20C. For example, in a case of a technical specification of design that does not prioritize the voltage-detection sensitivities, the signal-main floating-electrode $17q_n$may be arranged at a periphery of the vibration-cavity, and the referential-main floating-electrode $17q_{21}$may be arranged at a position outside of the vibration-cavity so that a proximity arrangement may be realized. The layout in which the signal-main floating-electrode $17q_{11}$ is arranged at the right periphery of the vibration-cavity as exemplified in FIG. 20C exhibits a single-winged butterfly-shaped topology in which the signal-auxiliary floating-electrode $17q_{12}$ extends only to the left of the signal-main floating-electrode $17q_{11}$. The configuration in which the HPF made of the first variable capacitor $C_1$ and the signal-delay resistor $R_{GND1}$ is connected to the gate electrode of the signal-IG semiconductor element $Q_{SIG}$ is like the AI semiconductor elements pertaining to the first to third variations in the third embodiment. As illustrated in FIG. 20C, the referential-main floating-electrode $17q_{21}$ is arranged outside of the vibration-cavity, and referential-auxiliary floating-electrode is not connected to the referential-main floating-electrode $17q_{21}$. Thus, the voltages caused by the ultrasonic waves are not generated by the effective gate region of the referential-IG semiconductor element $Q_{REF}$.

As illustrated in FIG. 20C, the fixed-potential electrode $17o$ is arranged in a periphery as a pattern surrounding the first signal-auxiliary floating-electrode $17q_{12}$, the second signal-auxiliary floating-electrode $17q_{13}$ and the reference-delay resistor $R_{GND2}$. The fixed-potential electrode $17o$ is a window-shaped pattern, which further includes a central and a right strip-shaped portions. The central strip-shaped portion is surrounded by the arrangement regions of the first signal-auxiliary floating-electrode $17q_{12}$ and the second signal-auxiliary floating-electrode $17q_{13}$. The right strip-shaped portion is sandwiched in between the arrangement region of the second signal-auxiliary floating-electrode $17q_{13}$ and the reference-delay resistor $R_{GND2}$.The signal-delay resistor $R_{GND1}$ is curved at right angles in a C-shape, surrounding the upper, left and lower latera of the signal-auxiliary floating-electrode $17q_{12}$. The signal-delay resistor $R_{GND1}$extends from the upper-right corner of the signal-auxiliary floating-electrode $17q_{12}$to be connected to the fixed-potential electrode $17o$ near the lower-right corner of the signal-auxiliary floating-electrode $17q_{12}$.

FIG. 20C exemplifies a meander line in which the reference-delay resistor $R_{GND2}$ meanders from the referential-main floating-electrode $17q_{21}$ to a right direction. To make approximately the same length as the signal-delay resistor $R_{GND1}$, the reference-delay resistor $R_{GND2}$ is arranged to meander with approximately the same line width as the signal-delay resistor $R_{GND1}$. Even in the AI semiconductor element of the fourth variation in the third embodiment, under a condition that the signal-delay resistor $R_{GND1}$ of one mega-ohms or more is connected to the signal-main floating-electrode $17q_{11}$ with the first variable capacitor $C_1$ = about 100 fF, a HPF is defined in the gate electrode of the signal-IG semiconductor element $Q_{SIG}$. Therefore, only high frequency signals of MHz order more are supplied to the signal-main floating-electrode $17q_{11}$ by the HPF, while the DC bias-voltage $V_{bias}$ at a high voltage is grounded through the signal-delay resistor $R_{GND1}$. That is, the voltages$V_s$ across the signal-main capacitor $C_{31}$, which are changed by the capacitance variations $\Delta C_1$ in the signal-first variable capacitor $C_1$, become the signals of MHz order, and the breakdown voltage of the first gate-insulating film can be maintained, for the generated voltages$V_3$.

Since the gate electrode of the referential-IG semiconductor element $Q_{REF}$ of the AI semiconductor element pertaining to the fourth variation of the third embodiment is allocated outside of the vibration-cavity, the voltage $V_3$ is not generated across the referential main capacitor $C_{31}$, and the voltage $V_3$ is not changed by irradiated ultrasonic waves.

As illustrated in FIG. 20C, from an external circuit, a reference gate voltage $V_{bref}$ can be applied to the referential-main floating-electrode $17q_{21}$ serving as the gate electrode of the referential-IG semiconductor element $Q_{REF}$. That is, similarly to the equivalent circuit illustrated in FIG. 20B (c), DC voltages, which have levels equivalent to voltages that occur in the gate electrode of the signal-IG semiconductor element $Q_{SIG}$ by the first variable capacitor $C_1$ in the vibration-cavity, or levels slightly lower than the voltages that occur in the gate electrode of the signal-IG semiconductor element $Q_{SIG}$, ca be applied to the gate electrode of the referential-IG semiconductor element $Q_{REF}$ outside of the vibration-cavity from the external circuit. When DC voltages are applied to the gate electrode of the referential-IG semiconductor element $Q_{REF}$, similarly to the configuration exemplified in FIG. 20B (c). The values of the reference gate voltage $V_{bref}$ can be adjusted by using a voltmeter, such that the voltages applied to the gate electrode of the referential-IG semiconductor element $Q_{REF}$ are adjusted, being monitored by the voltmeter.

Then, similarly to the third variation, the gate voltages applied to the respective gate electrodes of the signal-IG semiconductor element $Q_{SIG}$ being arranged in the vibration-cavity, and the referential-IG semiconductor element $Q_{REF}$ being arranged outside of the vibration-cavity can be controlled by voltages approximately equal, or the levels of the referential-IG semiconductor element $Q_{REF}$ are slightly lower than the signal-IG semiconductor element $Q_{SIG}$. By setting the levels of the gate voltages applied to the respective gate electrodes of the signal-IG semiconductor element $Q_{SIG}$ and the referential-IG semiconductor element $Q_{REF}$ to approximately equal, the operation characteristics of the signal-IG semiconductor element $Q_{SIG}$ in the vibration-cavity and the referential-IG semiconductor element $Q_{REF}$ outside of the vibration-cavity can be matched with each other.

As illustrated in FIG. 20C, the voltage-signal output $V_{SIG}$ is extracted through the output resistor $R_{D1}$ from the second main-electrode region of the signal-IG semiconductor element $Q_{SIG}$. Similarly, a reference signal $V_{REF}$ is extracted through an output resistor $R_{D2}$ from the second main-electrode region of the referential-IG semiconductor element $Q_{REF}$. The reference signal $V_{REF}$ is set to correspond with the voltage-signal output $V_{SIG}$ when the signal-IG semiconductor element $Q_{SIG}$ is unloaded, namely, when ultrasonic signals are not irradiated to the signal-IG semiconductor element $Q_{SIG}$. The gate electrode which is arranged outside of the vibration-cavity and always unloaded is biased to approximately the same reference gate voltages $V_{bref}$ as the gate voltages of the signal-IG semiconductor element $Q_{SIG}$.

As illustrated in FIG. 20C, by arranging the signal-IG semiconductor element $Q_{SIG}$ in the vibration-cavity and the referential-IG semiconductor element $Q_{REF}$ outside of the vibration-cavity, such that the signal-IG semiconductor element $Q_{SIG}$ and the referential-IG semiconductor element $Q_{REF}$ have identical characteristics, identical geometrical patterns and identical bias levels, and by comparing the voltage-signal output $V_{SIG}$ and the reference signal $V_{REF}$, the noises of the same phase can be removed. Especially, according to the AI semiconductor element of the fourth variation in the third embodiment, by using the signals from the referential-IG semiconductor element $Q_{REF}$ arranged outside of the vibration-cavity, the influence of the potential variability which is imparted to the signal-IG semiconductor element $Q_{SIG}$ by the vibration of the vibration electrode 25c can be cancelled out.

And, by cancelling out the influence of the potential variability which is imparted to the signal-IG semiconductor element $Q_{SIG}$ by the vibration of the vibration electrode 25c, the flexure of the vibration electrode 25c can be made deeper, which can increase a voltage applied to the vibration electrode 25c. By cancelling out the influence of the potential variability by the vibration, the proximity distance between the vibration electrode 25c and the floating electrode 17c can be made much smaller, which can make the height of the vibration-cavity low and increase the capacitance values of the first variable capacitor $C_1$. When the capacitance values of the first variable capacitor $C_1$ are increased, the infinitesimal changes $\Delta V_3$ of the voltage $V_3$ across the main capacitor $C_{31}$ represented by Eq. (22) are increased. Thus, according to the AI semiconductor element of the fourth variation in the third embodiment, the detection sensitivities can be improved by increasing amounts of the infinitesimal changes $\Delta V_3$ of the voltage $V_3$ applied to the main capacitor $C_{31}$.

FIG. 20C illustrates the configuration in which the reference gate voltages $V_{bref}$ can be applied to the gate electrode of the referential-IG semiconductor element $Q_{REF}$ from the external circuit. However, the above configuration is merely an exemplification. A configuration in which the reference gate voltages is $V_{bref}$ = 0 volt is allowable. That is, the reference gate voltages $V_{bref}$ may be connected through the reference-delay resistor $R_{GND2}$ to the ground potential. However, for a purpose in which the gate electrode of the referential-IG semiconductor element $Q_{REF}$ is set at ground potential, since the reference-delay resistor $R_{GND2}$ is also unnecessary, the reference-delay resistor may be set to $R_{GND2}$ = 0 ohms. Thus, similarly to the equivalent circuit exemplified in FIG. 20B (b), the reference-delay resistor $R_{GND2}$ may be removed to simplify the circuit configuration and the planar layout. For extracting the reference signal $V_{REF}$ from the referential-IG semiconductor element $Q_{REF}$ whose gate electrode is grounded, the referential-IG semiconductor element $Q_{REF}$ may be designed as depression-mode MOSFET. To make the characteristics of the signal-IG semiconductor element $Q_{SIG}$ and the referential-IG semiconductor element $Q_{REF}$ identical to each other, the signal-IG semiconductor element $Q_{SIG}$ may be also designed as the depression-mode MOSFET.

<<FIFTH VARIATION OF THIRD EMBODIMENT»

In the equivalent circuits illustrated in FIG. 18, FIG. 20B (a) to FIG. 20(c), the examples are illustrated in which the HPFs are defined by connecting the gate electrode through the delay resistors $R_{GND}$ or $R_{GND1}$ to the first potential (ground potential). However, in the equivalent circuit of the AI semiconductor element pertaining to the fifth variation of the third embodiment of the present invention illustrated in FIG. 20D, the signal-delay resistor $R_{GND1}$ is connected between the gate electrode of the signal-IG semiconductor element $Q_{SIG}$ and a voltage-supply applying signal-gate voltages $V_{bsig}$ (third potential). In FIG. 20D, there is a voltage-level raising corresponding to the gate voltages $V_{bsig}$ serving as the third potential. However, regarding a cutoff-frequency, the circuit topology illustrated in FIG. 20D is substantially equivalent to a configuration in which a HPF is connected to the gate electrode of the signal-IG semiconductor element $Q_{SIG}$. Here, the HPF is implemented by a circuit including the first variable capacitor $C_1$ and the signal-delay resistor $R_{GND1}$.

Because the signal-gate voltages $V_{bsig}$ are applied through the signal-delay resistor $R_{GND1}$ to the gate electrode of the signal-IG semiconductor element $Q_{SIG}$, voltages generated in the gate electrode of the signal-IG semiconductor element $Q_{SIG}$o wing to the changes of the capacitance in the first variable capacitor $C_1$ can be controlled to desirable values. As illustrated in FIG. 20D, the value of the signal-gate voltages $V_{bsig}$ may be adjusted by monitoring by the voltmeter. That is, in the AI semiconductor element pertaining to the fifth variation, because the gate-bias voltages of the signal-IG semiconductor element $Q_{SIG}$ can be controlled properly, the characteristics and current gains when the signal-IG semiconductor element $Q_{SIG}$ is operating can be optimized. Thus, by controlling the gate-bias voltage of the signal-IG semiconductor element $Q_{SIG}$, signal gains can be improved. Also, because the gate-bias voltages of the signal-IG semiconductor elements $Q_{SIG}$ can be freely selected, free degree of designing the IG semiconductor elements including depression mode, enhancement mode, etc. can be increased.

In the AI semiconductor element pertaining to the fifth variation of the third embodiment, a configuration in which the signal-IG semiconductor element $Q_{SIG}$ and the referential-IG semiconductor element $Q_{REF}$ are arranged in parallel can be employed like the AI semiconductor elements of the first to fourth variations of the third embodiment. However, since the configuration of the equivalent circuit illustrated in FIG. 20D is an exemplification, the configuration in which the referential-IG semiconductor element $Q_{REF}$ is arranged in parallel with the signal-IG semiconductor element $Q_{SIG}$ is not always essential. If the referential-IG semiconductor element $Q_{REF}$ is arranged closely in parallel with the signal-IG semiconductor element $Q_{SIG}$, by using the outputs from the referential-IG semiconductor element $Q_{REF}$, the noises generated in the signal-IG semiconductor element $Q_{SIG}$ under unloaded state can be removed, and cancel out the unevenness of the characteristics caused by process steps in manufacturing the IG semiconductor elements.

Similarly to the AI semiconductor elements of the first to third variations, the shield plate may be arranged at a level above the referential-main floating-electrode $17q_{21}$, or similarly to the AI semiconductor element of the fourth variation, the referential-main floating-electrode $17q_{21}$ may be arranged outside of the vibration-cavity. Due to the shielding effect by the shield plate or due to the external arrangement of the vibration-cavity, the first variable capacitor $C_1$ does not influence on the referential-main floating-electrode $17q_{21}$. Thus, a high voltage is not generated in the effective gate region of the referential-IG semiconductor element $Q_{REF}$. However, in the fifth variation represented by the equivalent circuit illustrated in FIG. 20D, a predetermined reference gate voltages $V_{bref}$ can be applied to the gate electrode of the referential-IG semiconductor element $Q_{REF}$. That is, although the voltage-level raisings can be executed by the values of the signal-gate voltages $V_{bsig}$, the voltages equivalent to potentials generated in the gate electrode of the signal-IG semiconductor element $Q_{SIG}$ owing to the changes of the capacitance in the first variable capacitor $C_1$can be applied to the gate electrode of the referential-IG semiconductor element $Q_{REF}$ by adjusting the reference gate voltages $V_{bref}$.

In the configuration in which the referential-IG semiconductor element $Q_{REF}$ is arranged in parallel with the signal-IG semiconductor element $Q_{SIG}$ as illustrated in FIG. 20D, the values of the reference gate voltages $V_{bref}$ can be adjusted, while the voltages applied to the gate electrode of the referential-IG semiconductor element $Q_{REF}$ are being monitored by the voltmeter. As results, in the AI semiconductor element pertaining to the fifth variation, the gate voltages applied to the respective gate electrodes of the signal-IG semiconductor element $Q_{SIG}$ and the referential-IG semiconductor element $Q_{REF}$ which are arranged closely to each other are controlled to values approximately identical to each other, or controlled to a desired voltage ratio so that the operation characteristics of the signal-IG semiconductor element $Q_{SIG}$ and the referential-IG semiconductor element $Q_{REF}$ can be matched each other. That is, in a case of adopting the configuration in which the $Q_{SIG}$ and the $Q_{REF}$ are arranged in parallel, by controlling the gate voltages of the $Q_{SIG}$ and the $Q_{REF}$ to approximately identical to each other, or controlling to the desired voltage ratio, the effectiveness of removing dark noises in the unloaded state, or the effectiveness of cancelling out the unevenness of the characteristics of the IG semiconductor elements can be achieved with a higher precision.

**(FOURTH EMBODIMENT)**

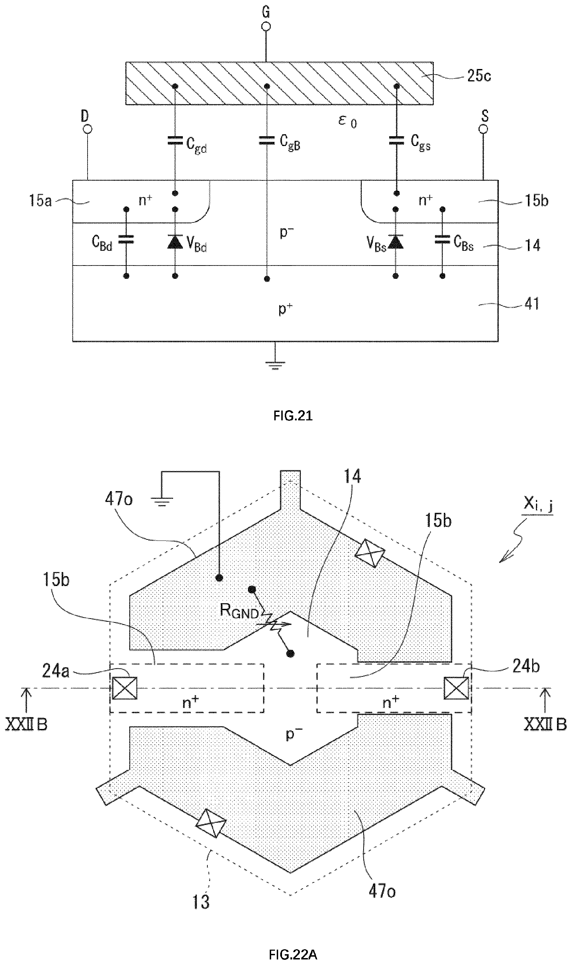

In the above-mentioned first to third embodiments, the cases are explained in which the potentials of the floating electrodes 17c and 17q are changed ascribable to the variations of the capacitance values of the first variable capacitor $C_1$, caused by applications of the ultrasonic waves, and the heights of the potential barriers generated in the channel of the IG semiconductor element is controlled indirectly through the gate-insulating films just under the floating electrodes 17c and 17q, and the current outputs are accordingly obtained. As illustrated in FIG. 21, an AI semiconductor element pertaining to a fourth embodiment of the present invention lacks the floating electrodes 17c and 17q, and further, lacks the gate-insulating film just under the floating electrodes 17c and 17q, which are used in the first to third embodiments. The vacuum implementing the vibration-cavity 28 is an insulator (dielectric) with a dielectric constant $\varepsilon_o$. Hence, the vacuum implementing the vibration-cavity 28 can realize the function as the gate-insulating film equivalent to a $SiO_2$ film of a dielectric constant $\varepsilon_o \varepsilon_r$.

Although a partial structure is illustrated in FIG. 21, the AI semiconductor element pertaining to the fourth embodiment encompasses a p-type semiconductor substrate 41, a channel-generating region 14 made of p-type semiconductor region laminated on the semiconductor substrate 41, an n-type first main-electrode region 15b and an n-type second main-electrode region 15a, which are buried in a mutually facing manner, and separated from each other in the surface of the channel-generating region 14, and a vibration electrode 25c, which is set to the second potential and facing through a vibration-cavity to the top surface of the channel-generating region 14. In an upper region at top surface side of the p-type channel-generating region 14, the n-type first main-electrode region 15b and the n-type second main-electrode region 15a are arranged separately from each other, to implement an n-p-n hook structure. A potential barrier to electrons is generated between a couple of depletion layers, namely between a depletion layer spreading from a p-n junction interface of the channel-generating region 14 and the first main-electrode region 15b and another depletion layer spreading from another p-n junction interface of the channel-generating region 14 and the second main-electrode region 15a.

Since the semiconductor substrate 41 is set to the first potential (ground potential), the channel-generating region 14 is also set to the first potential. Additionally, although FIG. 21 omits the illustration of the cavity-surrounding insulating-film 20, which surrounds the vibration-cavity to construct the vibration-cavity as the hermetically confined space between the channel-generating region 14 and the vibration electrode 25c as illustrated in FIG. 3, the cavity-surrounding insulating-film shall be included similarly to the configuration illustrated in FIG. 3, as a matter of course. That is, by constructing the hermetically confined space with the cavity-surrounding insulating-film whose illustration is omitted, the vibration-cavity of the AI semiconductor element pertaining to the fourth embodiment is kept in vacuum or reduced pressure state in which inert gas is filled. In preparation to an incident of gas discharges, the vibration-cavity may be filled with sulfur hexafluoride ($SF_6$) gas. However, when an inter-electrode distance d between the vibration electrode 25c and the semiconductor substrate 41 is small, the gas discharges can be suppressed. In the AI semiconductor element pertaining to the fourth embodiment illustrated in FIG. 21, a gate-substrate capacitor $C_{gB}$ between the vibration electrode 25c and the semiconductor substrate 41 shall serve as the first variable capacitor $C_1$, defined between the vibration electrode 25c and the floating electrodes 17c and 17p as explained in the first to third embodiments.

The AI semiconductor element pertaining to the fourth embodiment does not use the changes $\Delta V_3$ in the voltage $V_3$, which is determined by the capacitive voltage division as represented by Eq. (4) and Eq. (8), and therefore, the DC bias-voltage $V_{bias}$ between the vibration electrode 25c and the semiconductor substrate 41 is constant. In the AI semiconductor element pertaining to the fourth embodiment, capacitance-variations $\Delta C_{gB}$ in the gate-substrate capacitor $C_{gB}$, when an inter-electrode distance d between the vibration electrode 25c and the semiconductor substrate 41 changes by infinitesimal displacement $\Delta d$, are used to generate charge-variations $\Delta q$ induced between the first main-electrode region 15b and the second main-electrode region 15a. The charge-variations $\Delta q$ can be expressed by the following Eq. (46):

$$\Delta q = \Delta C_{gB} V_{bias} \qquad \text{--- --- (46)}.$$

An area of the vibration electrode 25c is assumed as S, and out of the area S, an effective area that can contribute to the gate-substrate capacitor $C_{gB}$ is set as $\alpha S$. That is, when approximation is conducted such as $C_{gB} \fallingdotseq C_1 \fallingdotseq \varepsilon_0(\alpha S/d)$, the capacitance-variations $\Delta C_{gB}$ in the gate-substrate capacitor $C_{gB}$ caused by infinitesimal displacement $\Delta d$ of the inter-electrode distance d is expressed by the following Eq. (47).

[Equation 17]

$$\frac{\partial C_{gB}}{\partial d} = - \frac{\varepsilon_0 \alpha S}{d^2} \qquad \text{········ (47)}$$

Thus, Eq. (46) can be expressed by the following Eq. (48):

$$\Delta q = \varepsilon_o(\alpha S/d^2)\Delta d V_{bias} \qquad \text{--- --- (48)}.$$

The potential barrier height for electrons generated in the n-p-n hook structure between the first main-electrode region 15b and the second main-electrode region 15a is controlled by the charge-variation $\Delta q$ represented by Eq. (48).

When ultrasonic waves are supplied to the vibration electrode 25c, the position of the vibration electrode 25c is displaced by the pressures of the ultrasonic waves. When the position of the vibration electrode 25c changes, the gate-substrate capacitor $C_{gB}$ between the vibration electrode 25c and the semiconductor substrate 41 changes, which changes the surface potential in a channel generated between the first main-electrode region 15b and the second main-electrode region 15a. That is, the high of the potential barrier for electrons generated in the channel between the first main-electrode region 15b and the second main-electrode region 15 alters by variations of the gate-substrate capacitor $C_{gB}$, and the changes lead to variations of current. Thus, according to the AI semiconductor element pertaining to the fourth embodiment, an IG semiconductor element having a vacuum gate-insulating film (hereafter, referred to as "a vacuum IG semiconductor element") is defined, and displacements caused by the ultrasonic waves at the vibration electrode 25c can be detected as the variations of current flowing between the first main-electrode region 15b and the second main-electrode region 15a.

The AI semiconductor element pertaining to the fourth embodiment is a vacuum insulated-gate transistor that uses the vacuum of a dielectric constant $\varepsilon_o$ as a gate-insulating film as illustrated in FIG. 21, compared with the usual MIS transistor that uses a gate-insulating film of usual dielectric constant $\varepsilon_o \varepsilon_r$. Therefore, comparing with the mutual conductance $g_m$ of the usual MOSFET represented by Eq. (30), a capacitor $C_{ox}$ per unit area in the vacuum IG semiconductor element becomes $(1/\varepsilon_r)C_{ox}$. Then, by using the specific dielectric constant $\varepsilon_r$ of the gate-insulating film for the usual MOSFET, the mutual conductance of the vacuum IG semiconductor element is expressed by the following Eq. (49):

$$g_m = \mu_n C_{ox} W / \varepsilon_r L_{eff} \qquad \text{--- --- } (49).$$

Thus, the mutual conductance of the vacuum IG semiconductor element can be expressed as small by $(1/\varepsilon_r)$ compared with the mutual conductance of the usual MOSFET.

**« FIRST VARIATION OF FOURTH EMBODIMENT »**

According to the results of the simulations illustrated in FIG. 19A (a) and FIG. 19A (b), the voltage-detection sensitivities of the divided-gate structure in which the floating electrode is concentrically divided and the floating electrode is arranged in the donut-shaped fixed-potential electrode 17o are higher than the voltage-detection sensitivities of the fixed-potential electrode of the uniform flat plate. That is, as illustrated in FIG. 6A, FIG. 28 etc., the capacitive acoustic-element has the problem that the central portion of the vibration electrode is deeply deflected. In view of the results of the simulations represented by FIG. 19A (a) and FIG. 19A (b), the AI semiconductor element pertaining to the fourth embodiment is preferred to further include an auxiliary-electrode 47o of a fixed potential (ground potential) made of conductive layer such as metal, etc., as illustrated in FIG. 22A. Or, a structure in which heavily impurity-doped semiconductor regions equivalent to the auxiliary-electrode 47o shall be buried in peripheries of the main gate region, having a planar pattern such as the topology illustrated in FIG. 22A, is preferred. When the auxiliary-electrode 47o is delineated by the impurity-doped layer, the auxiliary-electrode 47o may be extended, by buying the impurity-doped layer as a region having identical deepness and identical impurity concentration with the second main-electrode region 15a. Or, a pattern of the auxiliary-electrode 47o may be made of metal-silicide layer, etc.

FIG. 22A illustrates an example of a planar pattern of a hexagonal unit cells $X_{i,j}$ constructing a AEIC of the first variation in the fourth embodiment. In a vacuum IG semiconductor element constructing each of the unit cells $X_{i,j}$ of the AEIC of the first variation in the fourth embodiment, a first main-electrode region 15b and a second main-electrode region 15a, which are represented by rectangles of concealed lines in FIG. 22A, are delineated separately from each other while their short latera are facing to each other. Moreover, two auxiliary-electrodes 47o which are separately disposed at the upper and lower sides of the paper, respectively, are arranged with mirror-symmetry. In the mirror-symmetry, an upper hexagonal annular half-arc extends clockwise along the upper side toward an upper long latus of the second main-electrode region 15a, from an upper periphery of upper long latus of the first main-electrode region 15b. And, a lower hexagonal annular half-arc extends counter clockwise along the lower side toward a lower long latus of the second main-electrode region 15a, from a lower periphery of lower long latus of the first main-electrode region 15b.

FIG. 22A exemplifies a pattern of top surface of a functional area encompassing two approximately concentrically divided hexagonal half-arced auxiliary-electrodes 47o and an effective gate region sandwiched in between the auxiliary-electrodes 47o. However, a topology in which the pattern of the top surface of the functional area is separated into the two auxiliary-electrodes 47o and the effective gate region is not limited to the double half-arced hexagonal-annular topology exemplified in FIG. 22A. However, as explained by using FIG. 19A, etc., the topology of the top surface of the functional area in which the effective gate region and the auxiliary-electrodes 47o are concentrically arranged is preferred for the sensitivity characteristics.

As illustrated in FIG. 22B, in each of the hexagonal unit cells $X_{i,j}$ implementing the AEIC of the first variation in the fourth embodiment, the top surface of the common base-body 11 is separated into the channel-generating regions 14, which are isolated from each other, by an element-isolation insulating-film 13, and the unit cells $X_{i,j}$ are two-dimensionally arrayed with the channel-generating regions 14as a basis for structure. Moreover, on each of the channel-generating regions 14, the vibration-cavities 28 are incorporated as a monolithic structure. Thus, regarding the monolithic structure, an enclosure structure similar to the earlier capacitive acoustic-element is defined. As illustrated in the cross-sectional view in FIG. 22B, in the surface of the channel-generating regions 14 of first conductivity type, the first main-electrode region 15b and second main-electrode region 15a of second conductivity type are buried, thereby constructing the vacuum IG semiconductor elements.

A first contact plug 24b is arranged in the right end of the strip-shaped planar pattern sandwiched in between the two auxiliary-electrodes 47o which are separated into the upper and lower sides of the paper in FIG. 22A, respectively, and a second contact plug 24a is arranged in the left end of the strip-shaped planar pattern. Moreover, a third contact plug 24b is arranged on the lower-left oblique latus of the auxiliary-electrodes 47o illustrated on the lower side of the paper, and a fourth contact plug 24a is arranged on the upper-right oblique latus of the auxiliary-electrodes 47o illustrated on the upper side of the paper. As illustrated in FIG. 22A, the first contact plug 24b is provided such that the first main-electrode region 15b constructing each of the vacuum IG semiconductor elements of the unit cells $X_{i,j}$ is connected to the first potential (ground potential). The second contact plug 24a is provided to supply a power-supply voltage $V_{DD}$ from a power-supply interconnection $V_{DD}$ to the second main-electrode region 15a constructing the vacuum IG semiconductor elements of the unit cells $X_{i,j}$ and furthermore, the second contact plug 24a is provided to extract current signals from each of the unit cells $X_{i,j}$.

The AI semiconductor element pertaining to the first variation of the fourth embodiment can be made to serve not only as a unidirectional acoustic-element dedicated to a receiving function but also a bidirectional acoustic-element. When the AI semiconductor element of the first variation of the fourth embodiment illustrated in FIG. 22A is subjected to serve as the bidirectional acoustic-element, the auxiliary-electrodes 47o effectively acts in a transmitting mode. That is, the third contact plug arranged in the lower auxiliary-electrodes 47oon the paper in FIG. 22A supplies high-frequency signals between the vibration electrode 25c and the upper auxiliary-electrodes 47o of the paper, and further, between the vibration electrode 25c and the lower auxiliary-electrodes 47o on the paper, respectively, from a high-frequency signals-line in the transmitting mode. The fourth contact plug arranged on the upper auxiliary-electrodes 47o on the paper in FIG. 22A supplies a DC bias $V_{bias}$ at the second potential to the vibration electrode 25c from a DC bias-supply line $V_{bias}$ and applies a desirable potential between the vibration electrode 25c and the channel-generating region 14 in the receiving mode.

FIG. 22B illustrates a case in which the channel-generating region 14 is connected to the first potential (= ground potential GND). For operating as the vacuum IG semiconductor elements, top surfaces of the first main-electrode region 15b and the second main-electrode region 15a and atop surface of the channel-generating region 14 sandwiched between the first main-electrode region 15b and the second main-electrode region 15a are exposed to a vacuum state provided by the vibration-cavity 28. In the AI semiconductor element of the first variation of the fourth embodiment, the displacement of the vibration electrode 25c by ultrasonic waves $\Phi$ can be detected as the variations of current flowing between the first main-electrode region 15b and second main-electrode region 15a in the vacuum IG semiconductor elements. In FIG. 22B, a bidirectional white-arrow overlaid on the vibration electrode 25c mean the displacements of the vibration electrode 25c.

As illustrated in FIG. 22B, on the top surface of a vibration-membrane 23, a first surface-wiring layer 25b and a second surface-wiring layer 25 are disposed in addition to the vibration electrode 25c. The first contact plug 24b penetrating through the vibration-membrane 23 and the cavity-surrounding insulating-film 20 is provided in a location between the first surface-wiring layer 25b and the first main-electrode region 15b. Similarly, the second contact plug 24a penetrating through the vibration-membrane 23 and the cavity-surrounding insulating-film 20 is provided in a location between the second surface-wiring layer 25a and the second main-electrode region 15a. That is, the top end of the first contact plug 24b is metallurgically connected to the first surface-wiring layer 25b, and the bottom end of the first contact plug 24b is electrically connected to the first main-electrode region 15b. Thus, the first surface-wiring layer 25b and the first main-electrode region 15b are electrically connected by the first contact plug 24b.

Similarly, the top end of the second contact plug 24a is metallurgically connected to the second surface-wiring layer 25a, and the bottom end of the second contact plug 24a is electrically connected to the second main-electrode region 15b. Thus, the second surface-wiring layer 25a and the second main-electrode region 15a are electrically connected by the second contact plug 24a. The first surface-wiring layer 25b corresponds to the vertical output-signal line. On the other hand, the second surface-wiring layer 25a corresponds to the power-supply interconnection. On the vibration-membrane 23, a vibration-electrode protection-film 26 made of silicon oxide film is laminated to cover the vibration electrode 25c, the first surface-wiring layer 25b and the second surface-wiring layer 25. Moreover, a second vibration-electrode protection-film 34 made of polyimide film is stacked on the vibration-electrode protection-film 26. The second vibration-electrode protection-film 34 closes a liquid-introduction canal 27 through which a removal liquid is introduced to remove a sacrificial layer, at a process step of forming the vibration-cavity 28. The liquid-introduction canal 27 penetrates the first vibration-electrode protection-film 26 and the vibration-membrane 23 and arrives at the vibration-cavity 28. Since the liquid-introduction canal 27 is closed, the inside of the vibration-cavity 28 is kept in an atmosphere of inert gas at reduced pressure.

In FIG. 22B, a capacitance generated between the effective gate region and the vibration electrode 25c near the centre of the vibration-cavity 28 is illustrated as the first variable capacitor $C_1$. Around the first variable capacitor $C_1$, regarding the first variable capacitor $C_1$, as illustrated in FIG. 22A, there is a second variable capacitor $C_2$ which are allocated between the vibration electrode 25c and each of the two hexagonal half-arced auxiliary-electrodes 47o. When patterns of the double auxiliary-electrodes 47o illustrated in FIG. 22A are defined by conductors such as metal, the horizontal levels of the double auxiliary-electrodes 47o become higher than the top surface of the effective gate region establishing the first variable capacitor $C_1$ by the thicknesses of the conductive layers and the thickness of the insulating layers inserted under the conductive layers. Therefore, a structure, in which the auxiliary-electrodes 47o are buried with the impurity-doped layers, is easy to homologize the horizontal levels of the top surfaces of the auxiliary-electrodes 47o and the top surface of the effective gate region with each other.

In a cutoff state of the vacuum IG semiconductor element illustrated in FIG. 22B, a portion between the effective gate region and the ground potential can be represented by an equivalent circuit as a topology in which a variable delay resistor $R_{GND}$ is connected across the portion, substantially identical to the conditions of the simulations in FIG. 19A (a) and FIG. 19A (b). The second variable capacitor $C_2$ is a capacitance having a role equivalent to a capacitance defined between the upper and lower electrodes in the earlier capacitive acoustic-elements, and the first variable capacitor $C_1$ is a capacitor that induces the charges required to drive the vacuum IG semiconductor element unique to the AI semiconductor element of the first variation of the fourth embodiment. When the vacuum IG semiconductor element is turned on into the conductive state by the changes of the capacitance in the first variable capacitor $C_1$, the delay resistor $R_{GND}$ connected between the effective gate region and the ground potential is sharply changed to a small value so that a current can be flowed.

### « SECOND VARIATION OF FOURTH EMBODIMENT »

As FIG. 23 illustrates the configuration of a main portion, an AI semiconductor element of a second variation of the fourth embodiment encompasses a p-type semiconductor substrate 41, a channel-generating region 14 made of $p^-$-type (or i-type) semiconductor region laminated on the semiconductor substrate 41, an n-type first main-electrode region 15b and an n-type second main-electrode region 15a which are buried in a mutually facing manner, and separated from each other in the surface of the channel-generating region 14, and a vibration electrode 25c, which is set to the second potential that is facing via the vibration-cavity to the top surface of the channel-generating region 14. Although the configuration, in which in an upper surface side of the (p-type or i-type) channel-generating region 14, the n-type first main-electrode region 15b and the n-type second main-electrode region 15a are buried separately from each other, is like the configuration illustrated in FIG. 21, an n-p⁻(i)-n hook structure is implemented by the configuration illustrated in FIG. 23. A depletion layer from the p⁻(i)-n junction interface between the channel-generating region 14 and the first main-electrode region 15b will more easily spread than the configuration illustrated in FIG. 21.

Similarly, a depletion layer from the p⁻(i)-n junction interface between the channel-generating region 14 and the second main-electrode region 15a will more easily spread than the configuration illustrated in FIG. 21. Thus, when the depletion layer spreading from the p⁻(i)-n junction interface between the channel-generating region 14 and the first main-electrode region 15b and the depletion layer spreading from the p⁻(i)-n junction interface between the channel-generating region 14 and the second main-electrode region 15a are pinched off, a perfectly depleted portion is generated between the first main-electrode region 15b and the second main-electrode region 15a, and therefore, a potential barrier for electrons in the hook structure is established between the first main-electrode region 15b and the second main-electrode region 15a. A $p^{++}$-type potential-barrier control-region 42 is buried in the interface between the channel-generating region 14 and the semiconductor substrate 41, just under the position in which the potential barrier is scheduled to be generated. Since the $p^{++}$-type potential-barrier control-region 42 is buried, the potential barrier between the first main-electrode region 15b and the second main-electrode region 15a is set higher than that of the configuration illustrated in FIG. 21.

Since the semiconductor substrate 41 is set to the first potential (ground potential), the channel-generating region 14 will also set to the first potential. Additionally, regarding the cavity-surrounding insulating-film 20 surrounding the vibration-cavity to construct the vibration-cavity as the hermetically confined space between the channel-generating region 14 and the vibration electrode 25c as illustrated in FIG. 3 and FIG. 22, etc., it is legitimate to encompass the cavity-surrounding insulating-film as illustrated in FIG. 22B, although the illustration is omitted in FIG. 23. With the cavity-surrounding insulating-film, the vibration-cavity is kept in vacuum or reduced pressure state. In the AI semiconductor element of the second variation of the fourth embodiment illustrated in FIG. 23, the gate-substrate capacitor $C_{gB}$ between the vibration electrode 25c and the potential-barrier control-region 42 corresponds to the first variable capacitor $C_1$ between the vibration electrode 25c and the floating electrodes 17c and 17p, as explained in the first to third embodiments. Since a portion between the first main-electrode region 15b and the second main-electrode region 15a is perfectly depleted, the upper region of the channel-generating region 14 serves as the insulator (dielectric). Thus, the gate-substrate capacitor $C_{gB}$ between the vibration electrode 25c and the potential-barrier control-region 42 makes it easier for charges to be induced in the upper region of the perfectly-depleted channel-generating region 14.

When ultrasonic waves are supplied to the vibration electrode 25c, the position of the vibration electrode 25c is displaced by the pressures of the ultrasonic waves. When the position of the vibration electrode 25c is displaced, the gate-substrate capacitor $C_{gB}$ between the vibration electrode 25c and the semiconductor substrate 41 changes. Thus, the surface potential of the perfectly-depleted channel between the first main-electrode region 15b and the second main-electrode region 15changes similarly to the operational principle of MOS SIT. That is, the potential barrier height for electrons generated in the perfectly-depleted channel between the first main-electrode region 15b and the second main-electrode region 15a alters by variations of the gate-substrate capacitor $C_{gB}$, and the alterations of the potential barrier height results in the variations of current. Hence, according to the AI semiconductor element of the second variation of the fourth embodiment, the displacement owing to the ultrasonic waves of the vibration electrode 25c can be detected as the variations of current flowing between the first main-electrode region 15b and the second main-electrode region 15a.

## <<THIRD VARIATION OF FOURTH EMBODIMENT »

As FIG. 24 illustrates a configuration of a main portion, an AI semiconductor element of a third variation of the fourth embodiment encompasses a p-type semiconductor substrate 41, a channel-generating region 14made of $n^-$ -type (or i-type) semiconductor region laminated on the semiconductor substrate 41, a p-type first main-electrode region 15b and an n-type second main-electrode region 15a which are buried in a mutually facing manner, and separated from each other in the surface of the channel-generating region 14, and a vibration electrode 25c, which is set to the second potential and facing to the top surface of the channel-generating region 14via the vibration-cavity. The p-type first main-electrode region 15b is buried in an n-type buffer region 43. Due to the configuration in which the p-type first main-electrode region 15b, the n-type buffer region 43 and the n-type second main-electrode region 15a are arranged in an upper region at top surface side of the $n^-$ -type (or i-type) channel-generating region 14, a hook structure similar to an SI thyristor of n-$n^-$ (i)-n-p is defined. The configuration of the n-type buffer region 43 makes it difficult for the depletion layer to spread from the side of the first main-electrode region 15b.

On the other hand, the $n^-$(i)-n junction interface between the channel-generating region 14 and the second main-electrode region 15a is also depleted. Since a portion between the first main-electrode region 15b and the n-type buffer region 43 has an impurity concentration close to an intrinsic semiconductor (i-layer), the i-layer portion is originally close to the situation of the depletion layer, and therefore, the potential barrier for electrons in the hook structure is generated between the first main-electrode region 15b and the second main-electrode region 15a. In the interface between the semiconductor substrate 41 and the channel-generating region 14 just under the position in which the potential barrier is generated, a $p^{++}$- type potential-barrier control-region42 is buried similarly to FIG. 21. Since the $p^{++}$ -type potential-barrier control-region42 is buried, a depletion layer is provided in a location between the potential-barrier control-region 42 and the channel-generating region 14, and the potential barrier between the first main-electrode region 15b and the n-type buffer region 43 can be set higher than that of the configuration illustrated in FIG. 21.

Since the semiconductor substrate 41 is set to the first potential (ground potential), the channel-generating region 14 is also set to the first potential. Additionally, although illustration is omitted, the technical feature that the vibration-cavity to which the surface of the channel-generating region 14 is exposed is surrounded by the cavity-surrounding insulating-film similarly to FIG. 3 and FIG. 22B, etc., is like the subject matter explained related to FIG. 23, etc. The vibration-cavity is kept in the vacuum or reduced pressure state by the cavity-surrounding insulating-film. In the AI semiconductor element of the third variation of the fourth embodiment illustrated in FIG. 24, the gate-substrate capacitor $C_{gB}$ between the vibration electrode 25c and the potential-barrier control-region 42 can be made to correspond to the first variable capacitor $C_1$ between the vibration electrode 25c and the floating electrodes 17c and 17p, as explained in the first to third embodiments. Since a portion between the first main-electrode region 15b and the second main-electrode region 15a is perfectly depleted, the upper region of the channel-generating region 14 serves as the insulator (dielectric). Thus, by the gate-substrate capacitor $C_{gB}$ between the vibration electrode 25c and the potential-barrier control-region 42, the charges are easily induced in the perfectly-depleted channel-generating region 14.

When ultrasonic waves are supplied to the vibration electrode 25c, the position of the vibration electrode 25c is displaced by the pressures of the ultrasonic waves. When the position of the vibration electrode 25c is displaced, the gate-substrate capacitor $C_{gB}$ between the vibration electrode 25c and the semiconductor substrate 41 changes, thereby changing the surface potential of the perfectly-depleted channel between the first main-electrode region 15b and the second main-electrode region 15a. That is, the potential-barrier height for electrons generated in the perfectly-depleted channel between the first main-electrode region 15b and the second main-electrode region 15 alters by variations of the gate-substrate capacitor $C_{gB}$, and turn-on transition is activated similarly to an insulated-gate control SI thyristor. Accordingly, a larger current flow between the first main-electrode region (cathode region) 15b and the second main-electrode region (anode region) 15a.

Due to thyristor operation, by turn-on, electrons are injected from the first main-electrode region 15b into the channel-generating region 14, and holes are injected from the second main-electrode region 15a into the channel-generating region 14. When the second main-electrode region 15a is grounded, the potential-barrier control-region 42 and the second main-electrode region 15a is shorted, and the injected holes are pulled out by the potential-barrier control-region 42. According to the AI semiconductor element of the third variation of the fourth embodiment illustrated in FIG. 24, the displacements of the vibration electrode 25c owing to the ultrasonic waves can be detected as the variations of current flowing between the first main-electrode region 15b and the second main-electrode region 15a.

**« FOURTH VARIATION OF FOURTH EMBODIMENT »**

As FIG. 25 illustrates a configuration of a main portion, an AI semiconductor element of a fourth variation of the fourth embodiment encompasses a semiconductor substrate implementing an n-type first main-electrode region 15b, a channel-generating region 14made of p-type semiconductor region laminated on the first main-electrode region 15b, an n-type first main-electrode region 15a laminated on the channel-generating region 14, a recessed portion having a vertical inner-sidewall which penetrates through the first main-electrode region 15a and is dug up to a part of the channel-generating region 14, a fixed-potential electrode 17olaminated on the bottom surface of the recessed portion, and a vibration electrode 25v moving parallel to the inner-sidewall of the recessed portion in the inside of the recessed portion as vibration-cavity. Since the first main-electrode region 15b is set to the first potential (ground potential), the fixed-potential electrode 17o is also set to the first potential. Thus, a second variable capacitor $C_2$is generated between the fixed-potential electrode 17o of the first potential and the bottom surface of the vibration electrode 25v at the second potential.

As illustrated in FIG. 25, the driving portion of the vibration electrode 25v is square pillar having a hollow rectangular cross-section. A top portion of the square pillar shape with the hollow rectangle is plate-shaped, the plate having a predetermined spring constant, similarly to the vibration electrode 25c of the AI semiconductor elements of the first to third embodiments. The inner side walls of the recessed portion and the outer sidewalls of the square-pillar vibration-electrode 25v having the hollow rectangular cross-section are facing to each other via the vibration-cavity, and the square-pillar vibration-electrode 25v vibrates upwardly and downward along vertical direction with the predetermined spring constant, by the pressures of the ultrasonic waves irradiated, wherein the vibration electrode 25v is set to the second potential. Additionally, although illustration is omitted, the configuration such that the vibration-cavity, to which the surface of the channel-generating region 14 is exposed, is surrounded by the cavity-surrounding insulating-film, similarly to FIG. 3 and FIG. 22B, etc., is like the situations explained by referring to FIG. 24, etc. The vibration-cavity is kept in the vacuum or reduced pressure state by the cavity-surrounding insulating-film.

Maintaining intervals between the channel-generating regions 14 exposed at the inner planes of the recessed portion and the outer sidewalls of the vibration electrode 25v at a constant value, the vibration electrode 25v moves in the inside of the recessed portion upwardly and downward, together with the ultrasonic-wave vibrations. For materials of the square pillar portion of the hollow rectangle that moves upwardly and downward in the recessed portion., while maintaining the interval between the inner plane of the recessed portion and the outer sidewall of the vibration electrode 25v at the constant value, metals having strong rigidity such as W is preferred The gate-channel capacitors $C_{GC}$ corresponding to the first variable capacitors $C_1$ explained in the first to third embodiments are defined between the channel-generating regions 14 exposed at the inner planes of the recessed portion and the outer sidewalls of the vibration electrode 25v. As the interval between the inner plane and the outer sidewall is constant, because the gate-channel capacitor $C_{GC}$ will not alter with the inter electrode interval, but with overlapping area implemented by portions of the facing planes, the overlapping area can contribute as the effective capacitance, the gate-channel capacitor $C_{GC}$ alters by the vertical movement of the vibration electrode 25v.Then, the first variable capacitor $C_1$changes by variations of the overlapping area of the facing planes. As illustrated in FIG. 25, the p-type channel-generating region 14 is arranged on the n-type first main-electrode region 15b, and the n-type first main-electrode region 15b is arranged on the p-type channel-generating region 14. Consequently, n-p-n hook structures are built along the vertical direction of the inner planes of the recessed portion. And, the potential barriers for electrons are defined by the n-p-n hook structures.

The potential barriers for electrons between the first main-electrode region 15b and the second main-electrode region 15aare generated by the depletion layers spreading from the p-n junction interfaces between the channel-generating regions 14 and the second main-electrode regions 15a and the depletion layers spreading from the p-n junction interfaces between the channel-generating regions 14 and the second main-electrode regions 15a. Since portions between the first main-electrode regions 15b and the second main-electrode regions 15a are perfectly depleted, the vicinity of the surface of the channel-generating regions 14 exposed at the inner planes of the recessed portion serve as the insulator (dielectric). Thus, the charges are easily induced in the vicinity of the surfaces of the perfectly-depleted channel-generating regions 14 by the gate-channel capacitors $C_{GC}$ between the outer sidewalls of the vibration electrode 25v and the channel-generating regions 14 whose inner planes are exposed to the vibration-cavity.

When the ultrasonic waves are supplied to the vibration electrode 25v, by the pressures of the ultrasonic waves, the position of the vibration electrode 25v is displaced in the recessed portion constructing the vibration-cavity. When the position of the vibration electrode 25v is displaced, the gate-channel capacitors $C_{GC}$ between the vibration electrode 25v and the channel-generating regions 14 change owing to the changes of the overlapping area of the facing planes. Thus, the surface potentials of the perfectly-depleted channels between the first main-electrode regions 15b and the second main-electrode regions 15a change. That is, the potential-barrier heights for electrons generated in the perfectly-depleted channels between the first main-electrode regions 15b and the second main-electrode regions 15 alters by variations of the gate-channel capacitors $C_{GC}$. Thus, the current caused by electrons injected beyond the potential barriers changes. Hence, according to the AI semiconductor element of the fourth variation of the fourth embodiment, the displacement of the vibration electrode 25v owing to the ultrasonic waves can be detected as the variations of current flowing between the first main-electrode regions 15b and the second main-electrode regions 15a.

## (OTHER EMBODIMENTS

As mentioned above, the present invention has been described by using the first to fourth embodiments. However, the discussions and drawings that constitute a part of the disclosure should not be construed to limit the present invention. Various alternative embodiments, the various modifications, and the various application techniques may be apparent to the persons skilled in the art from the disclosure. For example, in the already-described explanations of the first to fourth embodiments, the AI semiconductor elements for receiving and the receiving cells using the AI semiconductor elements have been mainly explained. However, the improvement-scheme, in which the deformed profile of the vibration electrode becomes close to the topology of the flat parallel-plate as explained by using FIG. 6A, can improve the characteristics of the discrete capacitive acoustic-element as the transmitting cell. That is, because the problem of the deformed profile, in which only the centre of the vibration electrode approaches the fixed-potential electrode when the higher voltage is applied, is solved, stronger ultrasonic waves can be transmitted from the capacitive acoustic-element designed for transmission.

From the above subject matter, the present invention can be applied even to a technique in which a part of the technical ideas explained in the above-mentioned first to fourth embodiments is combined as appropriate. In the already-described explanations in the first to third embodiments, the case in which the first potential connected to the fixed-potential electrode 17o is assumed as the ground potential and the second potential connected to the vibration electrode is the second potential higher than the ground potential is exemplified. However, the assignments to the first potential and the second potential are not limited to the explanations in the first to third embodiments.

For example, as illustrated in FIG. 27, the second potential connected to a vibration electrode 25d maybe set as the ground potential, and the first potential connected to the fixed-potential electrode 17o and the channel-generating region 14 may be set as the DC bias-voltage $V_{bias}$ of higher potential. In a structure illustrated in FIG. 27, a second floating electrode 35 is provided through a second floating-electrode protection-film 36 to the lower portion of the vibration electrode 25d, as explained in the first to third embodiments. And, a stiffness-toughened lid portion 37c made of $Si_3N_4$ film is arranged on the vibration electrode 25d through a vibration-electrode protection-film 26d.

Even in the structure in which the side of the vibration electrode 25d is assigned to the ground potential as illustrated in FIG. 27, at a process step of forming a vibration-cavity 28, it is necessary to introduce a removing solution (etching solution) to selectively solve a sacrificial layer by wet etching. Therefore, a vacuum plug 37d made of $Si_3N_4$ film seals a solution introduction canal 34 that is opened to introduce the removing solution. The solution introduction canal 34 penetrates through the vibration-electrode protection-film26d, the second floating-electrode protection-film 36 and the vibration-membrane 23 and arrives at the vibration-cavity 28. A vacuum-plug upper-pattern 38 made of silicon oxide film, used as the etching mask for delineating the stiffness-toughened lid portion 37c, hangs over from the top surface of the stiffness-toughened lid portion 37c, remaining as cornice state. For example, the second floating electrode 35, the vibration electrode 25d and the lower electrode (17c, 17o) will be set to the same area. Here, the area of the lower electrode (17c, 17o) is the sum of the area of the floating electrode 17c and the fixed-potential electrode 17o.

It is assumed that the second floating-electrode protection-film 36 is made of $SiO_2$ film and a thickness of the second floating-electrode protection-film 36 between the second floating electrode 35 and the vibration electrode 25d is 10 nm. Moreover, when a portion between the second floating electrode 35 and the lower electrode (17c, 17o) is assumed to vacuum whose height is 10 nm, four-fifths of the DC bias-voltage $V_{bias}$ applied between the vibration electrode 25d and the fixed-potential electrode 17o is applied across a third variable capacitor $C_5$ defined between the second floating electrode 35 and the second floating electrode 35. On the other hand, one-fifth of the DC bias-voltage $V_{bias}$ is applied between the second floating electrode 35 and the floating electrode 17c. The reason for the voltage division of one-fifth is because, for example, when an area of the floating electrode 17c is assumed to be small and ignored, and the areas of the vibration electrode 25d and the fixed-potential electrode 17o are assumed to be approximately equal, a specific dielectric constant of the $SiO_2$ film is $\varepsilon_r = 4$ and the following Eq. (50) is established:

$$C_5 = 4C_6 \qquad\qquad \text{--- --- (50)}.$$

That is, in a case of a configuration in which the vibration electrode 25d is set at ground potential as illustrated in FIG. 27, four-fifths of the DC bias-voltage $V_{bias}$ is applied across the third variable capacitor $C_5$, and one-fifth of the DC bias-voltage $V_{bias}$ is applied across the first variable capacitors $C_4$.

Thus, it should be noted that the present invention is not limited to the description of the first to fourth embodiments described above, and various modifications can be made, which are also included within the scope of the invention. Therefore, the scope of the present invention is defined only by the technical features specifying the present invention pertaining to the description of the claims, which are reasonable from the above description.

[Reference Signs List]

12---gate-insulating film (first gate-insulating film); 14---channel-generating region; 15a-second main-electrode region; 15b---first main-electrode region; 16---gate-insulating film (second gate-insulating film); 17c 17q---main floating-electrode; 17o, 17p, 17r---fixed-potential electrode; 23---vibration membrane; 25c---vibration electrode

**Claims**

1. An acoustic induction-type semiconductor element comprising:

    a channel-generating region made of semiconductor region of first conductivity type, being set to a first potential; first and second main-electrode regions of second conductivity type which are disposed in a mutually facing manner, and separated from each other in the channel-generating region; a vibration electrode set to a second potential, facing through a vibration-cavity to a top surface of the channel-generating region; and a cavity-surrounding insulating-film surrounding the vibration-cavity so that the vibration-cavity is provided in a location between the channel-generating region and the vibration electrode, configured to implement a hermetically confined space with the vibration-cavity, wherein displacements of the vibration electrode by ultrasonic waves are detected as changes of current flowing between the first and second main-electrode regions.

2. The semiconductor element of claim 1, further comprising:

    a gate-insulating film laminated on the first and second main-electrode region and on the channel-generating region sandwiched between the first and second main-electrode regions: and a floating electrode made of a conductive layer set to be at least pseudo-floating state, disposed on the gate-insulating film, at a place above the channel-generating region sandwiched in between the first and second main-electrode regions.

3. The semiconductor element of claim 2 further comprising a fixed-potential electrode made of conductive layer set to the first potential, which is arranged adjacently to the floating electrode on the gate-insulating film, being separated from the floating electrode.

4. The semiconductor element of claim 2 or 3, further comprising a vibration-membrane made of an insulating film facing via the vibration-cavity to the floating electrode.

5. The semiconductor element as in any one of claims 2 to 4, further comprising a delay resistor, configured to connect the first potential to the floating electrode, wherein the delay resistor defines a high pass filter with a capacitance between the floating electrode and the vibration electrode.

6. An acoustic element integrated circuit comprising a plurality of unit cells which are arrayed on a common substrate, wherein each of the unit cells contains as at least a part of the unit cells an acoustic induction-type semiconductor element including:

a channel-generating region made of semiconductor region of first conductivity type, being set to a first potential;
first and second main-electrode region of second conductivity type which are disposed in a mutually facing manner, and separated from each other in the channel-generating region;
a vibration electrode set to a second potential, facing through a vibration-cavity to a top surface of the channel-generating region; and
a cavity-surrounding insulating-film surrounding the vibration-cavity so that the vibration-cavity is provided in a location between the channel-generating region and the vibration electrode, configured to implement a hermetically confined space with the vibration-cavity,
wherein displacements of the vibration electrode by ultrasonic waves are detected as changes of current flowing between the first and second main-electrode regions in each of the unit cells.

FIG1

FIG2

FIG3

FIG.4A

FIG.4B

FIG.4C

EP 4 422 209 A1

FIG.5A

FIG.5B

FIG.6A

FIG.6B

FIG.6C

FIG.7A

$^{49}BF_2^+$     $^{49}BF_2^+$     $^{49}BF_2^+$

12
13
n
11
13

FIG.7B

12
13
p
n
13
11    14

FIG.7C

15a

15b

13

13

12

n⁺

p

n⁺

n

11

14

FIG.7D

15a

16

17p

15b

13

13

12

n⁺

p

n⁺

n

11

14

FIG.7E

17o      17c      17o

16

13        $n^+$      p      $n^+$      13

12

n

15a      11      14      15b

FIG.7F

17o      17c      17o

21p
19p
18
16

13        $n^+$      p      $n^+$      13

12

n

15a      11      14      15b

FIG.7G

63

FIG.7H

FIG.7I

21o 19o 17o 22 23 17c 17o 19o 21o

18

16

13

12

n+       p·       n+      13

n

15a     11     14     15b

FIG.7J

23 19o     17o 22 23 17c 18 17o 19o 21o 23

21o                                      21o

18                                      24b

24a                                      18

16                                      16

13

12

n+       p·       n+      13

n

15a     11     14     16     15b

FIG.7K

21o 19o 24a     17o 22 23 25 17c     17o 19o 24b 21o

18

16

13

12

n+

p·

n+

13

n

15a 11 14 15b

FIG.7L

26 25a     17o 22 23 17c 25c     17o 24b 25b

21o

19o

18

24a

16

13

12

n+

p·

n+

13

n

15a 11 14 15b

21o

19o

13

FIG.7M

FIG.7N

FIG.7O

FIG.7P

FIG.7Q

FIG.7R

FIG.8

FIG.9

FIG.10

FIG.11

FIG.12

FIG.13

FIG.14

FIG.15

FIG.16

FIG.17

FIG.18

FIG.19A

FIG.19B

$V_{bias}$

$C_1$

$L_m$

$C_m$

$r_m$

28e

$C_3$

$R_{GND}$

FIG.19C

$V_{DD}$

$V_{SIG}$  $R_{D1}$    $R_{D2}$  $V_{REF}$

$I_{DS}$

$R_{GND1}$

$R_{GND2}$

17o

$n^+$   $n^+$

$Q_{SIG}$   $Q_{REF}$

$n^+$   $n^+$

$17q_{12}$    $17q_{11}$   $17q_{21}$    $17q_{22}$

FIG.20A

FIG.20B

FIG.20C

FIG.20D

FIG.22B

FIG.23

FIG.24

FIG.25

FIG.26

FIG.27

FIG.28

**EP 4 422 209 A1**

## INTERNATIONAL SEARCH REPORT

International application No.

**PCT/JP2022/034796**

| | | |
|---|---|---|
| **A. CLASSIFICATION OF SUBJECT MATTER** | | |

*H04R 19/00*(2006.01)i
FI: H04R19/00 330

According to International Patent Classification (IPC) or to both national classification and IPC

**B. FIELDS SEARCHED**

Minimum documentation searched (classification system followed by classification symbols)

H04R3/00,19/00; A61B8/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2004-274756 A (GENERAL ELECTRIC COMPANY) 30 September 2004 (2004-09-30) paragraphs [0018]-[0042] | 1-6 |
| A | JP 2018-530196 A (KONINKLIJKE PHILIPS N. V.) 11 October 2018 (2018-10-11) entire text | 1-6 |
| A | JP 2017-85257 A (CANON KABUSHIKI KAISHA) 18 May 2017 (2017-05-18) entire text | 1-6 |
| A | WO 2016/194208 A1 (HITACHI, LTD.) 08 December 2016 (2016-12-08) entire text | 1-6 |
| A | JP 2005-103294 A (GENERAL ELECTRIC COMPANY) 21 April 2005 (2005-04-21) entire text | 1-6 |
| A | JP 2004-503312 A (KONINKLIJKE PHILIPS ELECTRONICS N. V.) 05 February 2004 (2004-02-05) entire text | 1-6 |

☐ Further documents are listed in the continuation of Box C.　　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **28 November 2022** | **06 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

85

<div align="center">

**INTERNATIONAL SEARCH REPORT**
**Information on patent family members**

</div>

| International application No. |
| --- |
| **PCT/JP2022/034796** |

| Patent document cited in search report | | | Publication date (day/month/year) | Patent family member(s) | | | Publication date (day/month/year) |
| --- | --- | --- | --- | --- | --- | --- | --- |
| JP | 2004-274756 | A | 30 September 2004 | US paragraphs [0028]-[0050]<br>DE<br>KR<br>CN | 2004/0174773<br><br>102004011193<br>10-1037819<br>1527414 | A1<br><br>A1<br>B1<br>A | |
| JP | 2018-530196 | A | 11 October 2018 | US entire text<br>WO<br>EP<br>CN | 2018/0229268<br><br>2017/025438<br>3334538<br>107921480 | A1<br><br>A1<br>A1<br>A | |
| JP | 2017-85257 | A | 18 May 2017 | US entire text<br>WO | 2018/0310915<br><br>2017/068761 | A1<br><br>A1 | |
| WO | 2016/194208 | A1 | 08 December 2016 | US entire text<br>EP | 2018/0161813<br><br>3306952 | A1<br><br>A1 | |
| JP | 2005-103294 | A | 21 April 2005 | US entire text<br>DE | 2005/0075572<br><br>102004047814 | A1<br><br>A1 | |
| JP | 2004-503312 | A | 05 February 2004 | US entire text<br>WO<br>EP | 6443901<br><br>2001/097559<br>1294493 | B1<br><br>A2<br>A2 | |

Form PCT/ISA/210 (patent family annex) (January 2015)

**EP 4 422 209 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007074263 A